# EUROPEAN PATENT APPLICATION

(11) **EP 4 005 596 A1**
(43) Date of publication of application: **01.06.2022**
(21) Application number: 20844119.6
(22) Date of filing: 22.07.2020
(51) Int. Cl.: A61K 45/00, A61K 48/00, A61P 25/00, A61P 25/18, A61P 25/24, A61P 25/28, C12N 15/12, A61K 38/17, G01N 33/15, A61K 31/7105, A61K 31/711, A61K 31/713, A01K 67/027

(54) **TRANSGENIC NON-HUMAN ANIMAL CAPABLE OF CONTROLLING EXPRESSION OF TRANSCRIPTION FACTOR RP58**

(30) Priority: 24.07.2019 JP 2019136490
(71) Applicant: Tokyo Metropolitan Institute of Medical Science, Tokyo 156-8506 (JP)
(72) Inventor: OKADO, Haruo, Tokyo 156-8506 (JP); SHIMBO, Hiroko, Tokyo 156-8506 (JP)
(74) Representative: J A Kemp LLP
(86) International application number: PCT/JP2020/029372
(87) International publication number: WO 2021/015297

(57) **Abstract**

Provided is an invention that is based on the novel function of a transcription factor RP58 in cells of the central nervous system. The present invention relates to: a transgenic non-human animal capable of increasing or decreasing the expression of the transcription factor RP58 in cells of the central nervous system of a non-human animal in the nascent stage and/or during and after the developmental stage; and a pharmaceutical composition for use in the treatment or prevention of brain dysfunction, or behavioral disorder, or a disease related thereto, wherein the pharmaceutical composition comprises a transcription factor RP58 protein, or a gene encoding the transcription factor RP58; etc.

SEQUENCE LISTING

<110>  Tokyo Metropolitan Institute of Medical Science

<120>  Transgenic non-human animal capable of controlling expression of
       transcription factor RP58

<130>  G2419WO

<150>  JP 2019-136490
<151>  2019-07-24

<160>  36

<170>  PatentIn version 3.5

<210>  1
<211>  8538
<212>  DNA
<213>  Homo sapiens

<400>  1
gaggaggcgg aggacgcggc ggcggcgggg acgcggtgac ttagggccgc tccgtgtgag      60

tcccggcgcc ggcccctggc ccctgacctg gcgccccctc cgcgccccgg caccccggcg     120

ggcggggggc gccagctgtg cggccccggc cggctgggcg gcgcgggcgg agggcgggcc     180

gggtcggggg cgcccccgcg agtggagtta gtttgtgtgg ttggagctgt tggggcggcg     240

gggggcggcg cagctgcggg aggcccgggc gcggggacgg ggccgctgca gctgcggggt     300

cgggcggggt gcgggttccc ggctccgcgc cgcgcccccg cgggccggtg cagctgcggc     360

gcggggcggg ctcccggccg gtgcagctgc ggtggcgcct ctcgggtgcg gagggcggcg     420

ggctggggga gggggcttgg gagcagccgc agctgcagtg gcgcccggga cggccgcggc     480

tccggggacc ggtgcagctg cagtggcgcc cgggggaggg ggccggcggg ggagggggcc     540

ctcccgggct ggtgcagttg cccggaggcc ccgggggctc gcgcgtcgcg gcatcgggga     600

agcggtagca accactctcc tcccgctggc tgcttcttac gacttttctt ttgttgttgc     660

cgtgacccgg gtctgttttc tcctctgttg tttaaggtgt tttcctgctg caccggctct     720

gcgccctcca cctccttccc tgtggtttta accttcctct ttccccgtgt gttttatgca     780

cggcgaacta cgtaaagatt tgcattgctt ccccactcgg cacccccgcc cccacctctc     840

tgaaaaacaa aaccccaacc tcacaaaacc tctatggatc cgcggtagcg agacgtgaag     900

gggttgattt gtggagtggg agttgctggc ccattgcggt gcccgggact cattaaactg     960

tcactcaccc cccaccccac tgggtaaatg gggtgattaa tgtctgatat attggaatgg    1020

gtcgagggca tttgtggaga ataggtggtg tggctgggaa gaaattgatc tcccgaggat    1080

gtctcctgga ctaagtgttc ataattatgt cactcaccgc gaagttggag aaagttaggt    1140

ttgtgacttt gggaaagcga gaaggacagg acggagtcgt cgggttttgt tactctttaa    1200

ctctccgcca cggctttcta atgggaacga agtggttatc accagggctg ggacgctggc    1260

gcctcaggcc ccagttcctt ttctacccct cgccctccca agagtctgag gagatgtatt 1320

tgacattcag agatgtcgca gcaccctgcc cggtaccggt cagcccagcc cgggtcgcgt 1380

tacagtttca tcagctatta gaaaagaccc acaaactggc tgaaaagttc tcaaacttac 1440

tagacctggc ttccccccac acaaagattt tcttgtgatc ataaacatta gagtgacccc 1500

cccccaaaga aaaaaaagat tgttaaggga cacagatcaa ggggaaggca aggttctgct 1560

ttttctgagc agaaagatca gatatgcagg tgcagcattc attttaagtg actggagcag 1620

gttgtatcag tttattaatt ggtctcgttg aactgaagta ctttccagga tttcagaaag 1680

tgcagtttca gcttgttggt accgagaatt gaaatttgca ttgactgtgg cagtgtaaga 1740

aatttttctg agaaggtagt tgtagataag taattacata agagtgaaat aataaggtga 1800

accagtctga agtaatagtt ctgaacaagt ctgccaacat ttgagacatg tataccatta 1860

tctcagcaga aaaaaataaa aaagtgcggt aataatgttg aaatgggact ggagatgtca 1920

aatgggttgg tttaaaaata tccctaaact gctttagaaa ctaaacgttg gagttgaata 1980

aaagtgaatc tggatttcct gaatagcacc gtggcttgag tgtgctgtca gtatgtaagc 2040

cagtccacca acttaagaca gaagtgttgc tattccataa atacagtaga actccatatg 2100

ctgagttttt caatttcttc tgttagaaag tttggaaaaa aaggaagact ttgaagtatt 2160

ttgtttatac taacagaaga gaaaagttct cctaagaaaa agctggtggc aggagtgtag 2220

aggagtagca tatggcatta aaaggagcac agctggaggt agcatttcca tctgaacatg 2280

atgtcagagc agctgacagc ctgccatccc tcagcctttt attctaacac acagacaggg 2340

agttagtgtg tgcggatctg tggtggagaa ggtatctcat tcctctctaa catcatctcc 2400

actttgcatc tgtctctctt agtctgcttt ttttccaccg atgtaacaga cctggagcca 2460

gcaggactca gaggaaagga cttaatcagg tttatgtgtc ctaaaggtag gagtagcaag 2520

agattagatt gagcatattt ctgttttggt gttttgttta tttaatttta aaaatcacat 2580

tacttttcta acccagttta ggaataatat atgaaattga gtattaaatg aacatcttac 2640

tggagttgaa agtcttgagc ttgtttagtt ttaatttttt atccctttta cttgagcgag 2700

taaataactt taatacacaa atatttcaag ttttgaaatg ttaagaaagg ctttaaaatg 2760

ctgcttttgg aaatcacgtt ggattaaaat gcttttttgt tgttcactgc tatttagatt 2820

tgattggggt ggtggtggtg gggaaatagt aaactggaat ccagcattca aacttatatg 2880

agaagattat tctagttaag cagctagatt gttgtataat taattcctct gtgaaacttt 2940

attcttcaaa acattgagca gcactactga aacatacagc tgtagtgctg ttgaaactgc 3000

aggtttttaa gaagaagaaa aaaaaaccaa acttttgtcc ccaggagaat agtttgactg 3060

gtttcctcat ttctctttca aatatatatt taagagatct tttatttcag cataacaata 3120

attatttgca ggagtttttg tgttagcgac taatttagaa cttgtagatt tgagctgcct 3180
gaattggcca gacagctgta atcgcactcc tctattctta atctctttat ctgggcagca 3240
gctgccatat ggccacagtc agctggatca gatgtttata agcttccttc ttcgtccctc 3300
tctgtcctct cagcctccta atttgatcac ggctaccttg tgagctgccc tccaatcttt 3360
atttttagcc ttctcaagga ttaggattga tgttagctgt ggggcactta aagtgattgt 3420
attgtaaatc ttagtttatt ctagcagtgg catcttgcag aatgtgtctg gggttaggat 3480
aaagttttag tctgtcttgg gcaaagtgta gaagaacaag aagaaaggaa gtaatttctg 3540
aatattaaat taagtgtatc tattcccaaa atgcacttgc tgagatgggt ggtgtggagg 3600
cttgcttctg gtcacaggaa aggggaagtc gggtcttctt tatttttcag gagtttaccc 3660
actgcagttg aattctgacc ctttgtacat ctttgccttt ctcctccctc catttcactt 3720
tccctcatca caaaacaagg gcaatgatga atatttgcaa gaatagaagc tagtgggaaa 3780
acagaaatat atcaaattat attgaaacaa agtatttctt gttggctgag gtaaggcaga 3840
accaggaaaa aaaaaaaggt tctctttttc taaaaaggct taaaaaaaaa cgttgaacct 3900
gtaaagcttt tgagttttat ttaagcgctg tatgccttta tttttaaaag tttgaattaa 3960
tttcaatatg aaactcattt tgccttgcaa cggatcaaag tttagatgag aattttcgga 4020
gttcagtaaa gcccctctgg ttcctaatat tctgaaagta cagtttctac aaagaaatgt 4080
aactactttt ttctcagttc gttttgaaaa atctttggat ttgttcatag taaagatgat 4140
cttttccatc tgttggtgca gctccttctg atctcgtaca tgtgtgctat acctgcaaat 4200
ctgaaggcat taacatctgt ttttatacac agggcttttg ttgtaacacc ctgactttaa 4260
aagtaagttg tttgctaatt tctggactaa ttatgcaatt tcattttttt ttcttgattc 4320
agccaaatgt gtgtgtgtgt gtttaaactg tgctttctaa gcacagtcag gtagcaaaag 4380
taataaaaag gatggttgaa caagttttct tgtatgttcc aggatatgtt tgggactttt 4440
ctttgtttat tatatgagtt gttccctttg aaattaaagc tattttgtag gttttgtggg 4500
acataatttg ataagtagag ttaattaaat ttcttctgga agagatctaa attcttattc 4560
ttagtgagag actgtagtta aaggaaggct tttagaactt gggttcaagg aagatggaga 4620
tgcgtcggaa gctctttggc gggggtgagg aagttcagaa agtgtgcatt ttccttctgg 4680
catttaggtc ttgtccgtgt gatttggtgg tgcttgggtc ataagcctga ttaaaattca 4740
gggacatgta ccacggcggc caaagcggaa ttaatttttt tatatgggga ctggagcgct 4800
gaaaagttgt tcctgaccag gctctaatga gaaattcctc tctccccagg ttatgaagac 4860
agtatggagt ttccagacca tagtagacat ttgctacagt gtctgagcga gcagagacac 4920
cagggttttc tttgtgactg cactgttctg gtgggagatg cccagttccg agcgcaccga 4980
gctgtactgg cttcatgcag catgtatttc cacctctttt acaaggacca gctggacaaa 5040

agagacattg ttcatctgaa cagcgacatt gttacagccc ccgctttcgc tctcctgctt 5100

gaattcatgt atgaagggaa actccagttc aaagacttgc ccattgaaga cgtgctagca 5160

gctgccagtt atctccacat gtatgacatt gtcaaagtct gcaaaaagaa gctgaaagag 5220

aaagccacca cggaggcaga cagcaccaaa aaggaagaag atgcttcaag ttgttcggac 5280

aaagtcgaga gtctctccga tggcagcagc cacatagcag gcgatttgcc cagtgatgaa 5340

gatgaaggag aagatgaaaa attgaacatc ctgcccagca aaagggactt ggcggccgag 5400

cctgggaaca tgtggatgcg attgccctca gactcagcag gcatccccca ggctggcgga 5460

gaggcagagc cacacgccac agcagctgga aaaacagtag ccagcccctg cagctcaaca 5520

gagtctttgt cccagaggtc tgtcacctcc gtgagggatt cggcagatgt tgactgtgtg 5580

ctggacctgt ctgtcaagtc cagcctttca ggagttgaaa atctgaacag ctcttatttc 5640

tcttcacagg acgtgctgag aagcaacctg gtgcaggtga aggtggagaa agaggcttcc 5700

tgtgatgaga gtgatgttgg cactaatgac tatgacatgg aacatagcac tgtgaaagaa 5760

agtgtgagca ctaataacag ggtacagtat gagccggccc atctggctcc cctgagggag 5820

gactcggtct tgagggagct ggaccgggag gacaaagcca gtgatgatga gatgatgacc 5880

ccagagagcg agcgtgtcca ggtggaggga ggcatggaga gcagtctgct cccctacgtc 5940

tccaacatcc tgagccccgc gggccagatc ttcatgtgcc ccctgtgcaa caaggtcttc 6000

cccagccccc acatcctgca gatccacctg agcacgcact tccgcgagca ggacggcatc 6060

cgcagcaagc ccgccgccga tgtcaacgtg cccacgtgct cgctgtgtgg gaagactttc 6120

tcttgcatgt acaccctcaa gcgccacgag aggactcact cgggggagaa gccctacaca 6180

tgcacccagt gcggcaagag cttccagtac tcgcacaacc tgagccgcca tgccgtggtg 6240

cacacccgcg agaagccgca cgcctgcaag tggtgcgagc gcaggttcac gcagtccggg 6300

gacctgtaca gacacattcg caagttccac tgtgagttgg tgaactcctt gtcggtcaaa 6360

agcgaagcac tgagcttgcc tactgtcaga gactggacct tagaagatag ctctcaagaa 6420

ctttggaaat aattttatat atatataaat aatatatata tatatacata tatataaata 6480

gatctctata tagttgtggt acggtctaaa agcagtcttg tttcctggaa ataaaaagtt 6540

gggatattaa cttgtttttg cactttagaa tagcatgaga atctcactaa tttagcattc 6600

tgataaaaga aactttagag caagtcagaa tagagaggtg tttttccttt gaggggatag 6660

gggaagtaag ccaataagaa ccttttaaac aaatcgtcct gtcacaaaat gctttcatat 6720

ggcttaattt tgtcaacact gcattgtctt ttgagctctt ttttcccccc caacaaagtt 6780

tttttgtttt ttgttttttt ttttaagtag aaattccctc cagttttatt agcctcttta 6840

tatgtctcaa attgcatgaa ttttttctgg ctgttggaaa cctgaatgct tttagaccca 6900

aatggaaaat ttctgaaatg ctggattatc tatttttaaa caagcagttg acttaaaact 6960

ttctgtggca acttctggtt ttctgacagt tcccagtgag agaaatgctg aaagtacact 7020

gggatcactg ggacactgtc ttatgaaggt ttgcttggga tgaaaaagga tattgcagct 7080

tcagcagtgt tgaactgtgt gtttaaaaat gtgaattact gttattgtat actgtaattg 7140

attacatggg ctgggggggt gtcaaagaac ttgacaggtt gtgttgatgc tcttagttga 7200

gtcttgaaaa gtaaatatta acgctacaga aatgcatgag tttcaatata ttttttgtct 7260

ttgtttgcat tgtataactt taacgagtga gtttaaaatt atttaatttc cttagaaaaa 7320

taacaccatt tggaaaaaaa aactggtgtt atgaagaacg taaatgcact gtttttattt 7380

ttattttata taatttaaat tgactttccc actgtcttta agttgaaact gttaagctga 7440

ataaaaactt aagctgcaaa ttgataactt cgctacataa caaggaaaat ataaatgttt 7500

acaaacagct taaagatttg catgtgcagt gtgcatttat aacaaacttc taattgcaca 7560

aaacccatgc cagctcagag tttaggtgta cacatttacc cagttgagcg ttcttagaat 7620

aactactgca caagttgaca ataggtcgtt ctctcttttt ttttgtttgc tccctttttc 7680

tttttctccc cttcctcctt accctccctc ccttactctc cccccccacc accaccctcc 7740

acccccaact catgaaaaga ttctatggac tgaaaaagcc ccaggctgaa aggactggac 7800

tgccttgatt gacatgggga agggggttag tagactatgt ggattgcggc agcagaggct 7860

gcagcctaac gtgtggtttt aatgaccagc acgcaaggca aaagcatttt gcacagtgtt 7920

tgttttcctg tcttgcactt acaaataagg tctatgggag tagcatggaa aacgtttgct 7980

gtttttccct ttttttttta attgcttttg tttaaaattt gatcgcctta actactgtaa 8040

acatagccta tttttgtgct taagatactg aatggaaaac tccattgtgt gttgctggac 8100

tgttttggaa atatttggtt aaatgtgtgt taatttggct gtaatggcat ttaaagcaaa 8160

caaacaaaca aaaaaagctg tgaaaatggc cttggagcat tatctttagt tacttgaaga 8220

gtttctagtt tttttaaaat acagtttatg ttaaaataat ttttattaat ttagagaaga 8280

caatcaatgt ctgtgagaaa acggactttc ttttggattt tctttttgtg gtcattgtga 8340

gtgattgctt tttccttttc ttagtttcac attcttcctt tgttctaaaa cttagactga 8400

catctagctt tgacaatcat agtatgtttt attttcctga gggggaataa cttataatgc 8460

tgtttagttt tgtactattg gtgtgttggt gaatttttaa actgtgtgct aactgcaata 8520

aattatatga actgagaa 8538

<210> 2
<211> 55
<212> DNA
<213> Homo sapiens

<400> 2

gaggaggcgg aggacgcggc ggcggcgggg acgcggtgac ttagggccgc tccgt 55

<210> 3
<211> 186
<212> DNA
<213> Homo sapiens

<400> 3
attctaacac acagacaggg agttagtgtg tgcggatctg tggtggagaa ggtatctcat 60

tcctctctaa catcatctcc actttgcatc tgtctctctt agtctgcttt ttttccaccg 120

atgtaacaga cctggagcca gcaggactca gaggaaagga cttaatcagg tttatgtgtc 180

ctaaag 186

<210> 4
<211> 584
<212> DNA
<213> Homo sapiens

<400> 4
gttgtttgct aatttctgga ctaattatgc aatttcattt ttttttcttg attcagccaa 60

atgtgtgtgt gtgtgtttaa actgtgcttt ctaagcacag tcaggtagca aaagtaataa 120

aaaggatggt tgaacaagtt ttcttgtatg ttccaggata tgtttgggac ttttctttgt 180

ttattatatg agttgttccc tttgaaatta aagctatttt gtaggttttg tgggacataa 240

tttgataagt agagttaatt aaatttcttc tggaagagat ctaaattctt attcttagtg 300

agagactgta gttaaaggaa ggcttttaga acttgggttc aaggaagatg gagatgcgtc 360

ggaagctctt tggcgggggt gaggaagttc agaaagtgtg cattttcctt ctggcattta 420

ggtcttgtcc gtgtgatttg gtggtgcttg ggtcataagc ctgattaaaa ttcagggaca 480

tgtaccacgg cggccaaagc ggaattaatt tttttatatg gggactggag cgctgaaaag 540

ttgttcctga ccaggctcta atgagaaatt cctctctccc cagg 584

<210> 5
<211> 3688
<212> DNA
<213> Homo sapiens

<400> 5
ttatgaagac agtatggagt ttccagacca tagtagacat ttgctacagt gtctgagcga 60

gcagagacac cagggttttc tttgtgactg cactgttctg gtgggagatg cccagttccg 120

agcgcaccga gctgtactgg cttcatgcag catgtatttc cacctctttt acaaggacca 180

gctggacaaa agagacattg ttcatctgaa cagcgacatt gttacagccc ccgctttcgc 240

tctcctgctt gaattcatgt atgaagggaa actccagttc aaagacttgc ccattgaaga 300

cgtgctagca gctgccagtt atctccacat gtatgacatt gtcaaagtct gcaaaaagaa 360

gctgaaagag aaagccacca cggaggcaga cagcaccaaa aaggaagaag atgcttcaag 420

ttgttcggac aaagtcgaga gtctctccga tggcagcagc cacatagcag gcgatttgcc 480

cagtgatgaa gatgaaggag aagatgaaaa attgaacatc ctgcccagca aaagggactt 540

ggcggccgag cctgggaaca tgtggatgcg attgccctca gactcagcag gcatccccca 600

ggctggcgga gaggcagagc cacacgccac agcagctgga aaaacagtag ccagcccctg 660

cagctcaaca gagtctttgt cccagaggtc tgtcacctcc gtgagggatt cggcagatgt 720

tgactgtgtg ctggacctgt ctgtcaagtc cagcctttca ggagttgaaa atctgaacag 780

ctcttatttc tcttcacagg acgtgctgag aagcaacctg gtgcaggtga aggtggagaa 840

agaggcttcc tgtgatgaga gtgatgttgg cactaatgac tatgacatgg aacatagcac 900

tgtgaaagaa agtgtgagca ctaataacag ggtacagtat gagccggccc atctggctcc 960

cctgagggag gactcggtct tgagggagct ggaccgggag gacaaagcca gtgatgatga 1020

gatgatgacc ccagagagcg agcgtgtcca ggtggaggga ggcatggaga gcagtctgct 1080

cccctacgtc tccaacatcc tgagccccgc gggccagatc ttcatgtgcc ccctgtgcaa 1140

caaggtcttc cccagccccc acatcctgca gatccacctg agcacgcact tccgcgagca 1200

ggacggcatc cgcagcaagc ccgccgccga tgtcaacgtg cccacgtgct cgctgtgtgg 1260

gaagactttc tcttgcatgt acaccctcaa gcgccacgag aggactcact cgggggagaa 1320

gccctacaca tgcacccagt gcggcaagag cttccagtac tcgcacaacc tgagccgcca 1380

tgccgtggtg cacacccgcg agaagccgca cgcctgcaag tggtgcgagc gcaggttcac 1440

gcagtccggg gacctgtaca gacacattcg caagttccac tgtgagttgg tgaactcctt 1500

gtcggtcaaa agcgaagcac tgagcttgcc tactgtcaga gactggacct tagaagatag 1560

ctctcaagaa ctttggaaat aattttatat atatataaat aatatatata tatatacata 1620

tatataaata gatctctata tagttgtggt acggtctaaa agcagtcttg tttcctggaa 1680

ataaaaagtt gggatattaa cttgtttttg cactttagaa tagcatgaga atctcactaa 1740

tttagcattc tgataaaaga aactttagag caagtcagaa tagagaggtg tttttccttt 1800

gaggggatag gggaagtaag ccaataagaa ccttttaaac aaatcgtcct gtcacaaaat 1860

gctttcatat ggcttaattt tgtcaacact gcattgtctt ttgagctctt ttttcccccc 1920

caacaaagtt tttttgtttt ttgttttttt ttttaagtag aaattccctc cagttttatt 1980

agcctcttta tatgtctcaa attgcatgaa ttttttctgg ctgttggaaa cctgaatgct 2040

tttagaccca aatggaaaat ttctgaaatg ctggattatc tatttttaaa caagcagttg 2100

acttaaaact ttctgtggca acttctggtt ttctgacagt tcccagtgag agaaatgctg 2160

aaagtacact gggatcactg ggacactgtc ttatgaaggt ttgcttggga tgaaaaagga 2220

tattgcagct tcagcagtgt tgaactgtgt gtttaaaaat gtgaattact gttattgtat 2280

actgtaattg attacatggg ctgggggggt gtcaaagaac ttgacaggtt gtgttgatgc 2340

tcttagttga gtcttgaaaa gtaaatatta acgctacaga aatgcatgag tttcaatata 2400

ttttttgtct ttgtttgcat tgtataactt taacgagtga gtttaaaatt atttaatttc 2460

cttagaaaaa taacaccatt tggaaaaaaa aactggtgtt atgaagaacg taaatgcact 2520

gtttttattt ttattttata taatttaaat tgactttccc actgtcttta agttgaaact 2580

gttaagctga ataaaaactt aagctgcaaa ttgataactt cgctacataa caaggaaaat 2640

ataaatgttt acaaacagct taaagatttg catgtgcagt gtgcatttat aacaaacttc 2700

taattgcaca aaacccatgc cagctcagag tttaggtgta cacatttacc cagttgagcg 2760

ttcttagaat aactactgca caagttgaca ataggtcgtt ctctcttttt ttttgtttgc 2820

tccctttttc tttttctccc cttcctcctt accctccctc ccttactctc cccccccacc 2880

accaccctcc acccccaact catgaaaaga ttctatggac tgaaaaagcc ccaggctgaa 2940

aggactggac tgccttgatt gacatgggga agggggttag tagactatgt ggattgcggc 3000

agcagaggct gcagcctaac gtgtggtttt aatgaccagc acgcaaggca aaagcatttt 3060

gcacagtgtt tgttttcctg tcttgcactt acaaataagg tctatgggag tagcatggaa 3120

aacgtttgct gtttttccct ttttttttta attgcttttg tttaaaattt gatcgcctta 3180

actactgtaa acatagccta tttttgtgct taagatactg aatggaaaac tccattgtgt 3240

gttgctggac tgttttggaa atatttggtt aaatgtgtgt taatttggct gtaatggcat 3300

ttaaagcaaa caaacaaaca aaaaaagctg tgaaaatggc cttggagcat tatctttagt 3360

tacttgaaga gtttctagtt tttttaaaat acagtttatg ttaaaataat ttttattaat 3420

ttagagaaga caatcaatgt ctgtgagaaa acggactttc ttttggattt tctttttgtg 3480

gtcattgtga gtgattgctt tttccttttc ttagtttcac attcttcctt tgttctaaaa 3540

cttagactga catctagctt tgacaatcat agtatgtttt attttcctga gggggaataa 3600

cttataatgc tgtttagttt tgtactattg gtgtgttggt gaatttttaa actgtgtgct 3660

aactgcaata aattatatga actgagaa 3688

<210> 6
<211> 8409
<212> DNA
<213> Mus musculus

<400> 6
gcggccgccg ctgggctgct gccatgggga gccgttgaaa gagtggggcc ctgtggctcg 60

gccgcgcgcc tcaggctgcc agcgcgggcg gggggcggcg ggggcgcgcg gggcccgggc 120

cggggccggc gggcgggcgg gggccggcgg aggacgccca caagtccagc ggcggcggcg 180

gcggcagagg aggaggagga ggaggacgcg gcggggacgc ggcgactcgg gccgctccgt 240

gtgagtcccc gcgcccgccc tgccccgctg ccgaccgtgg gctcccgccg ggcggagtta 300

gttagtgtgg cggagggcag gcggtgcggg ctcgcctcgc ctcgcctggc cgcgctcccc 360

ggccggtgca gctgcggcgc ggggcggact cggtgcagct gcggcggcgc ctcccggtgc 420

ggagggcggg cgggcgggcg ggggaggggg ccgggccgcc gcagctgcag cgcccgggac 480

ggccgcggct ccggggccgg tgcagctgca gggccggggg aggggagggg cgcggcgccc 540

gggttgcggt tgccgggagg ccccgggggc tggcgcgcgg cggcggcgac gggggagcgg 600

tcgccgcgcc gcttcttgcg acttttcttt tgttgttgcc gcgaccgggg tctgttttct 660

cggccgttgt ggaaggtgct ttcctgctgc accggctctc tgccctgcac cttcttcccc 720

gtggtttcac ccttcctctt tccctgaagt gctttatgca ccgcgaactg cgggaagatt 780

tgcatctcct ctcccacccc tgaacctcac acacacaccc ggggcaccct gcccccacct 840

ctgaaaaaca aaccccctaa cctcacagaa cctccgaggg tccgcggtag cggtggacgg 900

gtcgatccgt ggagtggatg tcttcgggcc attgcggtgc ttgggactca ttaaactgtc 960

actcaccccc accaccaccc cactgggtaa actgggtgat taatgtcgga tatagtggaa 1020

tggggcgagg gcatctgtgg aaaagagggt gtgtgtgtgg cagggaagta atgtgtctat 1080

ctgcacagag tgcctcccga acgagttcag aattgtgtca ttcaccgcga agttagagaa 1140

agttaggttt gtgactcgtg agcgagagca gaacacagtt ggggttttgt tctctagctc 1200

cctgcctctg ttttctaagc gagcctaagt ggttgtcgcc tgggttggga cgcttgtgcc 1260

tcaggcccca acttcttttt tctactctcc aacctcccaa gggtcggtgg agatgtgtct 1320

gatattcata gatgtcgcag caccttgcag ggcacccagc gacccagccc cggttacatt 1380

aacattacat cagccattag aaaagatcca caaactgtag ccaaaaaaag ttcccaaagt 1440

ctacacttgg cctcccaacc ctcctggtga tcacaacccc tttccccaaa gttatagaga 1500

ccaagggcaa gacaaaagtg acttttctga gcaggaagac aagctgtgtg gtcaaacaac 1560

cattctaagt gactgtagca ggttttgtca gtttattaat ttgcctcatt gaactaaaat 1620

actttccagt atttcagaag tgcagtttga gtttgttgat gctgaaagtg gaaatttgca 1680

ctaacggtgg cagtgtgaac tattttcctg agaggtagat ccagataaat tgcttaggta 1740

agagtaaaac aataaggtga accacgctga agtaatagtt cttaataagt ctgccaacat 1800

ttcggagatg tctgccatta tctccgcagg ggaaggggga aaaacatgct gtaataatgt 1860

tgaaatggga ctagagatgt caaatgggtt ggtttaaaaa tatccctaag ctgcctttga 1920

aactagacgt tggagttgaa taaaagtgaa tgtgggtttc ctgaatagca ccgagctcga 1980

gtgtgctgtc atatgtaagc cagtccacca acttgaggca gaagtgttgc gattccataa 2040

atacagtagg actccatatg ctgagctttt caatttcttc tgttagaaag tttgggggga 2100

aaaaggaaga ctttgaagta ttttgtttat actaacagaa gagaaaagtt ctcctaagaa 2160

aaagctggtg gcaggagtgt agaggagtag catatggcat taaaggagca cagctggagg 2220

tagcatttcc atctgaacat gatgtcagag cagctgacag cctgccatcc ctcagccttt 2280

tattctaaca cacagacagg gagttagtgt gtgcggatct gtggtggaga aggtatctca 2340

ttcctctcca acatcatctc cactttgcat ctgtctctct tagtctgctt tttttccacc 2400

gatgtaacag acctgcagcc agcaagactc cgagggaagg acttaatcaa gtttatgtgt 2460

cctaaaggta ggagtagcag gagattaggc tgagcatctt tgtgtttggg tgttttattt 2520

aatttttttt aaaatcacat tacttttcta atccacttta taaataatat gtgaaattga 2580

gcatgaagtg atggtcttac tggattgaaa gtcttgggct tgcttggttt acgattttat 2640

ttcttctatt tgagcaagta aataacttta atacacaaaa atatttcaag ttttcagatg 2700

gtaagaaacg ctctggaata tggcttttgg agctcactgt ggattaagat gtcttttgtt 2760

cactgctgtt tagattcctt ttgcttgagg ggggcagaaa cagtaaactg tctggaatcc 2820

agctttcaaa cttacacgag aagattattc tggttaagaa gctaggttgt gtaattaatt 2880

ccactgtgaa actttattct tcaaaacatt cggcaagcat tactgaaagc agcatacagc 2940

tgtagtgctt ttgaaaccgg agggtttttg ttgttgggtt ttttttttgt tttgttttgt 3000

tttttgtttt ttaagaagga aggaagaaag aaagagaact tttgttccta ggagaatagt 3060

ttgaccagtt tcctcatttc tcttttaaat atatatttaa gagatctttt atttcaacat 3120

aacaataatt atttgcagga gtttttgtgt tagcgactaa tttagaactt gtagatgtga 3180

gctgcctgaa ttggccagac agctgtaatc gcactcctct attcttaatc tctttatctg 3240

ggcagcagct gccatatggc cacagtcagc tggatcagat gtttataagc ttccttcttc 3300

gtccctctgt cctctcagcc tcctaatttg atcacggcta ccttgtgagc tgccctccaa 3360

tctttatttt tagccctctc aaggattagg attgatgtta gctgtagggc acttaaagtg 3420

attgtattgt aaatcttggt ttattctatt ggtggtgtct ttctttcaga aggtatcggg 3480

gttaggatga aagtttcact gtgtctcagc atagtgtgga ggagagaagt agtttctgaa 3540

tagtagcttc atatccatcc aagatgcact tgcagaggta gatgttggcg agctctgcct 3600

ttggtcccag gatggggaag tgggctccgc tgatcttaag agagttttag ccactacaga 3660

tgaattttga ctgatctttg ccttcccttc tttcaccctc tcccttgcca aaaaacatgg 3720

gaaagatgca taattggaag aatagaaggt actgggaaaa caaaatagat caggttatta 3780

ttgaaacaaa gtattttctg ttggatacct gaggcagagc cagaaaaaaa agttttattt 3840

attttatttt ttctaggaag gctttaaaaa atatttaact tacaaagttt ttgcacctca 3900

ttttacagtg gtaaaagttt gattaatttt aatatgaagt tccttttgcc ctatagcaga 3960

tcaaagttca gatgaaaatg ttcagagtta acagactctc tggttgctaa tgttgttgag 4020

gtagaggatc tacaaagaaa caggaccccc cccccattca tttttaaaat gtttggattt 4080

gttcatagta aagatgatct ttcccatctg tcggtacagt tctgctcctc tgtgtgtgta 4140

gtacctgcag atcttaccgc accaacatct gttcttatac acagggcttt tgttgttgta 4200

acactctgac tttaaaagta agttgtttgc tgatttctgc actaattatg caatttcatt 4260

ttttttcccc ttcatttcag ccaaatgtgt gtgttcagtc tgtgctttct aagtagttgg 4320

gtatcagacg tgttacaaag aatggctgga catgtcttct tgactcttcc agaatttgtg 4380

tgggactttg cctgatatta tgagttcttt ccctttgaag ttaaagctct cttttaggtt 4440

ttgtaggatg tcatttgatg agttgttggt taaatttctg gaagaggtct gagttcttgt 4500

tctaagtcat aagctaaagg aagctttaaa cttgaccagg agttatgagt ggggtgagca 4560

aagccttggt gattgcattt aggttgaatg tgtggcttgg gagtccttgg ggtgtgagcc 4620

tgatttaaaa ttcagacatg taactctcca ccactaaggc ggatattttt taaagtggcg 4680

actagagtct aaattcctga ctaggctcta acgagaaact cctctttccc caggttatga 4740

agacagtatg gagtttccag accacagcag acatttgctg cagtgtctga gcgagcagag 4800

gcaccagggc tttctttgtg actgcactgt tctggtggga gatgcccagt tccgggcaca 4860

ccgagctgtt ctggcttcat gcagcatgta tttccacctc ttttacaagg accagctgga 4920

caaaagagac attgttcatc tgaacagcga cattgtgaca gcccccgctt tcgctctcct 4980

gcttgaattc atgtacgaag ggaaactcca gttcaaagac ttgcccattg aggacgtgct 5040

agcagctgcc agttatctcc acatgtatga cattgtcaaa gtctgcaaaa agaagctgaa 5100

agagaaagct accacagagg cggacagcac caaaaaagaa gaagatgctt caagttgttc 5160

ggataaagtc gagagcctct cagacggcag cagccacatg gcaggtgacc tgcccagtga 5220

tgaagatgaa ggcgaagatg acaaattgaa cattctaccc agcaaaaggg acttggcagc 5280

tgagcctggg aacatgtgga tgcgattgcc ctcagactca gcaggcatcc cccaggctgg 5340

cggagaggcc gagccacacg ccacagcagc tggaaaaaca gtagccagcc cctgcagctc 5400

aacagagtct ttgtcccaga ggtctgtcac ctccgtgagg gattcggcag atgttgactg 5460

tgtgctggac ctgtctgtca agtccagcct ttcaggagtt gaaaatctga acagctctta 5520

tttctcttca caggacgtgc tgagaagcaa cctggtgcag gtgaaggtgg agaaagaggc 5580

gtcctgtgat gagagtgatg ttggcactaa tgactatgac atggaacata gcactgtgaa 5640

agagagtgtg agcactaaca acagggtaca gtatgagcca gcccacctgg ctcctctgag 5700

ggaggactcg gtcttaaggg aactggatcg ggaggacaaa gccagcgatg atgagatgat 5760

gaccccagag agcgagcggg tccaggtgga agggggcatg gagaacagtc tgcttcccta 5820

cgtctccaat atcctgagcc ccgcaggcca gatcttcatg tgccccctgt gcaacaaagt 5880

cttccccagc ccccacatcc tgcagatcca cctgagcacg cacttccgag agcaggatgg 5940

catccgtagc aagcctgccg ccgatgtcaa tgtgcccacg tgctccctgt gtgggaagac 6000

tttctcctgc atgtacaccc tcaagcgcca cgagaggact cactcggggg agaagcccta 6060

cacgtgcacc caatgcggca agagtttcca gtactcgcac aacctgagcc gtcatgccgt 6120

ggtgcacacc cgcgagaagc cccatgcctg caagtggtgc gagcgcaggt tcacgcagtc 6180

cggagacctg tacagacaca tccgcaagtt ccactgtgag ttggtgaact ccttgtcggt 6240

caaaagtgaa gcgctgagct tgcccactgt cagagactgg accttagaag atagctctca 6300

agaactttgg aaataatttt atatatatat atatataaat aatatatata tatatatata 6360

catatatata catagatctc tatatagttg tggtacggtc taaaagcagt cttgtttcct 6420

ggaactgaaa agttgggatc ttaacttgtt tttgcacttt agaataatag catgagaatc 6480

tcactaattt agcattctga taaaagaaac tagagcaagt cacaagtaga gagctgtctt 6540

tcttttgagg gttaggccaa gttagccaat aaaaccttct caacaaatgg ttctgtcaca 6600

aacgctttca catggcttac ttttgtacac actgcattgt cttgagcgct ttccctgtgc 6660

tccacactgt tttgttttgt tttgttttca gagtagaaat cccctccagt ttattagcct 6720

ctttatatgt ctcaaattgc atgaatattt tctggctgtt ggaaacctga atgcttttag 6780

acccaaatgg aaaatttctg aaatgctgga ttatctattt ttaaacaagc agttgactta 6840

aaactttgtg tggcaacttc tggttttctg atggtcccca gtgagagaaa tctgacagta 6900

cactgggtca ctgggactgt ctctgtgaag gtttgcttga gatgaaaaag gatattgcag 6960

cttcagcagt gttgaactgt gtgtttaaaa tgtgaattac tgttcttgta tactgtaatt 7020

gattacacgg gctggggggg gtgtcaaaga acttgacagg ttgtgttgat gctcttagtt 7080

gagtcttgaa aagtaaatat taacgctaca gaaatgcatg agtttcaata tattttttgt 7140

ctttgtttgc attgtataac tttaacgagt gagtttaaaa ttatttaatt tccttagaaa 7200

aatagcacca tttgggaaaa aaactggtgt tacgaagaac ataaatgcac tgtctttatt 7260

tttattttta ttttatataa tttaaattga cttttcccac gtctttaagt ttgaaactgt 7320

taagctgaat aaaaacttaa gctgcaaatt gataacttcg ctacattaca aggaacatat 7380

aaatgtttac aaacggctta aagaattgca tgtgcagtgt gcattcatac cacacttgta 7440

attgcacaga acccatgcca gctcagagtt taggtgtaca cacgtaccca gccgagtgtt 7500

tttagaataa ctactgcaca aattgacaat aggtcatagg tcattctctc tctccctcct 7560

ttttgcttcc cctttctttt tctcccctat ttcctttcct tccccctacc ccctaactca 7620

tgaaaagatt ctatggactg aaaaagcccc aggctgaaag gactgaaccg ccttgattga 7680

catggggagg ggggttagta gactgtgtat cggcagcaga ggctgcagcc cgatgtgtgg 7740

ttctgatggc cagcgcgggg cagaagcgtt ttgcacactg tttgttttcc tgtcttgcac 7800

ttacaaataa ggtctatggg agtagcatgg gaaacagtgg ttgtttttcc ctttttcttt 7860

tttaattgct tttgtttaaa atttgatcgc cttaactact gtaaacatag cctatttttg 7920

tgcttaagat actgaatgga aaactccatt gtgtgttgct ggactgtttt ggaaatattt 7980

ggtcaaatgt atgttaattt ggctgtaatg gcatttaaag caaacaaaca aacaaacaaa 8040

caaagctgtg aaaatggcct tggagcatta tctttagtta cttgaagagt ttctagtttt 8100

ttttttttta atacagttta tgttaaaata atctttatta atctagagga gacaatcaat 8160

gtctgtggaa gaacggactt ttttggattt tcttttttaa tggtcattgt gagtgattgc 8220

tctttctttt acttagtttc acattcttcc tttgttctaa atcttagact gacatctagc 8280

tttgacaatc atagtatgtt ttatttcctg agggggaata acttataatg ctgtttagtt 8340

ttgtactatt ggtgtgttgg tgaattttta aactgtgtgc taactgcaat aaattatatg 8400

aactgagaa 8409

<210> 7
<211> 245
<212> DNA
<213> Mus musculus

<400> 7
aggctgcggc cgccgctggg ctgctgccat ggggagccgt tgaaagagtg gggccctgtg 60

gctcggccgc gcgcctcagg ctgccagcgc gggcgggggg cggcgggggc gcgcggggcc 120

cgggccgggg ccggcgggcg ggcgggggcc ggcggaggac gcccacaagt ccagcggcgg 180

cggcggcggc agaggaggag gaggaggagg acgcggcggg gacgcggcga ctcgggccgc 240

tccgt 245

<210> 8
<211> 162
<212> DNA
<213> Mus musculus

<400> 8
agtgtgtgcg gatctgtggt ggagaaggta tctcattcct ctccaacatc atctccactt 60

tgcatctgtc tctcttagtc tgcttttttt ccaccgatgt aacagacctg cagccagcaa 120

gactccgagg gaaggactta atcaagttta tgtgtcctaa ag 162

<210> 9
<211> 1268
<212> DNA
<213> Mus musculus

<400> 9
tcagaaggta tcggggttag gatgaaagtt tcactgtgtc tcagcatagt gtggaggaga 60

gaagtagttt ctgaatagta gcttcatatc catccaagat gcacttgcag aggtagatgt 120

tggcgagctc tgcctttggt cccaggatgg ggaagtgggc tccgctgatc ttaagagagt 180

tttagccact acagatgaat tttgactgat ctttgccttc ccttctttca ccctctccct 240

tgccaaaaaa catgggaaag atgcataatt ggaagaatag aaggtactgg gaaaacaaaa 300

tagatcaggt tattattgaa acaaagtatt ttctgttgga tacctgaggc agagccagaa 360

aaaaaagttt tatttatttt attttttcta ggaaggcttt aaaaaatatt taacttacaa 420

agtttttgca cctcatttta cagtggtaaa agtttgatta attttaatat gaagttcctt 480

ttgccctata gcagatcaaa gttcagatga aaatgttcag agttaacaga ctctctggtt 540

gctaatgttg ttgaggtaga ggatctacaa agaaacagga cccccccccc attcattttt 600

aaaatgtttg gatttgttca tagtaaagat gatctttccc atctgtcggt acagttctgc 660

tcctctgtgt gtgtagtacc tgcagatctt accgcaccaa catctgttct tatacacagg 720

gcttttgttg ttgtaacact ctgactttaa aagtaagttg tttgctgatt tctgcactaa 780

ttatgcaatt tcattttttt tccccttcat ttcagccaaa tgtgtgtgtt cagtctgtgc 840

tttctaagta gttgggtatc agacgtgtta caaagaatgg ctggacatgt cttcttgact 900

cttccagaat ttgtgtggga ctttgcctga tattatgagt tctttccctt tgaagttaaa 960

gctctctttt aggttttgta ggatgtcatt tgatgagttg ttggttaaat ttctggaaga 1020

ggtctgagtt cttgttctaa gtcataagct aaaggaagct ttaaacttga ccaggagtta 1080

tgagtggggt gagcaaagcc ttggtgattg catttaggtt gaatgtgtgg cttgggagtc 1140

cttggggtgt gagcctgatt taaaattcag acatgtaact ctccaccact aaggcggata 1200

ttttttaaag tggcgactag agtctaaatt cctgactagg ctctaacgag aaactcctct 1260

ttccccag 1268

<210> 10
<211> 3676
<212> DNA
<213> Mus musculus

<400> 10
gttatgaaga cagtatggag tttccagacc acagcagaca tttgctgcag tgtctgagcg 60

agcagaggca ccagggcttt ctttgtgact gcactgttct ggtgggagat gcccagttcc 120

gggcacaccg agctgttctg gcttcatgca gcatgtattt ccacctcttt tacaaggacc 180

agctggacaa aagagacatt gttcatctga acagcgacat tgtgacagcc cccgctttcg 240

ctctcctgct tgaattcatg tacgaaggga aactccagtt caaagacttg cccattgagg 300

acgtgctagc agctgccagt tatctccaca tgtatgacat tgtcaaagtc tgcaaaaaga 360

agctgaaaga gaaagctacc acagaggcgg acagcaccaa aaaagaagaa gatgcttcaa 420

gttgttcgga taaagtcgag agcctctcag acggcagcag ccacatggca ggtgacctgc 480

ccagtgatga agatgaaggc gaagatgaca aattgaacat tctacccagc aaaagggact 540

tggcagctga gcctgggaac atgtggatgc gattgccctc agactcagca ggcatccccc 600

aggctggcgg agaggccgag ccacacgcca cagcagctgg aaaaacagta gccagcccct 660

gcagctcaac agagtctttg tcccagaggt ctgtcacctc cgtgagggat tcggcagatg 720

ttgactgtgt gctggacctg tctgtcaagt ccagcctttc aggagttgaa aatctgaaca 780

gctcttattt ctcttcacag gacgtgctga gaagcaacct ggtgcaggtg aaggtggaga 840

aagaggcgtc ctgtgatgag agtgatgttg gcactaatga ctatgacatg gaacatagca 900

ctgtgaaaga gagtgtgagc actaacaaca gggtacagta tgagccagcc cacctggctc 960

ctctgaggga ggactcggtc ttaagggaac tggatcggga ggacaaagcc agcgatgatg 1020

agatgatgac cccagagagc gagcgggtcc aggtggaagg gggcatggag aacagtctgc 1080

ttccctacgt ctccaatatc ctgagccccg caggccagat cttcatgtgc cccctgtgca 1140

acaaagtctt ccccagcccc cacatcctgc agatccacct gagcacgcac ttccgagagc 1200

aggatggcat ccgtagcaag cctgccgccg atgtcaatgt gcccacgtgc tccctgtgtg 1260

ggaagacttt ctcctgcatg tacaccctca agcgccacga gaggactcac tcgggggaga 1320

agccctacac gtgcacccaa tgcggcaaga gtttccagta ctcgcacaac ctgagccgtc 1380

atgccgtggt gcacacccgc gagaagcccc atgcctgcaa gtggtgcgag cgcaggttca 1440

cgcagtccgg agacctgtac agacacatcc gcaagttcca ctgtgagttg gtgaactcct 1500

tgtcggtcaa aagtgaagcg ctgagcttgc ccactgtcag agactggacc ttagaagata 1560

gctctcaaga actttggaaa taattttata tatatatata tataaataat atatatatat 1620

atatatacat atatatacat agatctctat atagttgtgg tacggtctaa aagcagtctt 1680

gtttcctgga actgaaaagt tgggatctta acttgttttt gcactttaga ataatagcat 1740

gagaatctca ctaatttagc attctgataa aagaaactag agcaagtcac aagtagagag 1800

ctgtctttct tttgagggtt aggccaagtt agccaataaa accttctcaa caaatggttc 1860

tgtcacaaac gctttcacat ggcttacttt tgtacacact gcattgtctt gagcgctttc 1920

cctgtgctcc acactgtttt gttttgtttt gttttcagag tagaaatccc ctccagttta 1980

ttagcctctt tatatgtctc aaattgcatg aatattttct ggctgttgga aacctgaatg 2040

cttttagacc caaatggaaa atttctgaaa tgctggatta tctattttta aacaagcagt 2100

tgacttaaaa ctttgtgtgg caacttctgg ttttctgatg gtccccagtg agagaaatct 2160

gacagtacac tgggtcactg ggactgtctc tgtgaaggtt tgcttgagat gaaaaaggat 2220

attgcagctt cagcagtgtt gaactgtgtg tttaaaatgt gaattactgt tcttgtatac 2280

tgtaattgat tacacggcct ggggggggtg tcaaagaact tgacaggttg tgttgatgct 2340

cttagttgag tcttgaaaag taaatattaa cgctacagaa atgcatgagt ttcaatatat 2400

tttttgtctt tgtttgcatt gtataacttt aacgagtgag tttaaaatta tttaatttcc 2460

ttagaaaaat agcaccattt gggaaaaaaa ctggtgttac gaagaacata aatgcactgt 2520

ctttattttt atttttattt tatataattt aaattgactt ttcccacgtc tttaagtttg 2580

aaactgttaa gctgaataaa aacttaagct gcaaattgat aacttcgcta cattacaagg 2640

aacatataaa tgtttacaaa cggcttaaag aattgcatgt gcagtgtgca ttcataccac 2700

acttgtaatt gcacagaacc catgccagct cagagtttag gtgtacacac gtacccagcc 2760

gagtgttttt agaataacta ctgcacaaat tgacaatagg tcataggtca ttctctctct 2820

ccctcctttt tgcttcccct ttctttttct cccctatttc ctttccttcc ccctaccccc 2880

taactcatga aaagattcta tggactgaaa aagccccagg ctgaaaggac tgaaccgcct 2940

tgattgacat ggggaggggg gttagtagac tgtgtatcgg cagcagaggc tgcagcccga 3000

tgtgtggttc tgatggccag cgcggggcag aagcgttttg cacactgttt gttttcctgt 3060

cttgcactta caaataaggt ctatgggagt agcatgggaa acagtggttg tttttccctt 3120

tttctttttt aattgctttt gtttaaaatt tgatcgcctt aactactgta aacatagcct 3180

atttttgtgc ttaagatact gaatggaaaa ctccattgtg tgttgctgga ctgttttgga 3240

aatatttggt caaatgtatg ttaatttggc tgtaatggca tttaaagcaa acaaacaaac 3300

aaacaaacaa agctgtgaaa atggccttgg agcattatct ttagttactt gaagagtttc 3360

tagttttttt ttttttaata cagtttatgt taaaataatc tttattaatc tagaggagac 3420

aatcaatgtc tgtggaagaa cggacttttt tggatttctt tttttaatgg tcattgtgag 3480

tgattgctct ttcttttact tagtttcaca ttcttccttt gttctaaatc ttagactgac 3540

atctagcttt gacaatcata gtatgtttta tttcctgagg gggaataact tataatgctg 3600

tttagttttg tactattggt gtgttggtga atttttaaac tgtgtgctaa ctgcaataaa 3660

ttatatgaac tgagaa 3676

<210> 11
<211> 3791
<212> RNA
<213> Homo sapiens

<220>
<221> CDS
<222> (70)..(1638)

<400> 11
gaggaggcgg aggacgcggc ggcggcgggg acgcggugac uuagggccgc uccguguuau 60

gaagacagu aug gag uuu cca gac cau agu aga cau uug cua cag ugu cug 111
Met Glu Phe Pro Asp His Ser Arg His Leu Leu Gln Cys Leu
1               5                   10

agc gag cag aga cac cag ggu uuu cuu ugu gac ugc acu guu cug gug 159
Ser Glu Gln Arg His Gln Gly Phe Leu Cys Asp Cys Thr Val Leu Val
15                  20                  25                  30

gga gau gcc cag uuc cga gcg cac cga gcu gua cug gcu uca ugc agc 207
Gly Asp Ala Gln Phe Arg Ala His Arg Ala Val Leu Ala Ser Cys Ser
                35                  40                  45

aug uau uuc cac cuc uuu uac aag gac cag cug gac aaa aga gac auu 255

            50                  55                  60

guu cau cug aac agc gac auu guu aca gcc ccc gcu uuc gcu cuc cug 303

        65                  70                  75

cuu gaa uuc aug uau gaa ggg aaa cuc cag uuc aaa gac uug ccc auu 351

    80                  85                  90

gaa gac gug cua gca gcu gcc agu uau cuc cac aug uau gac auu guc 399

95                  100                 105                 110

aaa guc ugc aaa aag aag cug aaa gag aaa gcc acc acg gag gca gac 447

                115                 120                 125

agc acc aaa aag gaa gaa gau gcu uca agu ugu ucg gac aaa guc gag 495

            130                 135                 140

agu cuc ucc gau ggc agc agc cac aua gca ggc gau uug ccc agu gau 543

        145                 150                 155

gaa gau gaa gga gaa gau gaa aaa uug aac auc cug ccc agc aaa agg 591

    160                 165                 170

gac uug gcg gcc gag ccu ggg aac aug ugg aug cga uug ccc uca gac 639

175                 180                 185                 190

uca gca ggc auc ccc cag gcu ggc gga gag gca gag cca cac gcc aca 687

                195                 200                 205

gca gcu gga aaa aca gua gcc agc ccc ugc agc uca aca gag ucu uug 735

            210                 215                 220

ucc cag agg ucu guc acc ucc gug agg gau ucg gca gau guu gac ugu 783

        225                 230                 235

gug cug gac cug ucu guc aag ucc agc cuu uca gga guu gaa aau cug 831

    240                 245                 250

aac agc ucu uau uuc ucu uca cag gac gug cug aga agc aac cug gug 879


255                 260                 265                 270

cag gug aag gug gag aaa gag gcu ucc ugu gau gag agu gau guu ggc       927

                275                 280                 285

acu aau gac uau gac aug gaa cau agc acu gug aaa gaa agu gug agc       975

            290                 295                 300

acu aau aac agg gua cag uau gag ccg gcc cau cug gcu ccc cug agg      1023

        305                 310                 315

gag gac ucg guc uug agg gag cug gac cgg gag gac aaa gcc agu gau      1071

    320                 325                 330

gau gag aug aug acc cca gag agc gag cgu guc cag gug gag gga ggc      1119

335                 340                 345                 350

aug gag agc agu cug cuc ccc uac guc ucc aac auc cug agc ccc gcg      1167

                355                 360                 365

ggc cag auc uuc aug ugc ccc cug ugc aac aag guc uuc ccc agc ccc      1215

            370                 375                 380

cac auc cug cag auc cac cug agc acg cac uuc cgc gag cag gac ggc      1263

        385                 390                 395

auc cgc agc aag ccc gcc gcc gau guc aac gug ccc acg ugc ucg cug      1311

    400                 405                 410

ugu ggg aag acu uuc ucu ugc aug uac acc cuc aag cgc cac gag agg      1359

415                 420                 425                 430

acu cac ucg ggg gag aag ccc uac aca ugc acc cag ugc ggc aag agc      1407

                435                 440                 445

uuc cag uac ucg cac aac cug agc cgc cau gcc gug gug cac acc cgc      1455

            450                 455                 460

gag aag ccg cac gcc ugc aag ugg ugc gag cgc agg uuc acg cag ucc      1503

        465                 470                 475

ggg gac cug uac aga cac auu cgc aag uuc cac ugu gag uug gug aac      1551

    480                 485                 490

ucc uug ucg guc aaa agc gaa gca cug agc uug ccu acu guc aga gac      1599

495                 500                 505                 510

ugg acc uua gaa gau agc ucu caa gaa cuu ugg aaa uaa uuuuauauau       1648


                515                 520

auauaaauaa uauauauaua uauacauaua uauaaauaga ucucuauaua guugugguac      1708

ggucuaaaag cagucuuguu uccuggaaau aaaaaguugg gauauuaacu uguuuuugca      1768

cuuuagaaua gcaugagaau cucacuaauu uagcauucug auaaaagaaa cuuuagagca      1828

agucagaaua gagagguguu uuuccuuuga ggggauaggg gaaguaagcc aauaagaacc      1888

uuuuaaacaa aucguccugu cacaaaaugc uuucauaugg cuuaauuuug ucaacacugc      1948

auugucuuuu gagcucuuuu uuccccccca acaaaguuuu uuuguuuuuu guuuuuuuuu      2008

uuaaguagaa auucccucca guuuuauuag ccucuuuaua ugucucaaau ugcaugaauu      2068

uuuucuggcu guuggaaacc ugaaugcuuu uagacccaaa uggaaaauuu cugaaaugcu      2128

ggauuaucua uuuuuaaaca agcaguugac uuaaaacuuu cuguggcaac uucugguuuu      2188

cugacaguuc ccagugagag aaaugcugaa aguacacugg gaucacuggg acacugucuu      2248

augaagguuu gcuugggaug aaaaaggaua uugcagcuuc agcaguguug aacugugugu      2308

uuaaaaaugu gaauuacugu uauuguauac uguaauugau uacaugggcu gggggggugu      2368

caaagaacuu gacagguugu guugaugcuc uuaguugagu cuugaaaagu aaauauuaac      2428

gcuacagaaa ugcaugaguu ucaauauauu uuuugucuuu guuugcauug uauaacuuua      2488

acgagugagu uuaaaauuau uuaauuuccu uagaaaaaua gcaccauuug gaaaaaaaaa      2548

cugguguuau gaagaacgua aaugcacugu uuuuauuuuu auuuuauaua auuuaaauug      2608

acuuucccac ugucuuuaag uugaaacugu uaagcugaau aaaaacuuaa gcugcaaauu      2668

gauaacuucg cuacauaaca aggaaaauau aaauguuuac aaacagcuua aagauuugca      2728

ugugcagugu gcauuuauaa caaacuucua auugcacaaa acccaugcca gcucagaguu      2788

uagguguaca cauuuaccca guugagcguu cuuagaauaa cuacugcaca aguugacaau      2848

aggucguucu cucuuuuuuu uuguuugcuc ccuuuuucuu uuucuccccu uccuccuuac      2908

ccucccuccc uuacucuccc cccccaccac cacccuccac ccccaacuca ugaaaagauu      2968

cuauggacug aaaaagcccc aggcugaaag gacuggacug ccuugauuga cauggggaag      3028

gggguuagua gacuaugugg auugcggcag cagaggcugc agccuaacgu gugguuuuaa      3088

ugaccagcac gcaaggcaaa agcauuuugc acaguguuug uuuuccuguc uugcacuuac      3148

aaauaagguc uaugggagua gcauggaaaa cguuugcugu uuuucccuuu uuuuuuuaau      3208

ugcuuuuguu uaaaauuuga ucgccuuaac uacuguaaac auagccuauu uuugugcuua      3268

agauacugaa uggaaaacuc cauugugugu ugcuggacug uuuuggaaau auuugguuaa      3328

auguguguua auuuggcugu aauggcauuu aaagcaaaca aacaaacaaa aaaagcugug      3388

aaaauggccu uggagcauua ucuuuaguua cuugaagagu uucuaguuuu uuuaaaauac      3448

agtuuaugu aaaauaauuu uuauuaauuu agagaagaca aucaaugucu gugagaaaac 3508

ggacuuucuu uuggauuuuc uuuuuguggu cauugugagu gauugcuuuu uccuuuucuu 3568

aguuucacau ucuuccuuug uucuaaaacu uagacugaca ucuagcuuug acaaucauag 3628

uauguuuuau uuuccugagg gggaauaacu uauaaugcug uuuaguuuug uacuauuggu 3688

guguugguga auuuuuaaac ugugugcuaa cugcaauaaa uuauaugaac ugagaaaaaa 3748

aaaaaaaaaa aaaaaaaaaa aaaaaaaaaa aaaaaaaaaa aaa 3791

<210> 12
<211> 5905
<212> RNA
<213> Homo sapiens

<220>
<221> CDS
<222> (2231)..(3799)

<400> 12
guggcgccuc ucgggugcgg agggcggcgg gcugggggag ggggcuuggg agcagccgca 60

gcugcagugg cgcccgggac ggccgcggcu ccggggaccg gugcagcugc aguggcgccc 120

gggggagggg gccggcgggg gagggggccc ucccgggcug gugcaguugc ccggaggccc 180

cgggggcucg cgcgucgcgg caucggggaa gcgguagcaa ccacucuccu cccgcuggcu 240

gcuucuuacg acuuuucuuu uguuguugcc gugacccggg ucuguuuucu ccucuguugu 300

uuaagguguu uuccugcugc accggcucug cgcccuccac cuccuucccu gugguuuuaa 360

ccuuccucuu uccccgugug uuuuaugcac ggcgaacuac guaaagauuu gcauugcuuc 420

cccacucggc acccccgccc ccaccucucu gaaaaacaaa accccaaccu cacaaaaccu 480

cuauggaucc gcgguagcga gacgugaagg gguugauuug uggaguggga guugcuggcc 540

cauugcggug cccgggacuc auuaaacugu cacucacccc ccaccccacu ggguaaaugg 600

ggugauuaau gucugauaua uuggaauggg ucgagggcau uuguggagaa uagguggugu 660

ggcugggaag aaauugaucu cccgaggaug ucuccuggac uaaguguuca uaauuauguc 720

acucaccgcg aaguuggaga aaguuagguu ugugacuuug ggaaagcgag aaggacagga 780

cggagucguc ggguuuuguu acucuuuaac ucuccgccac ggcuuucuaa ugggaacgaa 840

gugguuauca ccagggcugg gacgcuggcg ccucaggccc caguuccuuu ucuaccccuc 900

gcccucccaa gagucugagg agauguauuu gacauucaga gaugucgcag cacccugccc 960

gguaccgguc agcccagccc gggucgcguu acaguuucau cagcuauuag aaaagaccca 1020

caaacuggcu gaaaaguucu caaacuuacu agaccuggcu uccccccaca caaagauuuu 1080

cuugugauca uaaacauuag agugaccccc ccccaaagaa aaaaaagauu guuaagggac 1140

acagaucaag gggaaggcaa gguucugcuu uuucugagca gaaagaucag auaugcaggu 1200

gcagcauuca uuuuaaguga cuggagcagg uuguaucagu uuauuaauug gucucguuga 1260

acugaaguac uuuccaggau uucagaaagu gcaguuucag cuuguuggua ccgagaauug 1320

aaauuugcau ugacuguggc aguguaagaa auuuuucuga gaagguaguu guagauaagu 1380

aauuacauaa gagugaaaua auaaggugaa ccagucugaa guaauaguuc ugaacaaguc 1440

ugccaacauu ugagacaugu auaccauuau cucagcagaa aaaaauaaaa aagugcggua 1500

auaauguuga aaugggacug gagaugucaa auggguuggu uuaaaaauau cccuaaacug 1560

cuuuagaaac uaaacguugg aguugaauaa aagugaaucu ggauuuccug aauagcaccg 1620

uggcuugagu gugcugucag uauguaagcc aguccaccaa cuuaagacag aaguguugcu 1680

auuccauaaa uacaguagaa cuccauaugc ugaguuuuuc aauuucuucu guuagaaagu 1740

uuggaaaaaa aggaagacuu ugaaguauuu uguuuauacu aacagaagag aaaaguucuc 1800

cuaagaaaaa gcugguggca ggaguguaga ggaguagcau auggcauuaa aaggagcaca 1860

gcuggaggua gcauuuccau cugaacauga ugucagagca gcugacagcc ugccaucccu 1920

cagccuuuua uucuaacaca cagacaggga guuagugugu gcggaucugu gguggagaag 1980

guaucucauu ccucucuaac aucaucucca cuuugcaucu gucucucuua gucugcuuuu 2040

uuuccaccga uguaacagac cuggagccag caggacucag aggaaaggac uuaaucaggu 2100

uuaugugucc uaaagcuccu ucugaucucg uacaugugug cuauaccugc aaaucugaag 2160

gcauuaacau cuguuuuuau acacagggcu uuuguuguaa cacccugacu uuaaaaguua 2220

ugaagacagu aug gag uuu cca gac cau agu aga cau uug cua cag ugu 2269
Met Glu Phe Pro Asp His Ser Arg His Leu Leu Gln Cys
1               5                   10

cug agc gag cag aga cac cag ggu uuu cuu ugu gac ugc acu guu cug 2317
Leu Ser Glu Gln Arg His Gln Gly Phe Leu Cys Asp Cys Thr Val Leu
    15                  20                  25

gug gga gau gcc cag uuc cga gcg cac cga gcu gua cug gcu uca ugc 2365
Val Gly Asp Ala Gln Phe Arg Ala His Arg Ala Val Leu Ala Ser Cys
30                  35                  40                  45

agc aug uau uuc cac cuc uuu uac aag gac cag cug gac aaa aga gac 2413
Ser Met Tyr Phe His Leu Phe Tyr Lys Asp Gln Leu Asp Lys Arg Asp
                50                  55                  60

auu guu cau cug aac agc gac auu guu aca gcc ccc gcu uuc gcu cuc 2461
Ile Val His Leu Asn Ser Asp Ile Val Thr Ala Pro Ala Phe Ala Leu
            65                  70                  75

cug cuu gaa uuc aug uau gaa ggg aaa cuc cag uuc aaa gac uug ccc 2509
Leu Leu Glu Phe Met Tyr Glu Gly Lys Leu Gln Phe Lys Asp Leu Pro
        80                  85                  90

auu gaa gac gug cua gca gcu gcc agu uau cuc cac aug uau gac auu 2557
Ile Glu Asp Val Leu Ala Ala Ala Ser Tyr Leu His Met Tyr Asp Ile
    95                  100                 105

gtc aaa gtc tgc aaa aag aag ctg aaa gag aaa gcc acc acg gag gca 2605
Val Lys Val Cys Lys Lys Lys Leu Lys Glu Lys Ala Thr Thr Glu Ala
110                 115                 120                 125

gac agc acc aaa aag gaa gaa gat gct tca agt tgt tcg gac aaa gtc 2653
Asp Ser Thr Lys Lys Glu Glu Asp Ala Ser Ser Cys Ser Asp Lys Val
                130                 135                 140

gag agt ctc tcc gat ggc agc agc cac ata gca ggc gat ttg ccc agt 2701
Glu Ser Leu Ser Asp Gly Ser Ser His Ile Ala Gly Asp Leu Pro Ser
            145                 150                 155

gat gaa gat gaa gga gaa gat gaa aaa ttg aac atc ctg ccc agc aaa 2749
Asp Glu Asp Glu Gly Glu Asp Glu Lys Leu Asn Ile Leu Pro Ser Lys
        160                 165                 170

agg gac ttg gcg gcc gag cct ggg aac atg tgg atg cga ttg ccc tca 2797
Arg Asp Leu Ala Ala Glu Pro Gly Asn Met Trp Met Arg Leu Pro Ser
    175                 180                 185

gac tca gca ggc atc ccc cag gct ggc gga gag gca gag cca cac gcc 2845
Asp Ser Ala Gly Ile Pro Gln Ala Gly Gly Glu Ala Glu Pro His Ala
190                 195                 200                 205

aca gca gct gga aaa aca gta gcc agc ccc tgc agc tca aca gag tct 2893
Thr Ala Ala Gly Lys Thr Val Ala Ser Pro Cys Ser Ser Thr Glu Ser
                210                 215                 220

ttg tcc cag agg tct gtc acc tcc gtg agg gat tcg gca gat gtt gac 2941
Leu Ser Gln Arg Ser Val Thr Ser Val Arg Asp Ser Ala Asp Val Asp
            225                 230                 235

tgt gtg ctg gac ctg tct gtc aag tcc agc ctt tca gga gtt gaa aat 2989
Cys Val Leu Asp Leu Ser Val Lys Ser Ser Leu Ser Gly Val Glu Asn
        240                 245                 250

ctg aac agc tct tat ttc tct tca cag gac gtg ctg aga agc aac ctg 3037
Leu Asn Ser Ser Tyr Phe Ser Ser Gln Asp Val Leu Arg Ser Asn Leu
    255                 260                 265

gtg cag gtg aag gtg gag aaa gag gct tcc tgt gat gag agt gat gtt 3085
Val Gln Val Lys Val Glu Lys Glu Ala Ser Cys Asp Glu Ser Asp Val
270                 275                 280                 285

ggc act aat gac tat gac atg gaa cat agc act gtg aaa gaa agt gtg 3133
Gly Thr Asn Asp Tyr Asp Met Glu His Ser Thr Val Lys Glu Ser Val
                290                 295                 300

agc act aat aac agg gta cag tat gag ccg gcc cat ctg gct ccc ctg 3181
Ser Thr Asn Asn Arg Val Gln Tyr Glu Pro Ala His Leu Ala Pro Leu
            305                 310                 315

agg gag gac tcg gtc ttg agg gag ctg gac cgg gag gac aaa gcc agt 3229
Arg Glu Asp Ser Val Leu Arg Glu Leu Asp Arg Glu Asp Lys Ala Ser
        320                 325                 330

gat gat gag atg atg acc cca gag agc gag cgt gtc cag gtg gag gga 3277
Asp Asp Glu Met Met Thr Pro Glu Ser Glu Arg Val Gln Val Glu Gly
    335                 340                 345

ggc atg gag agc agt ctg ctc ccc tac gtc tcc aac atc ctg agc ccc 3325
Gly Met Glu Ser Ser Leu Leu Pro Tyr Val Ser Asn Ile Leu Ser Pro
350                 355                 360                 365

gcg ggc cag auc uuc aug ugc ccc cug ugc aac aag guc uuc ccc agc 3373
Ala Gly Gln Ile Phe Met Cys Pro Leu Cys Asn Lys Val Phe Pro Ser
                370                 375                 380

ccc cac auc cug cag auc cac cug agc acg cac uuc cgc gag cag gac 3421
Pro His Ile Leu Gln Ile His Leu Ser Thr His Phe Arg Glu Gln Asp
            385                 390                 395

ggc auc cgc agc aag ccc gcc gcc gau guc aac gug ccc acg ugc ucg 3469
Gly Ile Arg Ser Lys Pro Ala Ala Asp Val Asn Val Pro Thr Cys Ser
        400                 405                 410

cug ugu ggg aag acu uuc ucu ugc aug uac acc cuc aag cgc cac gag 3517
Leu Cys Gly Lys Thr Phe Ser Cys Met Tyr Thr Leu Lys Arg His Glu
    415                 420                 425

agg acu cac ucg ggg gag aag ccc uac aca ugc acc cag ugc ggc aag 3565
Arg Thr His Ser Gly Glu Lys Pro Tyr Thr Cys Thr Gln Cys Gly Lys
430                 435                 440                 445

agc uuc cag uac ucg cac aac cug agc cgc cau gcc gug gug cac acc 3613
Ser Phe Gln Tyr Ser His Asn Leu Ser Arg His Ala Val Val His Thr
                450                 455                 460

cgc gag aag ccg cac gcc ugc aag ugg ugc gag cgc agg uuc acg cag 3661
Arg Glu Lys Pro His Ala Cys Lys Trp Cys Glu Arg Arg Phe Thr Gln
            465                 470                 475

ucc ggg gac cug uac aga cac auu cgc aag uuc cac ugu gag uug gug 3709
Ser Gly Asp Leu Tyr Arg His Ile Arg Lys Phe His Cys Glu Leu Val
        480                 485                 490

aac ucc uug ucg guc aaa agc gaa gca cug agc uug ccu acu guc aga 3757
Asn Ser Leu Ser Val Lys Ser Glu Ala Leu Ser Leu Pro Thr Val Arg
    495                 500                 505

gac ugg acc uua gaa gau agc ucu caa gaa cuu ugg aaa uaa 3799
Asp Trp Thr Leu Glu Asp Ser Ser Gln Glu Leu Trp Lys
510                 515                 520

uuuuauauau auauaaauaa uauauauaua uauacauaua uauaaauaga ucucuauaua 3859

guugugguac ggucuaaaag cagucuuguu uccuggaaau aaaaaguugg gauauuaacu 3919

uguuuuugca cuuuagaaua gcaugagaau cucacuaauu uagcauucug auaaaagaaa 3979

cuuuagagca agucagaaua gagagguguu uuuccuuuga ggggauaggg gaaguaagcc 4039

aauaagaacc uuuuaaacaa aucguccugu cacaaaaugc uuucauaugg cuuaauuuug 4099

ucaacacugc auugucuuuu gagcucuuuu uuccccccca acaaaguuuu uuuguuuuuu 4159

guuuuuuuuu uuaaguagaa auucccucca guuuuauuag ccucuuuaua ugucucaaau 4219

ugcaugaauu uuuucuggcu guuggaaacc ugaaugcuuu uagacccaaa uggaaaauuu 4279

cugaaaugcu ggauuaucua uuuuuaaaca agcaguugac uuaaaacuuu cuguggcaac 4339

uucugguuuu cugacaguuc ccagugagag aaaugcugaa aguacacugg gaucacuggg 4399

acacugucuu augaagguuu gcuugggaug aaaaaggaua uugcagcuuc agcaguguug 4459

aacugugugu uuaaaaaugu gaauuacugu uauuguauac uguaauugau uacaugggcu 4519

gggggggugu caaagaacuu gacagguugu guugaugcuc uuaguugagu cuugaaaagu 4579

aaauauuaac gcuacagaaa ugcaugaguu ucaauauauu uuuugucuuu guuugcauug 4639

uauaacuuua acgagugagu uuaaaauuau uuaauuuccu uagaaaaaua acaccauuug 4699

gaaaaaaaaa cugguguuau gaagaacgua aaugcacugu uuuuauuuuu auuuuauaua 4759

auuuaaauug acuuucccac ugucuuuaag uugaaacugu uaagcugaau aaaaacuuaa 4819

gcugcaaauu gauaacuucg cuacauaaca aggaaaauau aaauguuuac aaacagcuua 4879

aagauuugca ugugcagugu gcauuuauaa caaacuucua auugcacaaa acccaugcca 4939

gcucagaguu uagguguaca cauuuaccca guugagcguu cuuagaauaa cuacugcaca 4999

aguugacaau aggucguucu cucuuuuuuu uuguuugcuc ccuuuuucuu uuucuccccu 5059

uccuccuuac ccucccuccc uuacucuccc cccccaccac cacccuccac ccccaacuca 5119

ugaaaagauu cuauggacug aaaaagcccc aggcugaaag gacuggacug ccuugauuga 5179

cauggggaag gggguuagua gacuaugugg auugcggcag cagaggcugc agccuaacgu 5239

gugguuuuaa ugaccagcac gcaaggcaaa agcauuuugc acaguguuug uuuuccuguc 5299

uugcacuuac aaauaagguc uaugggagua gcauggaaaa cguuugcugu uuuucccuuu 5359

uuuuuuuaau ugcuuuuguu uaaaauuuga ucgccuuaac uacuguaaac auagccuauu 5419

uuugugcuua agauacugaa uggaaaacuc cauugugugu ugcuggacug uuuuggaaau 5479

auuugguuaa auguguguua auuuggcugu aauggcauuu aaagcaaaca aacaaacaaa 5539

aaaagcugug aaaauggccu uggagcauua ucuuuaguua cuugaagagu uucuaguuuu 5599

uuuaaaauac aguuuauguu aaaauaauuu uuauuaauuu agagaagaca aucaaugucu 5659

gugagaaaac ggacuuucuu uuggauuuuc uuuuuguggu cauugugagu gauugcuuuu 5719

uccuuuucuu aguuucacau ucuuccuuug uucuaaaacu uagacugaca ucuagcuuug 5779

acaaucauag uauguuuuau uuuccugagg gggaauaacu uauaaugcug uuuaguuuug 5839

uacuauuggu guguugguga auuuuuaaac ugugugcuaa cugcaauaaa uuauaugaac 5899

ugagaa 5905

<210> 13
<211> 3922
<212> RNA
<213> Homo sapiens

<220>
<221> CDS
<222> (174)..(1769)

<400> 13
auucuaacac acagacaggg aguuagugug ugcggaucug ugguggagaa gguaucucau 60
uccucucuaa caucaucucc acuuugcauc ugucucucuu agucugcuuu uuuuccaccg 120
auguaacaga ccuggagcca gcaggacuca gaggaaagga cuuaaucagg uuu aug 176
ugu ccu aaa ggu uau gaa gac agu aug gag uuu cca gac cau agu aga 224
cau uug cua cag ugu cug agc gag cag aga cac cag ggu uuu cuu ugu 272
gac ugc acu guu cug gug gga gau gcc cag uuc cga gcg cac cga gcu 320
gua cug gcu uca ugc agc aug uau uuc cac cuc uuu uac aag gac cag 368
cug gac aaa aga gac auu guu cau cug aac agc gac auu guu aca gcc 416
ccc gcu uuc gcu cuc cug cuu gaa uuc aug uau gaa ggg aaa cuc cag 464
uuc aaa gac uug ccc auu gaa gac gug cua gca gcu gcc agu uau cuc 512
cac aug uau gac auu guc aaa guc ugc aaa aag aag cug aaa gag aaa 560
gcc acc acg gag gca gac agc acc aaa aag gaa gaa gau gcu uca agu 608
ugu ucg gac aaa guc gag agu cuc ucc gau ggc agc agc cac aua gca 656
ggc gau uug ccc agu gau gaa gau gaa gga gaa gau gaa aaa uug aac 704
auc cug ccc agc aaa agg gac uug gcg gcc gag ccu ggg aac aug ugg 752
aug cga uug ccc uca gac uca gca ggc auc ccc cag gcu ggc gga gag 800
gca gag cca cac gcc aca gca gcu gga aaa aca gua gcc agc ccc ugc 848
agc uca aca gag ucu uug ucc cag agg ucu guc acc ucc gug agg gau 896
ucg gca gau guu gac ugu gug cug gac cug ucu guc aag ucc agc cuu 944
uca gga guu gaa aau cug aac agc ucu uau uuc ucu uca cag gac gug 992
cug aga agc aac cug gug cag gug aag gug gag aaa gag gcu ucc ugu 1040
gau gag agu gau guu ggc acu aau gac uau gac aug gaa cau agc acu 1088
gug aaa gaa agu gug agc acu aau aac agg gua cag uau gag ccg gcc 1136
cau cug gcu ccc cug agg gag gac ucg guc uug agg gag cug gac cgg 1184
gag gac aaa gcc agu gau gau gag aug aug acc cca gag agc gag cgu 1232
guc cag gug gag gga ggc aug gag agc agu cug cuc ccc uac guc ucc 1280
aac auc cug agc ccc gcg ggc cag auc uuc aug ugc ccc cug ugc aac 1328
aag guc uuc ccc agc ccc cac auc cug cag auc cac cug agc acg cac 1376
uuc cgc gag cag gac ggc auc cgc agc aag ccc gcc gcc gau guc aac 1424
gug ccc acg ugc ucg cug ugu ggg aag acu uuc ucu ugc aug uac acc 1472
cuc aag cgc cac gag agg acu cac ucg ggg gag aag ccc uac aca ugc 1520
acc cag ugc ggc aag agc uuc cag uac ucg cac aac cug agc cgc cau 1568
gcc gug gug cac acc cgc gag aag ccg cac gcc ugc aag ugg ugc gag 1616
cgc agg uuc acg cag ucc ggg gac cug uac aga cac auu cgc aag uuc 1664
cac ugu gag uug gug aac ucc uug ucg guc aaa agc gaa gca cug agc 1712
uug ccu acu guc aga gac ugg acc uua gaa gau agc ucu caa gaa cuu 1760
ugg aaa uaa uuuuauauau auauaaauaa uauauauaua uauacauaua 1809
uauaaauaga ucucuauaua guugugguac ggucuaaaag cagucuuguu uccuggaaau 1869
aaaaaguugg gauauuaacu uguuuuugca cuuuagaaua gcaugagaau cucacuaauu 1929
uagcauucug auaaaagaaa cuuuagagca agucagaaua gagagguguu uuuccuuuga 1989
ggggauaggg gaaguaagcc aauaagaacc uuuuaaacaa aucguccugu cacaaaaugc 2049
uuucauaugg cuuaauuuug ucaacacugc auugucuuuu gagcucuuuu uuccccccca 2109
acaaaguuuu uuuguuuuuu guuuuuuuuu uuaaguagaa auucccucca guuuuauuag 2169
ccucuuuaua ugucucaaau ugcaugaauu uuuucuggcu guuggaaacc ugaaugcuuu 2229
uagacccaaa uggaaaauuu cugaaaugcu ggauuaucua uuuuuaaaca agcaguugac 2289
uuaaaacuuu cuguggcaac uucugguuuu cugacaguuc ccagugagag aaaugcugaa 2349
aguacacugg gaucacuggg acacugucuu augaagguuu gcuugggaug aaaaaggaua 2409
uugcagcuuc agcaguguug aacugugugu uuaaaaaugu gaauuacugu uauuguauac 2469
uguaauugau uacaugggcu gggggggugu caaagaacuu gacagguugu guugaugcuc 2529
uuaguugagu cuugaaaagu aaauauuaac gcuacagaaa ugcaugaguu ucaauauauu 2589
uuuugucuuu guuugcauug uauaacuuua acgagugagu uuaaaauuau uuaauuuccu 2649
uagaaaaaua gcaccauuug gaaaaaaaaa cugguguuau gaagaacgua aaugcacugu 2709
uuuuauuuuu auuuuauaua auuuaaauug acuuucccac ugucuuuaag uugaaacugu 2769
uaagcugaau aaaaacuuaa gcugcaaauu gauaacuucg cuacauaaca aggaaaauau 2829
aaauguuuac aaacagcuua aagauuugca ugugcagugu gcauuuauaa caaacuucua 2889
auugcacaaa acccaugcca gcucagaguu uagguguaca cauuuaccca guugagcguu 2949
cuuagaauaa cuacugcaca aguugacaau aggucguucu cucuuuuuuu uuguuugcuc 3009
ccuuuuucuu uuucuccccu uccuccuuac ccucccuccc uuacucuccc cccccaccac 3069
cacccuccac ccccaacuca ugaaaagauu cuauggacug aaaaagcccc aggcugaaag 3129
gacuggacug ccuugauuga cauggggaag ggguuaguaa gacuaugugg auugcggcag 3189
cagaggcugc agccuaacgu gugguuuuaa ugaccagcac gcaaggcaaa agcauuuugc 3249
acaguguuug uuuuccuguc uugcacuuac aaauaagguc uaugggagua gcauggaaaa 3309
cguuugcugu uuuucccuuu uuuuuuuaau ugcuuuuguu uaaaauuuga ucgccuuaac 3369
uacuguaaac auagccuauu uuugugcuua agauacugaa uggaaaacuc cauugugugu 3429
ugcuggacug uuuuggaaau auuugguuaa augugguuua auuuggcugu aauggcauuu 3489
aaagcaaaca aacaaacaaa aaaagcugug aaaauggccu uggagcauua ucuuuaguua 3549
cuugaagagu uucuaguuuu uuuaaaauac aguuuauguu aaaauaauuu uuauuaauuu 3609
agagaagaca aucaaugucu gugagaaaac ggacuuucuu uuggauuuuc uuuuuguggu 3669
cauugugagu gauugcuuuu uccuuuucuu aguuucacau ucuuccuuug uucuaaaacu 3729
uagacugaca ucuagcuuug acaaucauag uauguuuuau uuuccugagg gggaauaacu 3789
uauaaugcug uuuaguuuug uacuauuggu guguugguga auuuuuaaac ugugugcuaa 3849
cugcaauaaa uuauaugaac ugagaaaaaa aaaaaaaaaa aaaaaaaaaa aaaaaaaaaa 3909
aaaaaaaaaa aaa 3922

<210> 14
<211> 3805
<212> RNA
<213> Homo sapiens

<220>
<221> CDS
<222> (131)..(1699)

<400> 14
   agucugcuuu uuuuccaccg auguaacaga ccuggagcca gcaggacuca gaggaaagga 60
   cuuaaucagg uuuauguguc cuaaagggcu uuuguuguaa cacccugacu uuaaaaguua 120
   ugaagacagu aug gag uuu cca gac cau agu aga cau uug cua cag ugu 169
   Met Glu Phe Pro Asp His Ser Arg His Leu Leu Gln Cys
   1 5 10
   cug agc gag cag aga cac cag ggu uuu cuu ugu gac ugc acu guu cug 217
   Leu Ser Glu Gln Arg His Gln Gly Phe Leu Cys Asp Cys Thr Val Leu
   15 20 25
   gug gga gau gcc cag uuc cga gcg cac cga gcu gua cug gcu uca ugc 265
   Val Gly Asp Ala Gln Phe Arg Ala His Arg Ala Val Leu Ala Ser Cys
   30 35 40 45
   agc aug uau uuc cac cuc uuu uac aag gac cag cug gac aaa aga gac 313
   Ser Met Tyr Phe His Leu Phe Tyr Lys Asp Gln Leu Asp Lys Arg Asp
   50 55 60
   auu guu cau cug aac agc gac auu guu aca gcc ccc gcu uuc gcu cuc 361
   Ile Val His Leu Asn Ser Asp Ile Val Thr Ala Pro Ala Phe Ala Leu
   65 70 75
   cug cuu gaa uuc aug uau gaa ggg aaa cuc cag uuc aaa gac uug ccc 409
   Leu Leu Glu Phe Met Tyr Glu Gly Lys Leu Gln Phe Lys Asp Leu Pro
           80                  85                  90
   auu gaa gac gug cua gca gcu gcc agu uau cuc cac aug uau gac auu        457
   Ile Glu Asp Val Leu Ala Ala Ala Ser Tyr Leu His Met Tyr Asp Ile
       95                  100                 105
   guc aaa guc ugc aaa aag aag cug aaa gag aaa gcc acc acg gag gca        505
   Val Lys Val Cys Lys Lys Lys Leu Lys Glu Lys Ala Thr Thr Glu Ala
   110                 115                 120                 125
   gac agc acc aaa aag gaa gaa gau gcu uca agu ugu ucg gac aaa guc        553
   Asp Ser Thr Lys Lys Glu Glu Asp Ala Ser Ser Cys Ser Asp Lys Val
                   130                 135                 140
   gag agu cuc ucc gau ggc agc agc cac aua gca ggc gau uug ccc agu        601
   Glu Ser Leu Ser Asp Gly Ser Ser His Ile Ala Gly Asp Leu Pro Ser
               145                 150                 155
   gau gaa gau gaa gga gaa gau gaa aaa uug aac auc cug ccc agc aaa        649
   Asp Glu Asp Glu Gly Glu Asp Glu Lys Leu Asn Ile Leu Pro Ser Lys
           160                 165                 170
   agg gac uug gcg gcc gag ccu ggg aac aug ugg aug cga uug ccc uca        697
   Arg Asp Leu Ala Ala Glu Pro Gly Asn Met Trp Met Arg Leu Pro Ser
       175                 180                 185
   gac uca gca ggc auc ccc cag gcu ggc gga gag gca gag cca cac gcc        745
   Asp Ser Ala Gly Ile Pro Gln Ala Gly Gly Glu Ala Glu Pro His Ala
   190                 195                 200                 205
   aca gca gcu gga aaa aca gua gcc agc ccc ugc agc uca aca gag ucu        793
   Thr Ala Ala Gly Lys Thr Val Ala Ser Pro Cys Ser Ser Thr Glu Ser
                   210                 215                 220
   uug ucc cag agg ucu guc acc ucc gug agg gau ucg gca gau guu gac        841
   Leu Ser Gln Arg Ser Val Thr Ser Val Arg Asp Ser Ala Asp Val Asp
               225                 230                 235
   ugu gug cug gac cug ucu guc aag ucc agc cuu uca gga guu gaa aau        889
   Cys Val Leu Asp Leu Ser Val Lys Ser Ser Leu Ser Gly Val Glu Asn
           240                 245                 250
   cug aac agc ucu uau uuc ucu uca cag gac gug cug aga agc aac cug        937
   Leu Asn Ser Ser Tyr Phe Ser Ser Gln Asp Val Leu Arg Ser Asn Leu
       255                 260                 265
   gug cag gug aag gug gag aaa gag gcu ucc ugu gau gag agu gau guu        985
   Val Gln Val Lys Val Glu Lys Glu Ala Ser Cys Asp Glu Ser Asp Val
   270                 275                 280                 285
   ggc acu aau gac uau gac aug gaa cau agc acu gug aaa gaa agu gug       1033
   Gly Thr Asn Asp Tyr Asp Met Glu His Ser Thr Val Lys Glu Ser Val
                   290                 295                 300
   agc acu aau aac agg gua cag uau gag ccg gcc cau cug gcu ccc cug       1081
   Ser Thr Asn Asn Arg Val Gln Tyr Glu Pro Ala His Leu Ala Pro Leu
               305                 310                 315
   agg gag gac ucg guc uug agg gag cug gac cgg gag gac aaa gcc agu       1129
   Arg Glu Asp Ser Val Leu Arg Glu Leu Asp Arg Glu Asp Lys Ala Ser
           320                 325                 330
   gau gau gag aug aug acc cca gag agc gag cgu guc cag gug gag gga 1177
   Asp Asp Glu Met Met Thr Pro Glu Ser Glu Arg Val Gln Val Glu Gly
   335 340 345
   ggc aug gag agc agu cug cuc ccc uac guc ucc aac auc cug agc ccc 1225
   Gly Met Glu Ser Ser Leu Leu Pro Tyr Val Ser Asn Ile Leu Ser Pro
   350 355 360 365
   gcg ggc cag auc uuc aug ugc ccc cug ugc aac aag guc uuc ccc agc 1273
   Ala Gly Gln Ile Phe Met Cys Pro Leu Cys Asn Lys Val Phe Pro Ser
   370 375 380
   ccc cac auc cug cag auc cac cug agc acg cac uuc cgc gag cag gac 1321
   Pro His Ile Leu Gln Ile His Leu Ser Thr His Phe Arg Glu Gln Asp
   385 390 395
   ggc auc cgc agc aag ccc gcc gcc gau guc aac gug ccc acg ugc ucg 1369
   Gly Ile Arg Ser Lys Pro Ala Ala Asp Val Asn Val Pro Thr Cys Ser
   400 405 410
   cug ugu ggg aag acu uuc ucu ugc aug uac acc cuc aag cgc cac gag 1417
   Leu Cys Gly Lys Thr Phe Ser Cys Met Tyr Thr Leu Lys Arg His Glu
   415 420 425
   agg acu cac ucg ggg gag aag ccc uac aca ugc acc cag ugc ggc aag 1465
   Arg Thr His Ser Gly Glu Lys Pro Tyr Thr Cys Thr Gln Cys Gly Lys
   430 435 440 445
   agc uuc cag uac ucg cac aac cug agc cgc cau gcc gug gug cac acc 1513
   Ser Phe Gln Tyr Ser His Asn Leu Ser Arg His Ala Val Val His Thr
   450 455 460
   cgc gag aag ccg cac gcc ugc aag ugg ugc gag cgc agg uuc acg cag 1561
   Arg Glu Lys Pro His Ala Cys Lys Trp Cys Glu Arg Arg Phe Thr Gln
   465 470 475
   ucc ggg gac cug uac aga cac auu cgc aag uuc cac ugu gag uug gug 1609
   Ser Gly Asp Leu Tyr Arg His Ile Arg Lys Phe His Cys Glu Leu Val
   480 485 490
   aac ucc uug ucg guc aaa agc gaa gca cug agc uug ccu acu guc aga 1657
   Asn Ser Leu Ser Val Lys Ser Glu Ala Leu Ser Leu Pro Thr Val Arg
   495 500 505
   gac ugg acc uua gaa gau agc ucu caa gaa cuu ugg aaa uaa 1699
   Asp Trp Thr Leu Glu Asp Ser Ser Gln Glu Leu Trp Lys
   510 515 520
   uuuuauauau auauaaauaa uauauauaua uauacauaua uauaaauaga ucucuauaua 1759
   guugugguac ggucuaaaag cagucuuguu uccuggaaau aaaaaguugg gauauuaacu 1819
   uguuuuugca cuuuagaaua gcaugagaau cucacuaauu uagcauucug auaaaagaaa 1879
   cuuuagagca agucagaaua gagagguguu uuuccuuuga ggggauaggg gaaguaagcc 1939
   aauaagaacc uuuuaaacaa aucguccugu cacaaaaugc uuucauaugg cuuaauuuug 1999
   ucaacacugc auugucuuuu gagcucuuuu uuccccccca acaaaguuuu uuuguuuuuu 2059
   guuuuuuuuu uuaaguagaa auucccucca guuuuauuag ccucuuuaua ugucucaaau 2119
   ugcaugaauu uuuucuggcu guuggaaacc ugaaugcuuu uagacccaaa uggaaaauuu 2179
   cugaaaugcu ggauuaucua uuuuuaaaca agcaguugac uuaaaacuuu cuguggcaac 2239
   uucugguuuu cugacaguuc ccagugagag aaaugcugaa aguacacugg gaucacuggg 2299
   acacugucuu augaagguuu gcuugggaug aaaaaggaua uugcagcuuc agcaguguug 2359
   aacugugugu uuaaaaaugu gaauuacugu uauuguauac uguaauugau uacaugggcu 2419
   gggggggugu caaagaacuu gacagguugu guugaugcuc uuaguugagu cuugaaaagu 2479
   aaauauuaac gcuacagaaa ugcaugaguu ucaauauauu uuuugucuuu guuugcauug 2539
   uauaacuuua acgagugagu uuaaaauuau uuaauuuccu uagaaaaaua acaccauuug 2599
   gaaaaaaaaa cugguguuau gaagaacgua aaugcacugu uuuuauuuuu auuuuauaua 2659
   auuuaaauug acuuucccac ugucuuuaag uugaaacugu uaagcugaau aaaaacuuaa 2719
   gcugcaaauu gauaacuucg cuacauaaca aggaaaauau aaauguuuac aaacagcuua 2779
   aagauuugca ugugcagugu gcauuuauaa caaacuucua auugcacaaa acccaugcca 2839
   gcucagaguu uagguguaca cauuuaccca guugagcguu cuuagaauaa cuacugcaca 2899
   aguugacaau aggucguucu cucuuuuuuu uuguuugcuc ccuuuuucuu uuucuccccu 2959
   uccuccuuac ccucccuccc uuacucuccc cccccaccac cacccuccac ccccaacuca 3019
   ugaaaagauu cuauggacug aaaaagcccc aggcugaaag gacuggacug ccuugauuga 3079
   cauggggaag gggguuagua gacuaugugg auugcggcag cagaggcugc agccuaacgu 3139
   gugguuuuaa ugaccagcac gcaaggcaaa agcauuuugc acaguguuug uuuuccuguc 3199
   uugcacuuac aaauaagguc uaugggagua gcauggaaaa cguuugcugu uuuucccuuu 3259
   uuuuuuuaau ugcuuuuguu uaaaauuuga ucgccuuaac uacuguaaac auagccuauu 3319
   uuugugcuua agauacugaa uggaaaacuc cauugugugu ugcuggacug uuuuggaaau 3379
   auuugguuaa auguguguua auuuggcugu aauggcauuu aaagcaaaca aacaaacaaa 3439
   aaaagcugug aaaauggccu uggagcauua ucuuuaguua cuugaagagu uucuaguuuu 3499
   uuuaaaauac aguuuauguu aaaauaauuu uuauuaauuu agagaagaca aucaaugucu 3559
   gugagaaaac ggacuuucuu uuggauuuuc uuuuuguggu cauugugagu gauugcuuuu 3619
   uccuuuucuu aguuucacau ucuuccuuug uucuaaaacu uagacugaca ucuagcuuug 3679
   acaaucauag uauguuuuau uuuccugagg gggaauaacu uauaaugcug uuuaguuuug 3739
   uacuauuggu guguugguga auuuuuaaac ugugugcuaa cugcaauaaa uuauaugaac 3799
   ugagaa 3805

<210> 15
<211> 4319
<212> RNA
<213> Homo sapiens

<220>
<221> CDS
<222> (598)..(2166)

<400> 15
guuguuugcu aauuucugga cuaauuaugc aauuucauuu uuuuuucuug auucagccaa 60

augugugugu guguguuuaa acugugcuuu cuaagcacag ucagguagca aaaguaauaa 120

aaaggauggu ugaacaaguu uucuuguaug uuccaggaua uguuugggac uuuucuuugu 180

uuauuauaug aguuguuccc uuugaaauua aagcuauuuu guagguuuug ugggacauaa 240

uuugauaagu agaguuaauu aaauuucuuc uggaagagau cuaaauucuu auucuuagug 300

agagacugua guuaaaggaa ggcuuuuaga acuuggguuc aaggaagaug gagaugcguc 360

ggaagcucuu uggcgggggu gaggaaguuc agaaagugug cauuuuccuu cuggcauuua 420

ggucuugucc gugugauuug guggugcuug ggucauaagc cugauuaaaa uucagggaca 480

uguaccacgg cggccaaagc ggaauuaauu uuuuuauaug gggacuggag cgcugaaaag 540

uuguuccuga ccaggcucua augagaaauu ccucucuccc cagguuauga agacagu 597

aug gag uuu cca gac cau agu aga cau uug cua cag ugu cug agc gag 645
Met Glu Phe Pro Asp His Ser Arg His Leu Leu Gln Cys Leu Ser Glu
1               5                   10                  15

cag aga cac cag ggu uuu cuu ugu gac ugc acu guu cug gug gga gau 693
Gln Arg His Gln Gly Phe Leu Cys Asp Cys Thr Val Leu Val Gly Asp
            20                  25                  30

gcc cag uuc cga gcg cac cga gcu gua cug gcu uca ugc agc aug uau 741
Ala Gln Phe Arg Ala His Arg Ala Val Leu Ala Ser Cys Ser Met Tyr
        35                  40                  45

uuc cac cuc uuu uac aag gac cag cug gac aaa aga gac auu guu cau 789
Phe His Leu Phe Tyr Lys Asp Gln Leu Asp Lys Arg Asp Ile Val His
    50                  55                  60

cug aac agc gac auu guu aca gcc ccc gcu uuc gcu cuc cug cuu gaa 837
Leu Asn Ser Asp Ile Val Thr Ala Pro Ala Phe Ala Leu Leu Leu Glu
65                  70                  75                  80

uuc aug uau gaa ggg aaa cuc cag uuc aaa gac uug ccc auu gaa gac 885
Phe Met Tyr Glu Gly Lys Leu Gln Phe Lys Asp Leu Pro Ile Glu Asp
                85                  90                  95

gug cua gca gcu gcc agu uau cuc cac aug uau gac auu guc aaa guc 933
Val Leu Ala Ala Ala Ser Tyr Leu His Met Tyr Asp Ile Val Lys Val
            100                 105                 110

ugc aaa aag aag cug aaa gag aaa gcc acc acg gag gca gac agc acc 981
Cys Lys Lys Lys Leu Lys Glu Lys Ala Thr Thr Glu Ala Asp Ser Thr
        115                 120                 125

aaa aag gaa gaa gau gcu uca agu ugu ucg gac aaa guc gag agu cuc 1029
Lys Lys Glu Glu Asp Ala Ser Ser Cys Ser Asp Lys Val Glu Ser Leu
    130                 135                 140

ucc gau ggc agc agc cac aua gca ggc gau uug ccc agu gau gaa gau 1077

Ser Asp Gly Ser Ser His Ile Ala Gly Asp Leu Pro Ser Asp Glu Asp
145                 150                 155                 160

gaa gga gaa gau gaa aaa uug aac auc cug ccc agc aaa agg gac uug      1125
Glu Gly Glu Asp Glu Lys Leu Asn Ile Leu Pro Ser Lys Arg Asp Leu
                165                 170                 175

gcg gcc gag ccu ggg aac aug ugg aug cga uug ccc uca gac uca gca      1173
Ala Ala Glu Pro Gly Asn Met Trp Met Arg Leu Pro Ser Asp Ser Ala
            180                 185                 190

ggc auc ccc cag gcu ggc gga gag gca gag cca cac gcc aca gca gcu      1221
Gly Ile Pro Gln Ala Gly Gly Glu Ala Glu Pro His Ala Thr Ala Ala
        195                 200                 205

gga aaa aca gua gcc agc ccc ugc agc uca aca gag ucu uug ucc cag      1269
Gly Lys Thr Val Ala Ser Pro Cys Ser Ser Thr Glu Ser Leu Ser Gln
    210                 215                 220

agg ucu guc acc ucc gug agg gau ucg gca gau guu gac ugu gug cug      1317
Arg Ser Val Thr Ser Val Arg Asp Ser Ala Asp Val Asp Cys Val Leu
225                 230                 235                 240

gac cug ucu guc aag ucc agc cuu uca gga guu gaa aau cug aac agc      1365
Asp Leu Ser Val Lys Ser Ser Leu Ser Gly Val Glu Asn Leu Asn Ser
                245                 250                 255

ucu uau uuc ucu uca cag gac gug cug aga agc aac cug gug cag gug      1413
Ser Tyr Phe Ser Ser Gln Asp Val Leu Arg Ser Asn Leu Val Gln Val
            260                 265                 270

aag gug gag aaa gag gcu ucc ugu gau gag agu gau guu ggc acu aau      1461
Lys Val Glu Lys Glu Ala Ser Cys Asp Glu Ser Asp Val Gly Thr Asn
        275                 280                 285

gac uau gac aug gaa cau agc acu gug aaa gaa agu gug agc acu aau      1509
Asp Tyr Asp Met Glu His Ser Thr Val Lys Glu Ser Val Ser Thr Asn
    290                 295                 300

aac agg gua cag uau gag ccg gcc cau cug gcu ccc cug agg gag gac      1557
Asn Arg Val Gln Tyr Glu Pro Ala His Leu Ala Pro Leu Arg Glu Asp
305                 310                 315                 320

ucg guc uug agg gag cug gac cgg gag gac aaa gcc agu gau gau gag      1605
Ser Val Leu Arg Glu Leu Asp Arg Glu Asp Lys Ala Ser Asp Asp Glu
                325                 330                 335

aug aug acc cca gag agc gag cgu guc cag gug gag gga ggc aug gag      1653
Met Met Thr Pro Glu Ser Glu Arg Val Gln Val Glu Gly Gly Met Glu
            340                 345                 350

agc agu cug cuc ccc uac guc ucc aac auc cug agc ccc gcg ggc cag      1701
Ser Ser Leu Leu Pro Tyr Val Ser Asn Ile Leu Ser Pro Ala Gly Gln
        355                 360                 365

auc uuc aug ugc ccc cug ugc aac aag guc uuc ccc agc ccc cac auc      1749
Ile Phe Met Cys Pro Leu Cys Asn Lys Val Phe Pro Ser Pro His Ile
    370                 375                 380

cug cag auc cac cug agc acg cac uuc cgc gag cag gac ggc auc cgc      1797
Leu Gln Ile His Leu Ser Thr His Phe Arg Glu Gln Asp Gly Ile Arg
385                 390                 395                 400

agc aag ccc gcc gcc gau guc aac gug ccc acg ugc ucg cug ugu ggg     1845
Ser Lys Pro Ala Ala Asp Val Asn Val Pro Thr Cys Ser Leu Cys Gly
                405                 410                 415

aag acu uuc ucu ugc aug uac acc cuc aag cgc cac gag agg acu cac     1893
Lys Thr Phe Ser Cys Met Tyr Thr Leu Lys Arg His Glu Arg Thr His
            420                 425                 430

ucg ggg gag aag ccc uac aca ugc acc cag ugc ggc aag agc uuc cag     1941
Ser Gly Glu Lys Pro Tyr Thr Cys Thr Gln Cys Gly Lys Ser Phe Gln
        435                 440                 445

uac ucg cac aac cug agc cgc cau gcc gug gug cac acc cgc gag aag     1989
Tyr Ser His Asn Leu Ser Arg His Ala Val Val His Thr Arg Glu Lys
    450                 455                 460

ccg cac gcc ugc aag ugg ugc gag cgc agg uuc acg cag ucc ggg gac     2037
Pro His Ala Cys Lys Trp Cys Glu Arg Arg Phe Thr Gln Ser Gly Asp
465                 470                 475                 480

cug uac aga cac auu cgc aag uuc cac ugu gag uug gug aac ucc uug     2085
Leu Tyr Arg His Ile Arg Lys Phe His Cys Glu Leu Val Asn Ser Leu
                485                 490                 495

ucg guc aaa agc gaa gca cug agc uug ccu acu guc aga gac ugg acc     2133
Ser Val Lys Ser Glu Ala Leu Ser Leu Pro Thr Val Arg Asp Trp Thr
            500                 505                 510

uua gaa gau agc ucu caa gaa cuu ugg aaa uaa uuuuauauau auauaaauaa     2186
Leu Glu Asp Ser Ser Gln Glu Leu Trp Lys
        515                 520

uauauauaua uauacauaua uauaaauaga ucucuauaua guugugguac ggucuaaaag     2246

cagucuuguu uccuggaaau aaaaaguugg gauauuaacu uguuuuugca cuuuagaaua     2306

gcaugagaau cucacuaauu uagcauucug auaaaagaaa cuuuagagca agucagaaua     2366

gagagguguu uuuccuuuga ggggauaggg gaaguaagcc aauaagaacc uuuuaaacaa     2426

aucguccugu cacaaaaugc uuucauaugg cuuaauuuug ucaacacugc auugucuuuu     2486

gagcucuuuu uuccccccca acaaaguuuu uuuguuuuuu guuuuuuuuu uuaaguagaa     2546

auucccucca guuuuauuag ccucuuuaua ugucucaaau ugcaugaauu uuuucuggcu     2606

guuggaaacc ugaaugcuuu uagacccaaa uggaaaauuu cugaaaugcu ggauuaucua     2666

uuuuuaaaca agcaguugac uuaaaacuuu cuguggcaac uucugguuuu cugacaguuc     2726

ccagugagag aaaugcugaa aguacacugg gaucacuggg acacugucuu augaagguuu     2786

gcuugggaug aaaaaggaua uugcagcuuc agcaguguug aacugugugu uuaaaaaugu     2846

gaauuacugu uauuguauac uguaauugau uacaugggcu gggggggugu caaagaacuu     2906

gacagguugu guugaugcuc uuaguugagu cuugaaaagu aaauauuaac gcuacagaaa     2966

ugcaugaguu ucaauauauu uuuugucuuu guuugcauug uauaacuuua acgagugagu     3026

uuaaaauuau uuaauuuccu uagaaaaaua gcaccauuug gaaaaaaaaa cugguguuau     3086

gaagaacgua aaugcacugu uuuuauuuuu auuuuauaua auuuaaauug acuuucccac     3146

ugucuuuaag uugaaacugu uaagcugaau aaaaacuuaa gcugcaaauu gauaacuucg 3206

cuacauaaca aggaaaauau aaauguuuac aaacagcuua aagauuugca ugugcagugu 3266

gcauuuauaa caaacuucua auugcacaaa acccaugcca gcucagaguu uagguguaca 3326

cauuuaccca guugagcguu cuuagaauaa cuacugcaca aguugacaau aggucguucu 3386

cucuuuuuuu uuguuugcuc ccuuuuucuu uuucuccccu uccuccuuac ccucccuccc 3446

uuacucuccc cccccaccac cacccuccac ccccaacuca ugaaaagauu cuauggacug 3506

aaaaagcccc aggcugaaag gacuggacug ccuugauuga cauggggaag gggguuagua 3566

gacuaugugg auugcggcag cagaggcugc agccuaacgu gugguuuuaa ugaccagcac 3626

gcaaggcaaa agcauuuugc acaguguuug uuuuccuguc uugcacuuac aaauaagguc 3686

uaugggagua gcauggaaaa cguuugcugu uuuucccuuu uuuuuuuaau ugcuuuuguu 3746

uaaaauuuga ucgccuuaac uacuguaaac auagccuauu uuugugcuua agauacugaa 3806

uggaaaacuc cauugugugu ugcuggacug uuuuggaaau auuugguuaa auguguguua 3866

auuuggcugu aauggcauuu aaagcaaaca aacaaacaaa aaaagcugug aaaauggccu 3926

uggagcauua ucuuuaguua cuugaagagu uucuaguuuu uuuaaaauac aguuuauguu 3986

aaaauaauuu uuauuaauuu agagaagaca aucaaugucu gugagaaaac ggacuuucuu 4046

uuggauuuuc uuuuuguggu cauugugagu gauugcuuuu uccuuuucuu aguuucacau 4106

ucuuccuuug uucuaaaacu uagacugaca ucuagcuuug acaaucauag uauguuuuau 4166

uuuccugagg gggaauaacu uauaaugcug uuuaguuuug uacuauuggu guguugguga 4226

auuuuuaaac ugugugcuaa cugcaauaaa uuauaugaac ugagaaaaaa aaaaaaaaaa 4286

aaaaaaaaaa aaaaaaaaaa aaaaaaaaaa aaa 4319

<210> 16
<211> 1596
<212> DNA
<213> Homo sapiens

<220>
<221> CDS
<222> (1)..(1596)

<400> 16
atg tgt cct aaa ggt tat gaa gac agt atg gag ttt cca gac cat agt 48
aga cat ttg cta cag tgt ctg agc gag cag aga cac cag ggt ttt ctt 96
tgt gac tgc act gtt ctg gtg gga gat gcc cag ttc cga gcg cac cga 144
gct gta ctg gct tca tgc agc atg tat ttc cac ctc ttt tac aag gac        192
cag ctg gac aaa aga gac att gtt cat ctg aac agc gac att gtt aca        240
gcc ccc gct ttc gct ctc ctg ctt gaa ttc atg tat gaa ggg aaa ctc        288
cag ttc aaa gac ttg ccc att gaa gac gtg cta gca gct gcc agt tat        336
ctc cac atg tat gac att gtc aaa gtc tgc aaa aag aag ctg aaa gag        384
aaa gcc acc acg gag gca gac agc acc aaa aag gaa gaa gat gct tca        432
agt tgt tcg gac aaa gtc gag agt ctc tcc gat ggc agc agc cac ata        480
gca ggc gat ttg ccc agt gat gaa gat gaa gga gaa gat gaa aaa ttg        528
aac atc ctg ccc agc aaa agg gac ttg gcg gcc gag cct ggg aac atg        576
tgg atg cga ttg ccc tca gac tca gca ggc atc ccc cag gct ggc gga        624
gag gca gag cca cac gcc aca gca gct gga aaa aca gta gcc agc ccc        672
tgc agc tca aca gag tct ttg tcc cag agg tct gtc acc tcc gtg agg        720
gat tcg gca gat gtt gac tgt gtg ctg gac ctg tct gtc aag tcc agc        768
ctt tca gga gtt gaa aat ctg aac agc tct tat ttc tct tca cag gac        816
gtg ctg aga agc aac ctg gtg cag gtg aag gtg gag aaa gag gct tcc        864
tgt gat gag agt gat gtt ggc act aat gac tat gac atg gaa cat agc        912
act gtg aaa gaa agt gtg agc act aat aac agg gta cag tat gag ccg      960
gcc cat ctg gct ccc ctg agg gag gac tcg gtc ttg agg gag ctg gac      1008
cgg gag gac aaa gcc agt gat gat gag atg atg acc cca gag agc gag      1056
cgt gtc cag gtg gag gga ggc atg gag agc agt ctg ctc ccc tac gtc      1104
tcc aac atc ctg agc ccc gcg ggc cag atc ttc atg tgc ccc ctg tgc      1152
aac aag gtc ttc ccc agc ccc cac atc ctg cag atc cac ctg agc acg      1200
cac ttc cgc gag cag gac ggc atc cgc agc aag ccc gcc gcc gat gtc      1248
aac gtg ccc acg tgc tcg ctg tgt ggg aag act ttc tct tgc atg tac      1296
acc ctc aag cgc cac gag agg act cac tcg ggg gag aag ccc tac aca      1344
tgc acc cag tgc ggc aag agc ttc cag tac tcg cac aac ctg agc cgc      1392
cat gcc gtg gtg cac acc cgc gag aag ccg cac gcc tgc aag tgg tgc      1440
gag cgc agg ttc acg cag tcc ggg gac ctg tac aga cac att cgc aag      1488
ttc cac tgt gag ttg gtg aac tcc ttg tcg gtc aaa agc gaa gca ctg      1536
agc ttg cct act gtc aga gac tgg acc tta gaa gat agc tct caa gaa      1584
ctt tgg aaa taa                                                      1596

<210>  17
<211>  531
<212>  PRT
<213>  Homo sapiens

<400>  17

<210>  18
<211>  1569
<212>  DNA
<213>  Homo sapiens

<220>
<221>  CDS
<222>  (1)..(1569)

<400>  18
atg gag ttt cca gac cat agt aga cat ttg cta cag tgt ctg agc gag      48
Met Glu Phe Pro Asp His Ser Arg His Leu Leu Gln Cys Leu Ser Glu
1               5                   10                  15

cag aga cac cag ggt ttt ctt tgt gac tgc act gtt ctg gtg gga gat      96
Gln Arg His Gln Gly Phe Leu Cys Asp Cys Thr Val Leu Val Gly Asp
            20                  25                  30

gcc cag ttc cga gcg cac cga gct gta ctg gct tca tgc agc atg tat      144
Ala Gln Phe Arg Ala His Arg Ala Val Leu Ala Ser Cys Ser Met Tyr
        35                  40                  45

ttc cac ctc ttt tac aag gac cag ctg gac aaa aga gac att gtt cat      192
Phe His Leu Phe Tyr Lys Asp Gln Leu Asp Lys Arg Asp Ile Val His
    50                  55                  60

ctg aac agc gac att gtt aca gcc ccc gct ttc gct ctc ctg ctt gaa      240
Leu Asn Ser Asp Ile Val Thr Ala Pro Ala Phe Ala Leu Leu Leu Glu
65                  70                  75                  80

ttc atg tat gaa ggg aaa ctc cag ttc aaa gac ttg ccc att gaa gac      288
Phe Met Tyr Glu Gly Lys Leu Gln Phe Lys Asp Leu Pro Ile Glu Asp
                85                  90                  95

gtg cta gca gct gcc agt tat ctc cac atg tat gac att gtc aaa gtc      336
Val Leu Ala Ala Ala Ser Tyr Leu His Met Tyr Asp Ile Val Lys Val
            100                 105                 110

tgc aaa aag aag ctg aaa gag aaa gcc acc acg gag gca gac agc acc      384
Cys Lys Lys Lys Leu Lys Glu Lys Ala Thr Thr Glu Ala Asp Ser Thr
        115                 120                 125

aaa aag gaa gaa gat gct tca agt tgt tcg gac aaa gtc gag agt ctc      432
Lys Lys Glu Glu Asp Ala Ser Ser Cys Ser Asp Lys Val Glu Ser Leu

130                 135                 140

tcc gat ggc agc agc cac ata gca ggc gat ttg ccc agt gat gaa gat        480
Ser Asp Gly Ser Ser His Ile Ala Gly Asp Leu Pro Ser Asp Glu Asp
145                 150                 155                 160

gaa gga gaa gat gaa aaa ttg aac atc ctg ccc agc aaa agg gac ttg        528
Glu Gly Glu Asp Glu Lys Leu Asn Ile Leu Pro Ser Lys Arg Asp Leu
                165                 170                 175

gcg gcc gag cct ggg aac atg tgg atg cga ttg ccc tca gac tca gca        576
Ala Ala Glu Pro Gly Asn Met Trp Met Arg Leu Pro Ser Asp Ser Ala
            180                 185                 190

ggc atc ccc cag gct ggc gga gag gca gag cca cac gcc aca gca gct        624
Gly Ile Pro Gln Ala Gly Gly Glu Ala Glu Pro His Ala Thr Ala Ala
        195                 200                 205

gga aaa aca gta gcc agc ccc tgc agc tca aca gag tct ttg tcc cag        672
Gly Lys Thr Val Ala Ser Pro Cys Ser Ser Thr Glu Ser Leu Ser Gln
    210                 215                 220

agg tct gtc acc tcc gtg agg gat tcg gca gat gtt gac tgt gtg ctg        720
Arg Ser Val Thr Ser Val Arg Asp Ser Ala Asp Val Asp Cys Val Leu
225                 230                 235                 240

gac ctg tct gtc aag tcc agc ctt tca gga gtt gaa aat ctg aac agc        768
Asp Leu Ser Val Lys Ser Ser Leu Ser Gly Val Glu Asn Leu Asn Ser
                245                 250                 255

tct tat ttc tct tca cag gac gtg ctg aga agc aac ctg gtg cag gtg        816
Ser Tyr Phe Ser Ser Gln Asp Val Leu Arg Ser Asn Leu Val Gln Val
            260                 265                 270

aag gtg gag aaa gag gct tcc tgt gat gag agt gat gtt ggc act aat        864
Lys Val Glu Lys Glu Ala Ser Cys Asp Glu Ser Asp Val Gly Thr Asn
        275                 280                 285

gac tat gac atg gaa cat agc act gtg aaa gaa agt gtg agc act aat        912
Asp Tyr Asp Met Glu His Ser Thr Val Lys Glu Ser Val Ser Thr Asn
    290                 295                 300

aac agg gta cag tat gag ccg gcc cat ctg gct ccc ctg agg gag gac        960
Asn Arg Val Gln Tyr Glu Pro Ala His Leu Ala Pro Leu Arg Glu Asp
305                 310                 315                 320

tcg gtc ttg agg gag ctg gac cgg gag gac aaa gcc agt gat gat gag       1008
Ser Val Leu Arg Glu Leu Asp Arg Glu Asp Lys Ala Ser Asp Asp Glu
                325                 330                 335

atg atg acc cca gag agc gag cgt gtc cag gtg gag gga ggc atg gag       1056
Met Met Thr Pro Glu Ser Glu Arg Val Gln Val Glu Gly Gly Met Glu
            340                 345                 350

agc agt ctg ctc ccc tac gtc tcc aac atc ctg agc ccc gcg ggc cag       1104
Ser Ser Leu Leu Pro Tyr Val Ser Asn Ile Leu Ser Pro Ala Gly Gln
        355                 360                 365

atc ttc atg tgc ccc ctg tgc aac aag gtc ttc ccc agc ccc cac atc       1152
Ile Phe Met Cys Pro Leu Cys Asn Lys Val Phe Pro Ser Pro His Ile
    370                 375                 380

ctg cag atc cac ctg agc acg cac ttc cgc gag cag gac ggc atc cgc       1200

Leu Gln Ile His Leu Ser Thr His Phe Arg Glu Gln Asp Gly Ile Arg
385                 390                 395                 400

agc aag ccc gcc gcc gat gtc aac gtg ccc acg tgc tcg ctg tgt ggg      1248
Ser Lys Pro Ala Ala Asp Val Asn Val Pro Thr Cys Ser Leu Cys Gly
                405                 410                 415

aag act ttc tct tgc atg tac acc ctc aag cgc cac gag agg act cac      1296
Lys Thr Phe Ser Cys Met Tyr Thr Leu Lys Arg His Glu Arg Thr His
            420                 425                 430

tcg ggg gag aag ccc tac aca tgc acc cag tgc ggc aag agc ttc cag      1344
Ser Gly Glu Lys Pro Tyr Thr Cys Thr Gln Cys Gly Lys Ser Phe Gln
        435                 440                 445

tac tcg cac aac ctg agc cgc cat gcc gtg gtg cac acc cgc gag aag      1392
Tyr Ser His Asn Leu Ser Arg His Ala Val Val His Thr Arg Glu Lys
    450                 455                 460

ccg cac gcc tgc aag tgg tgc gag cgc agg ttc acg cag tcc ggg gac      1440
Pro His Ala Cys Lys Trp Cys Glu Arg Arg Phe Thr Gln Ser Gly Asp
465                 470                 475                 480

ctg tac aga cac att cgc aag ttc cac tgt gag ttg gtg aac tcc ttg      1488
Leu Tyr Arg His Ile Arg Lys Phe His Cys Glu Leu Val Asn Ser Leu
                485                 490                 495

tcg gtc aaa agc gaa gca ctg agc ttg cct act gtc aga gac tgg acc      1536
Ser Val Lys Ser Glu Ala Leu Ser Leu Pro Thr Val Arg Asp Trp Thr
            500                 505                 510

tta gaa gat agc tct caa gaa ctt tgg aaa taa                          1569
Leu Glu Asp Ser Ser Gln Glu Leu Trp Lys
        515                 520


<210>  19
<211>  522
<212>  PRT
<213>  Homo sapiens

<400>  19

Met Glu Phe Pro Asp His Ser Arg His Leu Leu Gln Cys Leu Ser Glu
1               5                   10                  15


Gln Arg His Gln Gly Phe Leu Cys Asp Cys Thr Val Leu Val Gly Asp
            20                  25                  30


Ala Gln Phe Arg Ala His Arg Ala Val Leu Ala Ser Cys Ser Met Tyr
        35                  40                  45


Phe His Leu Phe Tyr Lys Asp Gln Leu Asp Lys Arg Asp Ile Val His
    50                  55                  60


Leu Asn Ser Asp Ile Val Thr Ala Pro Ala Phe Ala Leu Leu Leu Glu
65                  70                  75                  80

Phe Met Tyr Glu Gly Lys Leu Gln Phe Lys Asp Leu Pro Ile Glu Asp
                85                  90                  95

Val Leu Ala Ala Ala Ser Tyr Leu His Met Tyr Asp Ile Val Lys Val
            100                 105                 110

Cys Lys Lys Lys Leu Lys Glu Lys Ala Thr Thr Glu Ala Asp Ser Thr
        115                 120                 125

Lys Lys Glu Glu Asp Ala Ser Ser Cys Ser Asp Lys Val Glu Ser Leu
    130                 135                 140

Ser Asp Gly Ser Ser His Ile Ala Gly Asp Leu Pro Ser Asp Glu Asp
145                 150                 155                 160

Glu Gly Glu Asp Glu Lys Leu Asn Ile Leu Pro Ser Lys Arg Asp Leu
                165                 170                 175

Ala Ala Glu Pro Gly Asn Met Trp Met Arg Leu Pro Ser Asp Ser Ala
            180                 185                 190

Gly Ile Pro Gln Ala Gly Gly Glu Ala Glu Pro His Ala Thr Ala Ala
        195                 200                 205

Gly Lys Thr Val Ala Ser Pro Cys Ser Ser Thr Glu Ser Leu Ser Gln
    210                 215                 220

Arg Ser Val Thr Ser Val Arg Asp Ser Ala Asp Val Asp Cys Val Leu
225                 230                 235                 240

Asp Leu Ser Val Lys Ser Ser Leu Ser Gly Val Glu Asn Leu Asn Ser
                245                 250                 255

Ser Tyr Phe Ser Ser Gln Asp Val Leu Arg Ser Asn Leu Val Gln Val
            260                 265                 270

Lys Val Glu Lys Glu Ala Ser Cys Asp Glu Ser Asp Val Gly Thr Asn
        275                 280                 285

Asp Tyr Asp Met Glu His Ser Thr Val Lys Glu Ser Val Ser Thr Asn
    290                 295                 300

Asn Arg Val Gln Tyr Glu Pro Ala His Leu Ala Pro Leu Arg Glu Asp
305                 310                 315                 320

Ser Val Leu Arg Glu Leu Asp Arg Glu Asp Lys Ala Ser Asp Asp Glu
                325                 330                 335

Met Met Thr Pro Glu Ser Glu Arg Val Gln Val Glu Gly Gly Met Glu
            340                 345                 350

Ser Ser Leu Leu Pro Tyr Val Ser Asn Ile Leu Ser Pro Ala Gly Gln
        355                 360                 365

Ile Phe Met Cys Pro Leu Cys Asn Lys Val Phe Pro Ser Pro His Ile
    370                 375                 380

Leu Gln Ile His Leu Ser Thr His Phe Arg Glu Gln Asp Gly Ile Arg
385                 390                 395                 400

Ser Lys Pro Ala Ala Asp Val Asn Val Pro Thr Cys Ser Leu Cys Gly
                405                 410                 415

Lys Thr Phe Ser Cys Met Tyr Thr Leu Lys Arg His Glu Arg Thr His
            420                 425                 430

Ser Gly Glu Lys Pro Tyr Thr Cys Thr Gln Cys Gly Lys Ser Phe Gln
        435                 440                 445

Tyr Ser His Asn Leu Ser Arg His Ala Val Val His Thr Arg Glu Lys
    450                 455                 460

Pro His Ala Cys Lys Trp Cys Glu Arg Arg Phe Thr Gln Ser Gly Asp
465                 470                 475                 480

Leu Tyr Arg His Ile Arg Lys Phe His Cys Glu Leu Val Asn Ser Leu
                485                 490                 495

Ser Val Lys Ser Glu Ala Leu Ser Leu Pro Thr Val Arg Asp Trp Thr
            500                 505                 510

Leu Glu Asp Ser Ser Gln Glu Leu Trp Lys
        515                 520

<210>  20
<211>  3937
<212>  RNA
<213>  Mus musculus

<220>
<221>  CDS
<222>  (260)..(1828)

<400>  20
aggcugcggc cgccgcuggg cugcugccau ggggagccgu ugaaagagug gggcccugug      60

gcucggccgc gcgccucagg cugccagcgc gggcgggggg cggcgggggc gcgcggggcc     120

cgggccgggg ccggcgggcg ggcgggggcc ggcggaggac gcccacaagu ccagcggcgg 180

cggcggcggc agaggaggag gaggaggagg acgcggcggg gacgcggcga cucgggccgc 240

uccguguuau gaagacagu aug gag uuu cca gac cac agc aga cau uug cug 292
Met Glu Phe Pro Asp His Ser Arg His Leu Leu
1               5                   10

cag ugu cug agc gag cag agg cac cag ggc uuu cuu ugu gac ugc acu 340
Gln Cys Leu Ser Glu Gln Arg His Gln Gly Phe Leu Cys Asp Cys Thr
            15                  20                  25

guu cug gug gga gau gcc cag uuc cgg gca cac cga gcu guu cug gcu 388
Val Leu Val Gly Asp Ala Gln Phe Arg Ala His Arg Ala Val Leu Ala
        30                  35                  40

uca ugc agc aug uau uuc cac cuc uuu uac aag gac cag cug gac aaa 436
Ser Cys Ser Met Tyr Phe His Leu Phe Tyr Lys Asp Gln Leu Asp Lys
    45                  50                  55

aga gac auu guu cau cug aac agc gac auu gug aca gcc ccc gcu uuc 484
Arg Asp Ile Val His Leu Asn Ser Asp Ile Val Thr Ala Pro Ala Phe
60                  65                  70                  75

gcu cuc cug cuu gaa uuc aug uac gaa ggg aaa cuc cag uuc aaa gac 532
Ala Leu Leu Leu Glu Phe Met Tyr Glu Gly Lys Leu Gln Phe Lys Asp
                80                  85                  90

uug ccc auu gag gac gug cua gca gcu gcc agu uau cuc cac aug uau 580
Leu Pro Ile Glu Asp Val Leu Ala Ala Ala Ser Tyr Leu His Met Tyr
            95                  100                 105

gac auu guc aaa guc ugc aaa aag aag cug aaa gag aaa gcu acc aca 628
Asp Ile Val Lys Val Cys Lys Lys Lys Leu Lys Glu Lys Ala Thr Thr
        110                 115                 120

gag gcg gac agc acc aaa aaa gaa gaa gau gcu uca agu ugu ucg gau 676
Glu Ala Asp Ser Thr Lys Lys Glu Glu Asp Ala Ser Ser Cys Ser Asp
    125                 130                 135

aaa guc gag agc cuc uca gac ggc agc agc cac aug gca ggu gac cug 724
Lys Val Glu Ser Leu Ser Asp Gly Ser Ser His Met Ala Gly Asp Leu
140                 145                 150                 155

ccc agu gau gaa gau gaa ggc gaa gau gac aaa uug aac auu cua ccc 772
Pro Ser Asp Glu Asp Glu Gly Glu Asp Asp Lys Leu Asn Ile Leu Pro
                160                 165                 170

agc aaa agg gac uug gca gcu gag ccu ggg aac aug ugg aug cga uug 820
Ser Lys Arg Asp Leu Ala Ala Glu Pro Gly Asn Met Trp Met Arg Leu
            175                 180                 185

ccc uca gac uca gca ggc auc ccc cag gcu ggc gga gag gcc gag cca 868
Pro Ser Asp Ser Ala Gly Ile Pro Gln Ala Gly Gly Glu Ala Glu Pro
        190                 195                 200

cac gcc aca gca gcu gga aaa aca gua gcc agc ccc ugc agc uca aca 916
His Ala Thr Ala Ala Gly Lys Thr Val Ala Ser Pro Cys Ser Ser Thr
    205                 210                 215

gag ucu uug ucc cag agg ucu guc acc ucc gug agg gau ucg gca gau 964
Glu Ser Leu Ser Gln Arg Ser Val Thr Ser Val Arg Asp Ser Ala Asp

220                 225                 230                 235

guu gac ugu gug cug gac cug ucu guc aag ucc agc cuu uca gga guu        1012
Val Asp Cys Val Leu Asp Leu Ser Val Lys Ser Ser Leu Ser Gly Val
                240                 245                 250

gaa aau cug aac agc ucu uau uuc ucu uca cag gac gug cug aga agc        1060
Glu Asn Leu Asn Ser Ser Tyr Phe Ser Ser Gln Asp Val Leu Arg Ser
            255                 260                 265

aac cug gug cag gug aag gug gag aaa gag gcg ucc ugu gau gag agu        1108
Asn Leu Val Gln Val Lys Val Glu Lys Glu Ala Ser Cys Asp Glu Ser
        270                 275                 280

gau guu ggc acu aau gac uau gac aug gaa cau agc acu gug aaa gag        1156
Asp Val Gly Thr Asn Asp Tyr Asp Met Glu His Ser Thr Val Lys Glu
    285                 290                 295

agu gug agc acu aac aac agg gua cag uau gag cca gcc cac cug gcu        1204
Ser Val Ser Thr Asn Asn Arg Val Gln Tyr Glu Pro Ala His Leu Ala
300                 305                 310                 315

ccu cug agg gag gac ucg guc uua agg gaa cug gau cgg gag gac aaa        1252
Pro Leu Arg Glu Asp Ser Val Leu Arg Glu Leu Asp Arg Glu Asp Lys
                320                 325                 330

gcc agc gau gau gag aug aug acc cca gag agc gag cgg guc cag gug        1300
Ala Ser Asp Asp Glu Met Met Thr Pro Glu Ser Glu Arg Val Gln Val
            335                 340                 345

gaa ggg ggc aug gag aac agu cug cuu ccc uac guc ucc aau auc cug        1348
Glu Gly Gly Met Glu Asn Ser Leu Leu Pro Tyr Val Ser Asn Ile Leu
        350                 355                 360

agc ccc gca ggc cag auc uuc aug ugc ccc cug ugc aac aaa guc uuc        1396
Ser Pro Ala Gly Gln Ile Phe Met Cys Pro Leu Cys Asn Lys Val Phe
    365                 370                 375

ccc agc ccc cac auc cug cag auc cac cug agc acg cac uuc cga gag        1444
Pro Ser Pro His Ile Leu Gln Ile His Leu Ser Thr His Phe Arg Glu
380                 385                 390                 395

cag gau ggc auc cgu agc aag ccu gcc gcc gau guc aau gug ccc acg        1492
Gln Asp Gly Ile Arg Ser Lys Pro Ala Ala Asp Val Asn Val Pro Thr
                400                 405                 410

ugc ucc cug ugu ggg aag acu uuc ucc ugc aug uac acc cuc aag cgc        1540
Cys Ser Leu Cys Gly Lys Thr Phe Ser Cys Met Tyr Thr Leu Lys Arg
            415                 420                 425

cac gag agg acu cac ucg ggg gag aag ccc uac acg ugc acc caa ugc        1588
His Glu Arg Thr His Ser Gly Glu Lys Pro Tyr Thr Cys Thr Gln Cys
        430                 435                 440

ggc aag agu uuc cag uac ucg cac aac cug agc cgu cau gcc gug gug        1636
Gly Lys Ser Phe Gln Tyr Ser His Asn Leu Ser Arg His Ala Val Val
    445                 450                 455

cac acc cgc gag aag ccc cau gcc ugc aag ugg ugc gag cgc agg uuc        1684
His Thr Arg Glu Lys Pro His Ala Cys Lys Trp Cys Glu Arg Arg Phe
460                 465                 470                 475

acg cag ucc gga gac cug uac aga cac auc cgc aag uuc cac ugu gag        1732

Thr Gln Ser Gly Asp Leu Tyr Arg His Ile Arg Lys Phe His Cys Glu
                480                 485                 490

uug gug aac ucc uug ucg guc aaa agu gaa gcg cug agc uug ccc acu      1780
Leu Val Asn Ser Leu Ser Val Lys Ser Glu Ala Leu Ser Leu Pro Thr
            495                 500                 505

guc aga gac ugg acc uua gaa gau agc ucu caa gaa cuu ugg aaa uaa      1828
Val Arg Asp Trp Thr Leu Glu Asp Ser Ser Gln Glu Leu Trp Lys
        510                 515                 520

uuuuauauau auauauauau aaauaauaua uauauauaua uauacauaua uauacauaga      1888

ucucuauaua guugugguac ggucuaaaag cagucuuguu uccuggaacu gaaaaguugg      1948

gaucuuaacu uguuuuugca cuuuagaaua auagcaugag aaucucacua auuuagcauu      2008

cugauaaaag aaacuagagc aagucacaag uagagagcug ucuuucuuuu gaggguuagg      2068

ccaaguuagc caauaaaacc uucucaacaa augguucugu cacaaacgcu uucacauggc      2128

uuacuuuugu acacacugca uugucuugag cgcuuucccu gugcuccaca cuguuuuguu      2188

uuguuuuguu uucagaguag aaauccccuc caguuuauua gccucuuuau augucucaaa      2248

uugcaugaau auuuucuggc uguuggaaac cugaaugcuu uuagacccaa auggaaaauu      2308

ucugaaaugc uggauuaucu auuuuuaaac aagcaguuga cuuaaaacuu uguguggcaa      2368

cuucugguuu ucugaugguc cccagugaga gaaaucugac aguacacugg gucacuggga      2428

cugucucugu gaagguuugc uugagaugaa aaaggauauu gcagcuucag caguguugaa      2488

cuguguguuu aaaaugugaa uuacuguucu uguauacugu aauugauuac acgggcuggg      2548

ggggguguca aagaacuuga cagguugugu ugaugcucuu aguugagucu ugaaaaguaa      2608

auauuaacgc uacagaaaug caugaguuuc aauauauuuu uugucuuugu uugcauugua      2668

uaacuuuaac gagugaguuu aaaauuauuu aauuuccuua gaaaaauagc accauuuggg      2728

aaaaaaacug guguuacgaa gaacauaaau gcacugucuu uauuuuuauu uuuauuuuau      2788

auaauuuaaa uugacuuuuc ccacgucuuu aaguuugaaa cuguuaagcu gaauaaaaac      2848

uuaagcugca aauugauaac uucgcuacau uacaaggaac auauaaaugu uuacaaacgg      2908

cuuaaagaau ugcaugugca gugugcauuc auaccacacu uguaauugca cagaacccau      2968

gccagcucag aguuuaggug uacacacgua cccagccgag uguuuuuaga auaacuacug      3028

cacaaauuga caauagguca uaggucauuc ucucucuccc uccuuuuugc uuccccuuuc      3088

uuuuucuccc cuauuuccuu uccuuccccc uacccccuaa cucaugaaaa gauucuaugg      3148

acugaaaaag ccccaggcug aaaggacuga accgccuuga uugacauggg gagggggguu      3208

aguagacugu guaucggcag cagaggcugc agcccgaugu gugguucuga uggccagcgc      3268

ggggcagaag cguuuugcac acuguuuguu uuccugucuu gcacuuacaa auaaggucua      3328

ugggaguagc augggaaaca gugguuguuu uucccuuuuu cuuuuuuaau ugcuuuuguu      3388

uaaaauuuga ucgccuuaac uacuguaaac auagccuauu uuugugcuua agauacugaa 3448

uggaaaacuc cauugugugu ugcuggacug uuuuggaaau auuuggucaa auguauguua 3508

auuuggcugu aauggcauuu aaagcaaaca aacaaacaaa caaacaaagc ugugaaaaug 3568

gccuuggagc auuaucuuua guuacuugaa gaguuucuag uuuuuuuuuu uuuaauacag 3628

uuuauguuaa aauaaucuuu auuaaucuag aggagacaau caaugucugu ggaagaacgg 3688

acuuuuuugg auuuucuuuu uuaaugguca uugugaguga uugcucuuuc uuuuacuuag 3748

uuucacauuc uuccuuuguu cuaaaucuua gacugacauc uagcuuugac aaucauagua 3808

uguuuuauuu ccugaggggg aauaacuuau aaugcuguuu aguuuuguac uauuggugug 3868

uuggugaauu uuuaaacugu gugcuaacug caauaaauua uaugaacuga gaaaaaaaaa 3928

aaaaaaaaa 3937

<210> 21
<211> 4147
<212> RNA
<213> Mus musculus

<220>
<221> CDS
<222> (486)..(2054)

<400> 21
aggcugcggc cgccgcuggg cugcugccau ggggagccgu ugaaagagug gggcccugug 60

gcucggccgc gcgccucagg cugccagcgc gggcgggggg cggcgggggc gcgcggggcc 120

cgggccgggg ccggcgggcg ggcgggggcc ggcggaggac gcccacaagu ccagcggcgg 180

cggcggcggc agaggaggag gaggaggagg acgcggcggg gacgcggcga cucgggccgc 240

uccguaaccu ccgaggaucc gcgguagcgg uggacggguc gauccgugga guggaugucu 300

ucgggccauu gcggugcuug ggacucauua aacugucacu cacccccacc accaccccac 360

ugguauuuca gaagugcagu uugaguuugu ugaugcugaa aguggaaauu ugcacuaacg 420

guggcagugu gaacuauuuu ccugagaggu agauccagau aaauugcuua gguuaugaag 480

acagu aug gag uuu cca gac cac agc aga cau uug cug cag ugu cug agc 530
Met Glu Phe Pro Asp His Ser Arg His Leu Leu Gln Cys Leu Ser
1               5                   10                  15

gag cag agg cac cag ggc uuu cuu ugu gac ugc acu guu cug gug gga 578
Glu Gln Arg His Gln Gly Phe Leu Cys Asp Cys Thr Val Leu Val Gly
                20                  25                  30

gau gcc cag uuc cgg gca cac cga gcu guu cug gcu uca ugc agc aug 626
Asp Ala Gln Phe Arg Ala His Arg Ala Val Leu Ala Ser Cys Ser Met
            35                  40                  45

uau uuc cac cuc uuu uac aag gac cag cug gac aaa aga gac auu guu 674
Tyr Phe His Leu Phe Tyr Lys Asp Gln Leu Asp Lys Arg Asp Ile Val
        50                  55                  60

cau cug aac agc gac auu gug aca gcc ccc gcu uuc gcu cuc cug cuu     722
His Leu Asn Ser Asp Ile Val Thr Ala Pro Ala Phe Ala Leu Leu Leu
    65                  70                  75

gaa uuc aug uac gaa ggg aaa cuc cag uuc aaa gac uug ccc auu gag     770
Glu Phe Met Tyr Glu Gly Lys Leu Gln Phe Lys Asp Leu Pro Ile Glu
80                  85                  90                  95

gac gug cua gca gcu gcc agu uau cuc cac aug uau gac auu guc aaa     818
Asp Val Leu Ala Ala Ala Ser Tyr Leu His Met Tyr Asp Ile Val Lys
                100                 105                 110

guc ugc aaa aag aag cug aaa gag aaa gcu acc aca gag gcg gac agc     866
Val Cys Lys Lys Lys Leu Lys Glu Lys Ala Thr Thr Glu Ala Asp Ser
            115                 120                 125

acc aaa aaa gaa gaa gau gcu uca agu ugu ucg gau aaa guc gag agc     914
Thr Lys Lys Glu Glu Asp Ala Ser Ser Cys Ser Asp Lys Val Glu Ser
        130                 135                 140

cuc uca gac ggc agc agc cac aug gca ggu gac cug ccc agu gau gaa     962
Leu Ser Asp Gly Ser Ser His Met Ala Gly Asp Leu Pro Ser Asp Glu
    145                 150                 155

gau gaa ggc gaa gau gac aaa uug aac auu cua ccc agc aaa agg gac     1010
Asp Glu Gly Glu Asp Asp Lys Leu Asn Ile Leu Pro Ser Lys Arg Asp
160                 165                 170                 175

uug gca gcu gag ccu ggg aac aug ugg aug cga uug ccc uca gac uca     1058
Leu Ala Ala Glu Pro Gly Asn Met Trp Met Arg Leu Pro Ser Asp Ser
                180                 185                 190

gca ggc auc ccc cag gcu ggc gga gag gcc gag cca cac gcc aca gca     1106
Ala Gly Ile Pro Gln Ala Gly Gly Glu Ala Glu Pro His Ala Thr Ala
            195                 200                 205

gcu gga aaa aca gua gcc agc ccc ugc agc uca aca gag ucu uug ucc     1154
Ala Gly Lys Thr Val Ala Ser Pro Cys Ser Ser Thr Glu Ser Leu Ser
        210                 215                 220

cag agg ucu guc acc ucc gug agg gau ucg gca gau guu gac ugu gug     1202
Gln Arg Ser Val Thr Ser Val Arg Asp Ser Ala Asp Val Asp Cys Val
    225                 230                 235

cug gac cug ucu guc aag ucc agc cuu uca gga guu gaa aau cug aac     1250
Leu Asp Leu Ser Val Lys Ser Ser Leu Ser Gly Val Glu Asn Leu Asn
240                 245                 250                 255

agc ucu uau uuc ucu uca cag gac gug cug aga agc aac cug gug cag     1298
Ser Ser Tyr Phe Ser Ser Gln Asp Val Leu Arg Ser Asn Leu Val Gln
                260                 265                 270

gug aag gug gag aaa gag gcg ucc ugu gau gag agu gau guu ggc acu     1346
Val Lys Val Glu Lys Glu Ala Ser Cys Asp Glu Ser Asp Val Gly Thr
            275                 280                 285

aau gac uau gac aug gaa cau agc acu gug aaa gag agu gug agc acu     1394
Asn Asp Tyr Asp Met Glu His Ser Thr Val Lys Glu Ser Val Ser Thr
        290                 295                 300

aac aac agg gua cag uau gag cca gcc cac cug gcu ccu cug agg gag     1442
Asn Asn Arg Val Gln Tyr Glu Pro Ala His Leu Ala Pro Leu Arg Glu

305                 310                 315

gac ucg guc uua agg gaa cug gau cgg gag gac aaa gcc agc gau gau      1490
Asp Ser Val Leu Arg Glu Leu Asp Arg Glu Asp Lys Ala Ser Asp Asp
320                 325                 330                 335

gag aug aug acc cca gag agc gag cgg guc cag gug gaa ggg ggc aug      1538
Glu Met Met Thr Pro Glu Ser Glu Arg Val Gln Val Glu Gly Gly Met
                340                 345                 350

gag aac agu cug cuu ccc uac guc ucc aau auc cug agc ccc gca ggc      1586
Glu Asn Ser Leu Leu Pro Tyr Val Ser Asn Ile Leu Ser Pro Ala Gly
            355                 360                 365

cag auc uuc aug ugc ccc cug ugc aac aaa guc uuc ccc agc ccc cac      1634
Gln Ile Phe Met Cys Pro Leu Cys Asn Lys Val Phe Pro Ser Pro His
        370                 375                 380

auc cug cag auc cac cug agc acg cac uuc cga gag cag gau ggc auc      1682
Ile Leu Gln Ile His Leu Ser Thr His Phe Arg Glu Gln Asp Gly Ile
    385                 390                 395

cgu agc aag ccu gcc gcc gau guc aau gug ccc acg ugc ucc cug ugu      1730
Arg Ser Lys Pro Ala Ala Asp Val Asn Val Pro Thr Cys Ser Leu Cys
400                 405                 410                 415

ggg aag acu uuc ucc ugc aug uac acc cuc aag cgc cac gag agg acu      1778
Gly Lys Thr Phe Ser Cys Met Tyr Thr Leu Lys Arg His Glu Arg Thr
                420                 425                 430

cac ucg ggg gag aag ccc uac acg ugc acc caa ugc ggc aag agu uuc      1826
His Ser Gly Glu Lys Pro Tyr Thr Cys Thr Gln Cys Gly Lys Ser Phe
            435                 440                 445

cag uac ucg cac aac cug agc cgu cau gcc gug gug cac acc cgc gag      1874
Gln Tyr Ser His Asn Leu Ser Arg His Ala Val Val His Thr Arg Glu
        450                 455                 460

aag ccc cau gcc ugc aag ugg ugc gag cgc agg uuc acg cag ucc gga      1922
Lys Pro His Ala Cys Lys Trp Cys Glu Arg Arg Phe Thr Gln Ser Gly
    465                 470                 475

gac cug uac aga cac auc cgc aag uuc cac ugu gag uug gug aac ucc      1970
Asp Leu Tyr Arg His Ile Arg Lys Phe His Cys Glu Leu Val Asn Ser
480                 485                 490                 495

uug ucg guc aaa agu gaa gcg cug agc uug ccc acu guc aga gac ugg      2018
Leu Ser Val Lys Ser Glu Ala Leu Ser Leu Pro Thr Val Arg Asp Trp
                500                 505                 510

acc uua gaa gau agc ucu caa gaa cuu ugg aaa uaa uuuuauauau           2064
Thr Leu Glu Asp Ser Ser Gln Glu Leu Trp Lys
            515                 520

auauauauau aaauaauaua uauauauaua uauacauaua uauacauaga ucucuauaua      2124

guugugguac ggucuaaaag cagucuuguu uccuggaacu gaaaaguugg gaucuuaacu      2184

uguuuuugca cuuuagaaua auagcaugag aaucucacua auuuagcauu cugauaaaag      2244

aaacuagagc aagucacaag uagagagcug ucuuucuuuu gaggguuagg ccaaguuagc      2304

caauaaaacc uucucaacaa augguucugu cacaaacgcu uucacauggc uuacuuuugu      2364

acacacugca uugucuugag cgcuuucccu gugcuccaca cuguuuuguu uuguuuuguu 2424

uucagaguag aaauccccuc caguuuauua gccucuuuau augucucaaa uugcaugaau 2484

auuuucuggc uguuggaaac cugaaugcuu uuagacccaa auggaaaauu ucugaaaugc 2544

uggauuaucu auuuuuaaac aagcaguuga cuuaaaacuu uguguggcaa cuucugguuu 2604

ucugaugguc cccagugaga gaaaucugac aguacacugg gucacuggga cugucucugu 2664

gaagguuugc uugagaugaa aaaggauauu gcagcuucag caguguugaa cuguguguuu 2724

aaaaugugaa uuacuguucu uguauacugu aauugauuac acgggcuggg ggggguguca 2784

aagaacuuga cagguugugu ugaugcucuu aguugagucu ugaaaaguaa auauuaacgc 2844

uacagaaaug caugaguuuc aauauauuuu uugucuuugu uugcauugua uaacuuuaac 2904

gagugaguuu aaaauuauuu aauuuccuua gaaaaauagc accauuuggg aaaaaaacug 2964

guguuacgaa gaacauaaau gcacugucuu uauuuuuauu uuuauuuuau auaauuuaaa 3024

uugacuuuuc ccacgucuuu aaguuugaaa cuguuaagcu gaauaaaaac uuaagcugca 3084

aauugauaac uucgcuacau uacaaggaac auauaaaugu uuacaaacgg cuuaaagaau 3144

ugcaugugca gugugcauuc auaccacacu uguaauugca cagaacccau gccagcucag 3204

aguuuaggug uacacacgua cccagccgag uguuuuuaga auaacuacug cacaaauuga 3264

caauagguca uaggucauuc ucucucuccc uccuuuuugc uuccccuuuc uuuuucuccc 3324

cuauuuccuu uccuuccccc uacccccuaa cucaugaaaa gauucuaugg acugaaaaag 3384

ccccaggcug aaaggacuga accgccuuga uugacauggg gagggggguu aguagacugu 3444

guaucggcag cagaggcugc agcccgaugu gugguucuga uggccagcgc ggggcagaag 3504

cguuuugcac acuguuuguu uuccugucuu gcacuuacaa auaaggucua ugggaguagc 3564

augggaaaca gugguuguuu uucccuuuuu cuuuuuuaau ugcuuuuguu uaaaauuuga 3624

ucgccuuaac uacuguaaac auagccuauu uuugugcuua agauacugaa uggaaaacuc 3684

cauugugugu ugcuggacug uuuuggaaau auuuggucaa auguauguua auuuggcugu 3744

aauggcauuu aaagcaaaca aacaaacaaa caaacaaagc ugugaaaaug gccuuggagc 3804

auuaucuuua guuacuugaa gaguuucuag uuuuuuuuuu uuuaauacag uuuauguuaa 3864

aauaaucuuu auuaaucuag aggagacaau caaugucugu ggaagaacgg acuuuuuugg 3924

auuuucuuuu uuaaugguca uugugaguga uugcucuuuc uuuuacuuag uuucacauuc 3984

uuccuuuguu cuaaaucuua gacugacauc uagcuuugac aaucauagua uguuuuauuu 4044

ccugaggggg aauaacuuau aaugcuguuu aguuuuguac uauuggugug uuggugaauu 4104

uuuaaacugu gugcuaacug caauaaauua uaugaacuga gaa 4147

<210> 22

<211>  4039
<212>  RNA
<213>  Mus musculus

<220>
<221>  CDS
<222>  (378)..(1946)

<400>  22
aggcugcggc cgccgcuggg cugcugccau ggggagccgu ugaaagagug gggcccugug      60

gcucggccgc gcgccucagg cugccagcgc gggcgggggg cggcgggggc gcgcggggcc     120

cgggccgggg ccggcgggcg ggcgggggcc ggcggaggac gcccacaagu ccagcggcgg     180

cggcggcggc agaggaggag gaggaggagg acgcggcggg gacgcggcga cucgggccgc     240

uccguaaccu ccgaggaucc gcgguagcgg uggacggguc gauccgugga guggaugucu     300

ucgggccauu gcggugcuug ggacucauua aacugucacu cacccccacc accaccccac     360

uggguuauga agacagu aug gag uuu cca gac cac agc aga cau uug cug       410
                   Met Glu Phe Pro Asp His Ser Arg His Leu Leu
                   1               5                   10

cag ugu cug agc gag cag agg cac cag ggc uuu cuu ugu gac ugc acu       458
Gln Cys Leu Ser Glu Gln Arg His Gln Gly Phe Leu Cys Asp Cys Thr
            15                  20                  25

guu cug gug gga gau gcc cag uuc cgg gca cac cga gcu guu cug gcu       506
Val Leu Val Gly Asp Ala Gln Phe Arg Ala His Arg Ala Val Leu Ala
        30                  35                  40

uca ugc agc aug uau uuc cac cuc uuu uac aag gac cag cug gac aaa       554
Ser Cys Ser Met Tyr Phe His Leu Phe Tyr Lys Asp Gln Leu Asp Lys
    45                  50                  55

aga gac auu guu cau cug aac agc gac auu gug aca gcc ccc gcu uuc       602
Arg Asp Ile Val His Leu Asn Ser Asp Ile Val Thr Ala Pro Ala Phe
60                  65                  70                  75

gcu cuc cug cuu gaa uuc aug uac gaa ggg aaa cuc cag uuc aaa gac       650
Ala Leu Leu Leu Glu Phe Met Tyr Glu Gly Lys Leu Gln Phe Lys Asp
                80                  85                  90

uug ccc auu gag gac gug cua gca gcu gcc agu uau cuc cac aug uau       698
Leu Pro Ile Glu Asp Val Leu Ala Ala Ala Ser Tyr Leu His Met Tyr
            95                  100                 105

gac auu guc aaa guc ugc aaa aag aag cug aaa gag aaa gcu acc aca       746
Asp Ile Val Lys Val Cys Lys Lys Lys Leu Lys Glu Lys Ala Thr Thr
        110                 115                 120

gag gcg gac agc acc aaa aaa gaa gaa gau gcu uca agu ugu ucg gau       794
Glu Ala Asp Ser Thr Lys Lys Glu Glu Asp Ala Ser Ser Cys Ser Asp
    125                 130                 135

aaa guc gag agc cuc uca gac ggc agc agc cac aug gca ggu gac cug       842
Lys Val Glu Ser Leu Ser Asp Gly Ser Ser His Met Ala Gly Asp Leu
140                 145                 150                 155

ccc agu gau gaa gau gaa ggc gaa gau gac aaa uug aac auu cua ccc       890

Pro Ser Asp Glu Asp Glu Gly Glu Asp Asp Lys Leu Asn Ile Leu Pro
                160                 165                 170

agc aaa agg gac uug gca gcu gag ccu ggg aac aug ugg aug cga uug 938
Ser Lys Arg Asp Leu Ala Ala Glu Pro Gly Asn Met Trp Met Arg Leu
            175                 180                 185

ccc uca gac uca gca ggc auc ccc cag gcu ggc gga gag gcc gag cca 986
Pro Ser Asp Ser Ala Gly Ile Pro Gln Ala Gly Gly Glu Ala Glu Pro
        190                 195                 200

cac gcc aca gca gcu gga aaa aca gua gcc agc ccc ugc agc uca aca 1034
His Ala Thr Ala Ala Gly Lys Thr Val Ala Ser Pro Cys Ser Ser Thr
    205                 210                 215

gag ucu uug ucc cag agg ucu guc acc ucc gug agg gau ucg gca gau 1082
Glu Ser Leu Ser Gln Arg Ser Val Thr Ser Val Arg Asp Ser Ala Asp
220                 225                 230                 235

guu gac ugu gug cug gac cug ucu guc aag ucc agc cuu uca gga guu 1130
Val Asp Cys Val Leu Asp Leu Ser Val Lys Ser Ser Leu Ser Gly Val
                240                 245                 250

gaa aau cug aac agc ucu uau uuc ucu uca cag gac gug cug aga agc 1178
Glu Asn Leu Asn Ser Ser Tyr Phe Ser Ser Gln Asp Val Leu Arg Ser
            255                 260                 265

aac cug gug cag gug aag gug gag aaa gag gcg ucc ugu gau gag agu 1226
Asn Leu Val Gln Val Lys Val Glu Lys Glu Ala Ser Cys Asp Glu Ser
        270                 275                 280

gau guu ggc acu aau gac uau gac aug gaa cau agc acu gug aaa gag 1274
Asp Val Gly Thr Asn Asp Tyr Asp Met Glu His Ser Thr Val Lys Glu
    285                 290                 295

agu gug agc acu aac aac agg gua cag uau gag cca gcc cac cug gcu 1322
Ser Val Ser Thr Asn Asn Arg Val Gln Tyr Glu Pro Ala His Leu Ala
300                 305                 310                 315

ccu cug agg gag gac ucg guc uua agg gaa cug gau cgg gag gac aaa 1370
Pro Leu Arg Glu Asp Ser Val Leu Arg Glu Leu Asp Arg Glu Asp Lys
                320                 325                 330

gcc agc gau gau gag aug aug acc cca gag agc gag cgg guc cag gug 1418
Ala Ser Asp Asp Glu Met Met Thr Pro Glu Ser Glu Arg Val Gln Val
            335                 340                 345

gaa ggg ggc aug gag aac agu cug cuu ccc uac guc ucc aau auc cug 1466
Glu Gly Gly Met Glu Asn Ser Leu Leu Pro Tyr Val Ser Asn Ile Leu
        350                 355                 360

agc ccc gca ggc cag auc uuc aug ugc ccc cug ugc aac aaa guc uuc 1514
Ser Pro Ala Gly Gln Ile Phe Met Cys Pro Leu Cys Asn Lys Val Phe
    365                 370                 375

ccc agc ccc cac auc cug cag auc cac cug agc acg cac uuc cga gag 1562
Pro Ser Pro His Ile Leu Gln Ile His Leu Ser Thr His Phe Arg Glu
380                 385                 390                 395

cag gau ggc auc cgu agc aag ccu gcc gcc gau guc aau gug ccc acg 1610
Gln Asp Gly Ile Arg Ser Lys Pro Ala Ala Asp Val Asn Val Pro Thr
                400                 405                 410

ugc ucc cug ugu ggg aag acu uuc ucc ugc aug uac acc cuc aag cgc     1658
Cys Ser Leu Cys Gly Lys Thr Phe Ser Cys Met Tyr Thr Leu Lys Arg
            415                 420                 425

cac gag agg acu cac ucg ggg gag aag ccc uac acg ugc acc caa ugc     1706
His Glu Arg Thr His Ser Gly Glu Lys Pro Tyr Thr Cys Thr Gln Cys
        430                 435                 440

ggc aag agu uuc cag uac ucg cac aac cug agc cgu cau gcc gug gug     1754
Gly Lys Ser Phe Gln Tyr Ser His Asn Leu Ser Arg His Ala Val Val
    445                 450                 455

cac acc cgc gag aag ccc cau gcc ugc aag ugg ugc gag cgc agg uuc     1802
His Thr Arg Glu Lys Pro His Ala Cys Lys Trp Cys Glu Arg Arg Phe
460                 465                 470                 475

acg cag ucc gga gac cug uac aga cac auc cgc aag uuc cac ugu gag     1850
Thr Gln Ser Gly Asp Leu Tyr Arg His Ile Arg Lys Phe His Cys Glu
                480                 485                 490

uug gug aac ucc uug ucg guc aaa agu gaa gcg cug agc uug ccc acu     1898
Leu Val Asn Ser Leu Ser Val Lys Ser Glu Ala Leu Ser Leu Pro Thr
            495                 500                 505

guc aga gac ugg acc uua gaa gau agc ucu caa gaa cuu ugg aaa uaa     1946
Val Arg Asp Trp Thr Leu Glu Asp Ser Ser Gln Glu Leu Trp Lys
        510                 515                 520

uuuuauauau auauauauau aaauaauaua uauauauaua uauacauaua uauacauaga     2006

ucucuauaua guugugguac ggucuaaaag cagucuuguu uccuggaacu gaaaaguugg     2066

gaucuuaacu uguuuuugca cuuuagaaua auagcaugag aaucucacua auuuagcauu     2126

cugauaaaag aaacuagagc aagucacaag uagagagcug ucuuucuuuu gaggguuagg     2186

ccaaguuagc caauaaaacc uucucaacaa augguucugu cacaaacgcu uucacauggc     2246

uuacuuuugu acacacugca uugucuugag cgcuuucccu gugcuccaca cuguuuuguu     2306

uuguuuuguu uucagaguag aaauccccuc caguuuauua gccucuuuau augucucaaa     2366

uugcaugaau auuuucuggc uguuggaaac cugaaugcuu uuagacccaa auggaaaauu     2426

ucugaaaugc uggauuaucu auuuuuaaac aagcaguuga cuuaaaacuu uguguggcaa     2486

cuucugguuu ucugaugguc cccagugaga gaaaucugac aguacacugg gucacuggga     2546

cugucucugu gaagguuugc uugagaugaa aaaggauauu gcagcuucag caguguugaa     2606

cuguguguuu aaaaugugaa uuacuguucu uguauacugu aauugauuac acggcugggg     2666

ggggguguca aagaacuuga cagguugugu ugaugcucuu aguugagucu ugaaaaguaa     2726

auauuaacgc uacagaaaug caugaguuuc aauauauuuu uugucuuugu uugcauugua     2786

uaacuuuaac gagugaguuu aaaauuauuu aauuuccuua gaaaaauagc accauuuggg     2846

aaaaaaacug guguuacgaa gaacauaaau gcacugucuu uauuuuuauu uuuauuuuau     2906

auaauuuaaa uugacuuuuc ccacgucuuu aaguuugaaa cuguuaagcu gaauaaaaac     2966

uuaagcugca aauugauaac uucgcuacau uacaaggaac auauaaaugu uuacaaacgg     3026

cuuaaagaau ugcaugugca gugugcauuc auaccacacu uguaauugca cagaacccau 3086

gccagcucag aguuuaggug uacacacgua cccagccgag uguuuuuaga auaacuacug 3146

cacaaauuga caauagguca uaggucauuc ucucucuccc uccuuuuugc uuccccuuuc 3206

uuuuucuccc cuauuuccuu uccuuccccc uacccccuaa cucaugaaaa gauucuaugg 3266

acugaaaaag ccccaggcug aaaggacuga accgccuuga uugacauggg gagggggguu 3326

aguagacugu guaucggcag cagaggcugc agcccgaugu gugguucuga uggccagcgc 3386

ggggcagaag cguuuugcac acuguuuguu uuccugucuu gcacuuacaa auaaggucua 3446

ugggaguagc augggaaaca gugguuguuu uucccuuuuu cuuuuuuaau ugcuuuuguu 3506

uaaaauuuga ucgccuuaac uacuguaaac auagccuauu uuugugcuua agauacugaa 3566

uggaaaacuc cauugugugu ugcuggacug uuuuggaaau auuuggucaa auguauguua 3626

auuuggcugu aauggcauuu aaagcaaaca aacaaacaaa caaacaaagc ugugaaaaug 3686

gccuuggagc auuaucuuua guuacuugaa gaguuucuag uuuuuuuuuu uuuaauacag 3746

uuuauguuaa aauaaucuuu auuaaucuag aggagacaau caaugucugu ggaagaacgg 3806

acuuuuuugg auuuucuuuu uuaaugguca uugugaguga uugcucuuuc uuuuacuuag 3866

uuucacauuc uuccuuuguu cuaaaucuua gacugacauc uagcuuugac aaucauagua 3926

uguuuuauuu ccugaggggg aauaacuuau aaugcuguuu aguuuuguac uauuggugug 3986

uuggugaauu uuuaaacugu gugcuaacug caauaaauua uaugaacuga gaa 4039

<210> 23
<211> 3810
<212> RNA
<213> Mus musculus

<220>
<221> CDS
<222> (149)..(1717)

<400> 23
ucccgccggg cggaguuagu uaguguggcg gagggcaggc ggugcgggcu cgccucgccu 60

cgccuggccg cgcuccccgg ccggugcagc ugcggcgcgg ggcggacucg gugcagcugc 120

ggcggcgccu cccgguuaug aagacagu aug gag uuu cca gac cac agc aga 172
Met Glu Phe Pro Asp His Ser Arg
1               5

cau uug cug cag ugu cug agc gag cag agg cac cag ggc uuu cuu ugu 220
His Leu Leu Gln Cys Leu Ser Glu Gln Arg His Gln Gly Phe Leu Cys
    10                  15                  20

gac ugc acu guu cug gug gga gau gcc cag uuc cgg gca cac cga gcu 268
Asp Cys Thr Val Leu Val Gly Asp Ala Gln Phe Arg Ala His Arg Ala
25                  30                  35                  40

guu cug gcu uca ugc agc aug uau uuc cac cuc uuu uac aag gac cag 316
Val Leu Ala Ser Cys Ser Met Tyr Phe His Leu Phe Tyr Lys Asp Gln
                45                  50                  55

cug gac aaa aga gac auu guu cau cug aac agc gac auu gug aca gcc 364
Leu Asp Lys Arg Asp Ile Val His Leu Asn Ser Asp Ile Val Thr Ala
            60                  65                  70

ccc gcu uuc gcu cuc cug cuu gaa uuc aug uac gaa ggg aaa cuc cag 412
Pro Ala Phe Ala Leu Leu Leu Glu Phe Met Tyr Glu Gly Lys Leu Gln
        75                  80                  85

uuc aaa gac uug ccc auu gag gac gug cua gca gcu gcc agu uau cuc 460
Phe Lys Asp Leu Pro Ile Glu Asp Val Leu Ala Ala Ala Ser Tyr Leu
    90                  95                  100

cac aug uau gac auu guc aaa guc ugc aaa aag aag cug aaa gag aaa 508
His Met Tyr Asp Ile Val Lys Val Cys Lys Lys Lys Leu Lys Glu Lys
105                 110                 115                 120

gcu acc aca gag gcg gac agc acc aaa aaa gaa gaa gau gcu uca agu 556
Ala Thr Thr Glu Ala Asp Ser Thr Lys Lys Glu Glu Asp Ala Ser Ser
                125                 130                 135

ugu ucg gau aaa guc gag agc cuc uca gac ggc agc agc cac aug gca 604
Cys Ser Asp Lys Val Glu Ser Leu Ser Asp Gly Ser Ser His Met Ala
            140                 145                 150

ggu gac cug ccc agu gau gaa gau gaa ggc gaa gau gac aaa uug aac 652
Gly Asp Leu Pro Ser Asp Glu Asp Glu Gly Glu Asp Asp Lys Leu Asn
        155                 160                 165

auu cua ccc agc aaa agg gac uug gca gcu gag ccu ggg aac aug ugg 700
Ile Leu Pro Ser Lys Arg Asp Leu Ala Ala Glu Pro Gly Asn Met Trp
    170                 175                 180

aug cga uug ccc uca gac uca gca ggc auc ccc cag gcu ggc gga gag 748
Met Arg Leu Pro Ser Asp Ser Ala Gly Ile Pro Gln Ala Gly Gly Glu
185                 190                 195                 200

gcc gag cca cac gcc aca gca gcu gga aaa aca gua gcc agc ccc ugc 796
Ala Glu Pro His Ala Thr Ala Ala Gly Lys Thr Val Ala Ser Pro Cys
                205                 210                 215

agc uca aca gag ucu uug ucc cag agg ucu guc acc ucc gug agg gau 844
Ser Ser Thr Glu Ser Leu Ser Gln Arg Ser Val Thr Ser Val Arg Asp
            220                 225                 230

ucg gca gau guu gac ugu gug cug gac cug ucu guc aag ucc agc cuu 892
Ser Ala Asp Val Asp Cys Val Leu Asp Leu Ser Val Lys Ser Ser Leu
        235                 240                 245

uca gga guu gaa aau cug aac agc ucu uau uuc ucu uca cag gac gug 940
Ser Gly Val Glu Asn Leu Asn Ser Ser Tyr Phe Ser Ser Gln Asp Val
    250                 255                 260

cug aga agc aac cug gug cag gug aag gug gag aaa gag gcg ucc ugu 988
Leu Arg Ser Asn Leu Val Gln Val Lys Val Glu Lys Glu Ala Ser Cys
265                 270                 275                 280

gau gag agu gau guu ggc acu aau gac uau gac aug gaa cau agc acu 1036
Asp Glu Ser Asp Val Gly Thr Asn Asp Tyr Asp Met Glu His Ser Thr
                285                 290                 295

gug aaa gag agu gug agc acu aac aac agg gua cag uau gag cca gcc 1084
Val Lys Glu Ser Val Ser Thr Asn Asn Arg Val Gln Tyr Glu Pro Ala
            300                 305                 310

cac cug gcu ccu cug agg gag gac ucg guc uua agg gaa cug gau cgg 1132
His Leu Ala Pro Leu Arg Glu Asp Ser Val Leu Arg Glu Leu Asp Arg
        315                 320                 325

gag gac aaa gcc agc gau gau gag aug aug acc cca gag agc gag cgg 1180
Glu Asp Lys Ala Ser Asp Asp Glu Met Met Thr Pro Glu Ser Glu Arg
    330                 335                 340

guc cag gug gaa ggg ggc aug gag aac agu cug cuu ccc uac guc ucc 1228
Val Gln Val Glu Gly Gly Met Glu Asn Ser Leu Leu Pro Tyr Val Ser
345                 350                 355                 360

aau auc cug agc ccc gca ggc cag auc uuc aug ugc ccc cug ugc aac 1276
Asn Ile Leu Ser Pro Ala Gly Gln Ile Phe Met Cys Pro Leu Cys Asn
                365                 370                 375

aaa guc uuc ccc agc ccc cac auc cug cag auc cac cug agc acg cac 1324
Lys Val Phe Pro Ser Pro His Ile Leu Gln Ile His Leu Ser Thr His
            380                 385                 390

uuc cga gag cag gau ggc auc cgu agc aag ccu gcc gcc gau guc aau 1372
Phe Arg Glu Gln Asp Gly Ile Arg Ser Lys Pro Ala Ala Asp Val Asn
        395                 400                 405

gug ccc acg ugc ucc cug ugu ggg aag acu uuc ucc ugc aug uac acc 1420
Val Pro Thr Cys Ser Leu Cys Gly Lys Thr Phe Ser Cys Met Tyr Thr
    410                 415                 420

cuc aag cgc cac gag agg acu cac ucg ggg gag aag ccc uac acg ugc 1468
Leu Lys Arg His Glu Arg Thr His Ser Gly Glu Lys Pro Tyr Thr Cys
425                 430                 435                 440

acc caa ugc ggc aag agu uuc cag uac ucg cac aac cug agc cgu cau 1516
Thr Gln Cys Gly Lys Ser Phe Gln Tyr Ser His Asn Leu Ser Arg His
                445                 450                 455

gcc gug gug cac acc cgc gag aag ccc cau gcc ugc aag ugg ugc gag 1564
Ala Val Val His Thr Arg Glu Lys Pro His Ala Cys Lys Trp Cys Glu
            460                 465                 470

cgc agg uuc acg cag ucc gga gac cug uac aga cac auc cgc aag uuc 1612
Arg Arg Phe Thr Gln Ser Gly Asp Leu Tyr Arg His Ile Arg Lys Phe
        475                 480                 485

cac ugu gag uug gug aac ucc uug ucg guc aaa agu gaa gcg cug agc 1660
His Cys Glu Leu Val Asn Ser Leu Ser Val Lys Ser Glu Ala Leu Ser
    490                 495                 500

uug ccc acu guc aga gac ugg acc uua gaa gau agc ucu caa gaa cuu 1708
Leu Pro Thr Val Arg Asp Trp Thr Leu Glu Asp Ser Ser Gln Glu Leu
505                 510                 515                 520

ugg aaa uaa uuuuauauau auauauauau aaauaauaua uauauauaua 1757
Trp Lys

uauacauaua uauacauaga ucucuauaua guugugguac ggucuaaaag cagucuuguu 1817

uccuggaacu gaaaaguugg gaucuuaacu uguuuuugca cuuuagaaua auagcaugag 1877

aaucucacua auuuagcauu cugauaaaag aaacuagagc aagucacaag uagagagcug 1937

ucuuucuuuu gaggguuagg ccaaguuagc caauaaaacc uucucaacaa augguucugu 1997

cacaaacgcu uucacauggc uuacuuuugu acacacugca uugucuugag cgcuuucccu 2057

gugcuccaca cuguuuuguu uuguuuuguu uucagaguag aaauccccuc caguuuauua 2117

gccucuuuau augucucaaa uugcaugaau auuuucuggc uguuggaaac cugaaugcuu 2177

uuagacccaa auggaaaauu ucugaaaugc uggauuaucu auuuuuaaac aagcaguuga 2237

cuuaaaacuu uguguggcaa cuucugguuu ucugaugguc cccagugaga gaaaucugac 2297

aguacacugg gucacuggga cugucucugu gaagguuugc uugagaugaa aaaggauauu 2357

gcagcuucag caguguugaa cuguguguuu aaaaugugaa uuacuguucu uguauacugu 2417

aauugauuac acgggcuggg ggggguguca aagaacuuga cagguugugu ugaugcucuu 2477

aguugagucu ugaaaaguaa auauuaacgc uacagaaaug caugaguuuc aauauauuuu 2537

uugucuuugu uugcauugua uaacuuuaac gagugaguuu aaaauuauuu aauuuccuua 2597

gaaaaauagc accauuuggg aaaaaaacug guguuacgaa gaacauaaau gcacugucuu 2657

uauuuuuauu uuuauuuuau auaauuuaaa uugacuuuuc ccacgucuuu aaguuugaaa 2717

cuguuaagcu gaauaaaaac uuaagcugca aauugauaac uucgcuacau uacaaggaac 2777

auauaaaugu uuacaaacgg cuuaaagaau ugcaugugca gugugcauuc auaccacacu 2837

uguaauugca cagaacccau gccagcucag aguuuaggug uacacacgua cccagccgag 2897

uguuuuuaga auaacuacug cacaaauuga caauagguca uaggucauuc ucucucuccc 2957

uccuuuuugc uuccccuuuc uuuuucuccc cuauuuccuu uccuuccccc uacccccuaa 3017

cucaugaaaa gauucuaugg acugaaaaag ccccaggcug aaaggacuga accgccuuga 3077

uugacauggg gagggggguu aguagacugu guaucggcag cagaggcugc agcccgaugu 3137

gugguucuga uggccagcgc ggggcagaag cguuuugcac acuguuuguu uuccugucuu 3197

gcacuuacaa auaaggucua ugggaguagc augggaaaca gugguuguuu uucccuuuuu 3257

cuuuuuuaau ugcuuuuguu uaaaauuuga ucgccuuaac uacuguaaac auagccuauu 3317

uuugugcuua agauacugaa uggaaaacuc cauugugugu ugcuggacug uuuuggaaau 3377

auuuggucaa auguauguua auuuggcugu aauggcauuu aaagcaaaca aacaaacaaa 3437

caaacaaagc ugugaaaaug gccuuggagc auuaucuuua guuacuugaa gaguuucuag 3497

uuuuuuuuuu uuuaauacag uuuauguuaa aauaaucuuu auuaaucuag aggagacaau 3557

caaugucugu ggaagaacgg acuuuuuugg auuuucuuuu uuaaugguca uugugaguga 3617

uugcucuuuc uuuuacuuag uuucacauuc uuccuuuguu cuaaaucuua gacugacauc 3677

uagcuuugac aaucauagua uguuuuauuu ccugaggggg aauaacuuau aaugcuguuu 3737

agtuuuguac uauuggugug uuggugaauu uuuaaacugu gugcuaacug caauaaauua 3797

uaugaacuga gaa 3810

<210> 24
<211> 4312
<212> RNA
<213> Mus musculus

<220>
<221> CDS
<222> (624)..(2219)

<400> 24
guugcgguug ccgggaggcc ccgggggcug gcgcgcggcg gcggcgacgg gggagcgguc 60

gccgcgccgc uucuugcgac uuuucuuuug uuguugccgc gaccgggguc uguuuucucg 120

gccguugugg aaggugcuuu ccugcugcac cggcucucug cccugcaccu ucuuccccgu 180

gguuucaccc uuccucuuuc ccugaagugc uuuaugcacc gcgaacugcg ggaagauuug 240

caucuccucu cccaccccug aaccucacac acacacccgg ggcacccugc ccccaccucu 300

gaaaaacaaa cccccuaacc ucacagaacc uccgaggguc cgcgguagcg guggacgggu 360

cgauccgugg aguggauguc uucgggccau ugcggugcuu gggacucauu aaacugucac 420

ucacccccac caccacccca cugguauuuc agaagugcag uuugaguuug uugaugcuga 480

aaguggaaau uugcacuaac gguggcagug ugaacuauuu uccugagagg uagauccaga 540

uaaauugcuu agucugcuuu uuuuccaccg auguaacaga ccugcagcca gcaagacucc 600

gagggaagga cuuaaucaag uuu aug ugu ccu aaa ggu uau gaa gac agu aug 653
Met Cys Pro Lys Gly Tyr Glu Asp Ser Met
1               5                   10

gag uuu cca gac cac agc aga cau uug cug cag ugu cug agc gag cag 701
Glu Phe Pro Asp His Ser Arg His Leu Leu Gln Cys Leu Ser Glu Gln
                15                  20                  25

agg cac cag ggc uuu cuu ugu gac ugc acu guu cug gug gga gau gcc 749
Arg His Gln Gly Phe Leu Cys Asp Cys Thr Val Leu Val Gly Asp Ala
            30                  35                  40

cag uuc cgg gca cac cga gcu guu cug gcu uca ugc agc aug uau uuc 797
Gln Phe Arg Ala His Arg Ala Val Leu Ala Ser Cys Ser Met Tyr Phe
        45                  50                  55

cac cuc uuu uac aag gac cag cug gac aaa aga gac auu guu cau cug 845
His Leu Phe Tyr Lys Asp Gln Leu Asp Lys Arg Asp Ile Val His Leu
    60                  65                  70

aac agc gac auu gug aca gcc ccc gcu uuc gcu cuc cug cuu gaa uuc 893
Asn Ser Asp Ile Val Thr Ala Pro Ala Phe Ala Leu Leu Leu Glu Phe
75                  80                  85                  90

aug uac gaa ggg aaa cuc cag uuc aaa gac uug ccc auu gag gac gug 941
Met Tyr Glu Gly Lys Leu Gln Phe Lys Asp Leu Pro Ile Glu Asp Val

                95                  100                 105

cua gca gcu gcc agu uau cuc cac aug uau gac auu guc aaa guc ugc 989
Leu Ala Ala Ala Ser Tyr Leu His Met Tyr Asp Ile Val Lys Val Cys
            110                 115                 120

aaa aag aag cug aaa gag aaa gcu acc aca gag gcg gac agc acc aaa 1037
Lys Lys Lys Leu Lys Glu Lys Ala Thr Thr Glu Ala Asp Ser Thr Lys
        125                 130                 135

aaa gaa gaa gau gcu uca agu ugu ucg gau aaa guc gag agc cuc uca 1085
Lys Glu Glu Asp Ala Ser Ser Cys Ser Asp Lys Val Glu Ser Leu Ser
    140                 145                 150

gac ggc agc agc cac aug gca ggu gac cug ccc agu gau gaa gau gaa 1133
Asp Gly Ser Ser His Met Ala Gly Asp Leu Pro Ser Asp Glu Asp Glu
155                 160                 165                 170

ggc gaa gau gac aaa uug aac auu cua ccc agc aaa agg gac uug gca 1181
Gly Glu Asp Asp Lys Leu Asn Ile Leu Pro Ser Lys Arg Asp Leu Ala
                175                 180                 185

gcu gag ccu ggg aac aug ugg aug cga uug ccc uca gac uca gca ggc 1229
Ala Glu Pro Gly Asn Met Trp Met Arg Leu Pro Ser Asp Ser Ala Gly
            190                 195                 200

auc ccc cag gcu ggc gga gag gcc gag cca cac gcc aca gca gcu gga 1277
Ile Pro Gln Ala Gly Gly Glu Ala Glu Pro His Ala Thr Ala Ala Gly
        205                 210                 215

aaa aca gua gcc agc ccc ugc agc uca aca gag ucu uug ucc cag agg 1325
Lys Thr Val Ala Ser Pro Cys Ser Ser Thr Glu Ser Leu Ser Gln Arg
    220                 225                 230

ucu guc acc ucc gug agg gau ucg gca gau guu gac ugu gug cug gac 1373
Ser Val Thr Ser Val Arg Asp Ser Ala Asp Val Asp Cys Val Leu Asp
235                 240                 245                 250

cug ucu guc aag ucc agc cuu uca gga guu gaa aau cug aac agc ucu 1421
Leu Ser Val Lys Ser Ser Leu Ser Gly Val Glu Asn Leu Asn Ser Ser
                255                 260                 265

uau uuc ucu uca cag gac gug cug aga agc aac cug gug cag gug aag 1469
Tyr Phe Ser Ser Gln Asp Val Leu Arg Ser Asn Leu Val Gln Val Lys
            270                 275                 280

gug gag aaa gag gcg ucc ugu gau gag agu gau guu ggc acu aau gac 1517
Val Glu Lys Glu Ala Ser Cys Asp Glu Ser Asp Val Gly Thr Asn Asp
        285                 290                 295

uau gac aug gaa cau agc acu gug aaa gag agu gug agc acu aac aac 1565
Tyr Asp Met Glu His Ser Thr Val Lys Glu Ser Val Ser Thr Asn Asn
    300                 305                 310

agg gua cag uau gag cca gcc cac cug gcu ccu cug agg gag gac ucg 1613
Arg Val Gln Tyr Glu Pro Ala His Leu Ala Pro Leu Arg Glu Asp Ser
315                 320                 325                 330

guc uua agg gaa cug gau cgg gag gac aaa gcc agc gau gau gag aug 1661
Val Leu Arg Glu Leu Asp Arg Glu Asp Lys Ala Ser Asp Asp Glu Met
                335                 340                 345

aug acc cca gag agc gag cgg guc cag gug gaa ggg ggc aug gag aac 1709

Met Thr Pro Glu Ser Glu Arg Val Gln Val Glu Gly Gly Met Glu Asn
            350                 355                 360

agu cug cuu ccc uac guc ucc aau auc cug agc ccc gca ggc cag auc      1757
Ser Leu Leu Pro Tyr Val Ser Asn Ile Leu Ser Pro Ala Gly Gln Ile
        365                 370                 375

uuc aug ugc ccc cug ugc aac aaa guc uuc ccc agc ccc cac auc cug      1805
Phe Met Cys Pro Leu Cys Asn Lys Val Phe Pro Ser Pro His Ile Leu
    380                 385                 390

cag auc cac cug agc acg cac uuc cga gag cag gau ggc auc cgu agc      1853
Gln Ile His Leu Ser Thr His Phe Arg Glu Gln Asp Gly Ile Arg Ser
395                 400                 405                 410

aag ccu gcc gcc gau guc aau gug ccc acg ugc ucc cug ugu ggg aag      1901
Lys Pro Ala Ala Asp Val Asn Val Pro Thr Cys Ser Leu Cys Gly Lys
                415                 420                 425

acu uuc ucc ugc aug uac acc cuc aag cgc cac gag agg acu cac ucg      1949
Thr Phe Ser Cys Met Tyr Thr Leu Lys Arg His Glu Arg Thr His Ser
            430                 435                 440

ggg gag aag ccc uac acg ugc acc caa ugc ggc aag agu uuc cag uac      1997
Gly Glu Lys Pro Tyr Thr Cys Thr Gln Cys Gly Lys Ser Phe Gln Tyr
        445                 450                 455

ucg cac aac cug agc cgu cau gcc gug gug cac acc cgc gag aag ccc      2045
Ser His Asn Leu Ser Arg His Ala Val Val His Thr Arg Glu Lys Pro
    460                 465                 470

cau gcc ugc aag ugg ugc gag cgc agg uuc acg cag ucc gga gac cug      2093
His Ala Cys Lys Trp Cys Glu Arg Arg Phe Thr Gln Ser Gly Asp Leu
475                 480                 485                 490

uac aga cac auc cgc aag uuc cac ugu gag uug gug aac ucc uug ucg      2141
Tyr Arg His Ile Arg Lys Phe His Cys Glu Leu Val Asn Ser Leu Ser
                495                 500                 505

guc aaa agu gaa gcg cug agc uug ccc acu guc aga gac ugg acc uua      2189
Val Lys Ser Glu Ala Leu Ser Leu Pro Thr Val Arg Asp Trp Thr Leu
            510                 515                 520

gaa gau agc ucu caa gaa cuu ugg aaa uaa uuuuauauau auauauauau      2239
Glu Asp Ser Ser Gln Glu Leu Trp Lys
        525                 530

aaauaauaua uauauauaua uauacauaua uauacauaga ucucuauaua guugugguac      2299

ggucuaaaag cagucuuguu uccuggaacu gaaaaguugg gaucuuaacu uguuuuugca      2359

cuuuagaaua auagcaugag aaucucacua auuuagcauu cugauaaaag aaacuagagc      2419

aagucacaag uagagagcug ucuuucuuuu gaggguuagg ccaaguuagc caauaaaacc      2479

uucucaacaa augguucugu cacaaacgcu uucacauggc uuacuuuugu acacacugca      2539

uugucuugag cgcuuucccu gugcuccaca cuguuuuguu uuguuuuguu uucagaguag      2599

aaauccccuc caguuuauua gccucuuuau augucucaaa uugcaugaau auuuucuggc      2659

uguuggaaac cugaaugcuu uuagacccaa auggaaaauu ucugaaaugc uggauuaucu      2719

auuuuuaaac aagcaguuga cuuaaaacuu uguguggcaa cuucugguuu ucugaugguc 2779

cccagugaga gaaaucugac aguacacugg gucacuggga cugucucugu gaagguuugc 2839

uugagaugaa aaaggauauu gcagcuucag caguguugaa cuguguguuu aaaaugugaa 2899

uuacuguucu uguauacugu aauugauuac acgggcuggg ggggguguca aagaacuuga 2959

cagguugugu ugaugcucuu aguugagucu ugaaaaguaa auauuaacgc uacagaaaug 3019

caugaguuuc aauauauuuu uugucuuugu uugcauugua uaacuuuaac gagugaguuu 3079

aaaauuauuu aauuuccuua gaaaaauagc accauuuggg aaaaaaacug guguuacgaa 3139

gaacauaaau gcacugucuu uauuuuuauu uuuauuuuau auaauuuaaa uugacuuuuc 3199

ccacgucuuu aaguuugaaa cuguuaagcu gaauaaaaac uuaagcugca aauugauaac 3259

uucgcuacau uacaaggaac auauaaaugu uuacaaacgg cuuaaagaau ugcaugugca 3319

gugugcauuc auaccacacu uguaauugca cagaacccau gccagcucag aguuuaggug 3379

uacacacgua cccagccgag uguuuuuaga auaacuacug cacaaauuga caauagguca 3439

uaggucauuc ucucucuccc uccuuuuugc uuccccuuuc uuuuucuccc cuauuuccuu 3499

uccuuccccc uacccccuaa cucaugaaaa gauucuaugg acugaaaaag ccccaggcug 3559

aaaggacuga accgccuuga uugacauggg gagggggguu aguagacugu guaucggcag 3619

cagaggcugc agcccgaugu gugguucuga uggccagcgc ggggcagaag cguuuugcac 3679

acuguuuguu uuccugucuu gcacuuacaa auaaggucua ugggaguagc augggaaaca 3739

gugguuguuu uucccuuuuu cuuuuuuaau ugcuuuuguu uaaaauuuga ucgccuuaac 3799

uacuguaaac auagccuauu uuugugcuua agauacugaa uggaaaacuc cauugugugu 3859

ugcuggacug uuuuggaaau auuuggucaa auguauguua auuuggcugu aauggcauuu 3919

aaagcaaaca aacaaacaaa caaacaaagc ugugaaaaug gccuuggagc auuaucuuua 3979

guuacuugaa gaguuucuag uuuuuuuuuu uuuaauacag uuuauguuaa aauaaucuuu 4039

auuaaucuag aggagacaau caaugucugu ggaagaacgg acuuuuuugg auuuucuuuu 4099

uuaaugguca uugugaguga uugcucuuuc uuuuacuuag uuucacauuc uuccuuuguu 4159

cuaaaucuua gacugacauc uagcuuugac aaucauagua uguuuuauuu ccugaggggg 4219

aauaacuuau aaugcuguuu aguuuuguac uauuggugug uuggugaauu uuuaaacugu 4279

gugcuaacug caauaaauua uaugaacuga gaa 4312

<210> 25
<211> 4204
<212> RNA
<213> Mus musculus

<220>
<221> CDS

<222>  (516)..(2111)

<400>  25
guugcgguug ccgggaggcc ccgggggcug gcgcgcggcg gcggcgacgg gggagcgguc      60

gccgcgccgc uucuugcgac uuuucuuuug uuguugccgc gaccgggguc uguuuucucg      120

gccguugugg aaggugcuuu ccugcugcac cggcucucug cccugcaccu ucuuccccgu      180

gguuucaccc uuccucuuuc ccugaagugc uuuaugcacc gcgaacugcg ggaagauuug      240

caucuccucu cccaccccug aaccucacac acacacccgg ggcacccugc ccccaccucu      300

gaaaaacaaa cccccuaacc ucacagaacc uccgaggguc cgcgguagcg guggacgggu      360

cgauccgugg aguggauguc uucgggccau ugcggugcuu gggacucauu aaacugucac      420

ucacccccac caccacccca cuggucugcu uuuuuuccac cgauguaaca gaccugcagc      480

cagcaagacu ccgagggaag gacuuaauca aguuu aug ugu ccu aaa ggu uau      533
                                       Met Cys Pro Lys Gly Tyr
                                       1               5

gaa gac agu aug gag uuu cca gac cac agc aga cau uug cug cag ugu      581
Glu Asp Ser Met Glu Phe Pro Asp His Ser Arg His Leu Leu Gln Cys
            10                  15                  20

cug agc gag cag agg cac cag ggc uuu cuu ugu gac ugc acu guu cug      629
Leu Ser Glu Gln Arg His Gln Gly Phe Leu Cys Asp Cys Thr Val Leu
        25                  30                  35

gug gga gau gcc cag uuc cgg gca cac cga gcu guu cug gcu uca ugc      677
Val Gly Asp Ala Gln Phe Arg Ala His Arg Ala Val Leu Ala Ser Cys
    40                  45                  50

agc aug uau uuc cac cuc uuu uac aag gac cag cug gac aaa aga gac      725
Ser Met Tyr Phe His Leu Phe Tyr Lys Asp Gln Leu Asp Lys Arg Asp
55                  60                  65                  70

auu guu cau cug aac agc gac auu gug aca gcc ccc gcu uuc gcu cuc      773
Ile Val His Leu Asn Ser Asp Ile Val Thr Ala Pro Ala Phe Ala Leu
                75                  80                  85

cug cuu gaa uuc aug uac gaa ggg aaa cuc cag uuc aaa gac uug ccc      821
Leu Leu Glu Phe Met Tyr Glu Gly Lys Leu Gln Phe Lys Asp Leu Pro
            90                  95                  100

auu gag gac gug cua gca gcu gcc agu uau cuc cac aug uau gac auu      869
Ile Glu Asp Val Leu Ala Ala Ala Ser Tyr Leu His Met Tyr Asp Ile
        105                 110                 115

guc aaa guc ugc aaa aag aag cug aaa gag aaa gcu acc aca gag gcg      917
Val Lys Val Cys Lys Lys Lys Leu Lys Glu Lys Ala Thr Thr Glu Ala
    120                 125                 130

gac agc acc aaa aaa gaa gaa gau gcu uca agu ugu ucg gau aaa guc      965
Asp Ser Thr Lys Lys Glu Glu Asp Ala Ser Ser Cys Ser Asp Lys Val
135                 140                 145                 150

gag agc cuc uca gac ggc agc agc cac aug gca ggu gac cug ccc agu      1013
Glu Ser Leu Ser Asp Gly Ser Ser His Met Ala Gly Asp Leu Pro Ser
                155                 160                 165

gau gaa gau gaa ggc gaa gau gac aaa uug aac auu cua ccc agc aaa 1061
Asp Glu Asp Glu Gly Glu Asp Asp Lys Leu Asn Ile Leu Pro Ser Lys
            170                 175                 180

agg gac uug gca gcu gag ccu ggg aac aug ugg aug cga uug ccc uca 1109
Arg Asp Leu Ala Ala Glu Pro Gly Asn Met Trp Met Arg Leu Pro Ser
        185                 190                 195

gac uca gca ggc auc ccc cag gcu ggc gga gag gcc gag cca cac gcc 1157
Asp Ser Ala Gly Ile Pro Gln Ala Gly Gly Glu Ala Glu Pro His Ala
    200                 205                 210

aca gca gcu gga aaa aca gua gcc agc ccc ugc agc uca aca gag ucu 1205
Thr Ala Ala Gly Lys Thr Val Ala Ser Pro Cys Ser Ser Thr Glu Ser
215                 220                 225                 230

uug ucc cag agg ucu guc acc ucc gug agg gau ucg gca gau guu gac 1253
Leu Ser Gln Arg Ser Val Thr Ser Val Arg Asp Ser Ala Asp Val Asp
                235                 240                 245

ugu gug cug gac cug ucu guc aag ucc agc cuu uca gga guu gaa aau 1301
Cys Val Leu Asp Leu Ser Val Lys Ser Ser Leu Ser Gly Val Glu Asn
            250                 255                 260

cug aac agc ucu uau uuc ucu uca cag gac gug cug aga agc aac cug 1349
Leu Asn Ser Ser Tyr Phe Ser Ser Gln Asp Val Leu Arg Ser Asn Leu
        265                 270                 275

gug cag gug aag gug gag aaa gag gcg ucc ugu gau gag agu gau guu 1397
Val Gln Val Lys Val Glu Lys Glu Ala Ser Cys Asp Glu Ser Asp Val
    280                 285                 290

ggc acu aau gac uau gac aug gaa cau agc acu gug aaa gag agu gug 1445
Gly Thr Asn Asp Tyr Asp Met Glu His Ser Thr Val Lys Glu Ser Val
295                 300                 305                 310

agc acu aac aac agg gua cag uau gag cca gcc cac cug gcu ccu cug 1493
Ser Thr Asn Asn Arg Val Gln Tyr Glu Pro Ala His Leu Ala Pro Leu
                315                 320                 325

agg gag gac ucg guc uua agg gaa cug gau cgg gag gac aaa gcc agc 1541
Arg Glu Asp Ser Val Leu Arg Glu Leu Asp Arg Glu Asp Lys Ala Ser
            330                 335                 340

gau gau gag aug aug acc cca gag agc gag cgg guc cag gug gaa ggg 1589
Asp Asp Glu Met Met Thr Pro Glu Ser Glu Arg Val Gln Val Glu Gly
        345                 350                 355

ggc aug gag aac agu cug cuu ccc uac guc ucc aau auc cug agc ccc 1637
Gly Met Glu Asn Ser Leu Leu Pro Tyr Val Ser Asn Ile Leu Ser Pro
    360                 365                 370

gca ggc cag auc uuc aug ugc ccc cug ugc aac aaa guc uuc ccc agc 1685
Ala Gly Gln Ile Phe Met Cys Pro Leu Cys Asn Lys Val Phe Pro Ser
375                 380                 385                 390

ccc cac auc cug cag auc cac cug agc acg cac uuc cga gag cag gau 1733
Pro His Ile Leu Gln Ile His Leu Ser Thr His Phe Arg Glu Gln Asp
                395                 400                 405

ggc auc cgu agc aag ccu gcc gcc gau guc aau gug ccc acg ugc ucc 1781
Gly Ile Arg Ser Lys Pro Ala Ala Asp Val Asn Val Pro Thr Cys Ser
            410                 415                 420

cug ugu ggg aag acu uuc ucc ugc aug uac acc cuc aag cgc cac gag     1829
Leu Cys Gly Lys Thr Phe Ser Cys Met Tyr Thr Leu Lys Arg His Glu
        425                 430                 435

agg acu cac ucg ggg gag aag ccc uac acg ugc acc caa ugc ggc aag     1877
Arg Thr His Ser Gly Glu Lys Pro Tyr Thr Cys Thr Gln Cys Gly Lys
    440                 445                 450

agu uuc cag uac ucg cac aac cug agc cgu cau gcc gug gug cac acc     1925
Ser Phe Gln Tyr Ser His Asn Leu Ser Arg His Ala Val Val His Thr
455                 460                 465                 470

cgc gag aag ccc cau gcc ugc aag ugg ugc gag cgc agg uuc acg cag     1973
Arg Glu Lys Pro His Ala Cys Lys Trp Cys Glu Arg Arg Phe Thr Gln
                475                 480                 485

ucc gga gac cug uac aga cac auc cgc aag uuc cac ugu gag uug gug     2021
Ser Gly Asp Leu Tyr Arg His Ile Arg Lys Phe His Cys Glu Leu Val
            490                 495                 500

aac ucc uug ucg guc aaa agu gaa gcg cug agc uug ccc acu guc aga     2069
Asn Ser Leu Ser Val Lys Ser Glu Ala Leu Ser Leu Pro Thr Val Arg
        505                 510                 515

gac ugg acc uua gaa gau agc ucu caa gaa cuu ugg aaa uaa             2111
Asp Trp Thr Leu Glu Asp Ser Ser Gln Glu Leu Trp Lys
    520                 525                 530

uuuuauauau auauauauau aaauaauaua uauauauaua uauacauaua uauacauaga     2171

ucucuauaua guugugguac ggucuaaaag cagucuuguu uccuggaacu gaaaaguugg     2231

gaucuuaacu uguuuuugca cuuuagaaua auagcaugag aaucucacua auuuagcauu     2291

cugauaaaag aaacuagagc aagucacaag uagagagcug ucuuucuuuu gaggguuagg     2351

ccaaguuagc caauaaaacc uucucaacaa augguucugu cacaaacgcu uucacauggc     2411

uuacuuuugu acacacugca uugucuugag cgcuuucccu gugcuccaca cuguuuuguu     2471

uuguuuuguu uucagaguag aaauccccuc caguuuauua gccucuuuau augucucaaa     2531

uugcaugaau auuuucuggc uguuggaaac cugaaugcuu uuagacccaa auggaaaauu     2591

ucugaaaugc uggauuaucu auuuuuaaac aagcaguuga cuuaaaacuu uguguggcaa     2651

cuucugguuu ucugaugguc cccagugaga gaaaucugac aguacacugg gucacuggga     2711

cugucucugu gaagguuugc uugagaugaa aaaggauauu gcagcuucag caguguugaa     2771

cuguguguuu aaaaugugaa uuacuguucu uguauacugu aauugauuac acgggcuggg     2831

ggggguguca aagaacuuga cagguugugu ugaugcucuu aguugagucu ugaaaaguaa     2891

auauuaacgc uacagaaaug caugaguuuc aauauauuuu uugucuuugu uugcauugua     2951

uaacuuuaac gagugaguuu aaaauuauuu aauuuccuua gaaaaauagc accauuuggg     3011

aaaaaaacug guguuacgaa gaacauaaau gcacugucuu uauuuuuauu uuuauuuuau     3071

auaauuuaaa uugacuuuuc ccacgucuuu aaguuugaaa cuguuaagcu gaauaaaaac     3131

uuaagcugca aauugauaac uucgcuacau uacaaggaac auauaaaugu uuacaaacgg 3191

cuuaaagaau ugcaugugca gugugcauuc auaccacacu uguaauugca cagaacccau 3251

gccagcucag aguuuaggug uacacacgua cccagccgag uguuuuuaga auaacuacug 3311

cacaaauuga caauagguca uaggucauuc ucucucuccc uccuuuuugc uuccccuuuc 3371

uuuuucuccc cuauuuccuu uccuuccccc uacccccuaa cucaugaaaa gauucuaugg 3431

acugaaaaag ccccaggcug aaaggacuga accgccuuga uugacauggg gagggggguu 3491

aguagacugu guaucggcag cagaggcugc agcccgaugu gugguucuga uggccagcgc 3551

ggggcagaag cguuuugcac acuguuuguu uuccugucuu gcacuuacaa auaaggucua 3611

ugggaguagc augggaaaca gugguuguuu uucccuuuuu cuuuuuuaau ugcuuuuguu 3671

uaaaauuuga ucgccuuaac uacuguaaac auagccuauu uuugugcuua agauacugaa 3731

uggaaaacuc cauugugugu ugcuggacug uuuuggaaau auuuggucaa auguauguua 3791

auuuggcugu aauggcauuu aaagcaaaca aacaaacaaa caaacaaagc ugugaaaaug 3851

gccuuggagc auuaucuuua guuacuugaa gaguuucuag uuuuuuuuuu uuuaauacag 3911

uuuauguuaa aauaaucuuu auuaaucuag aggagacaau caaugucugu ggaagaacgg 3971

acuuuuuugg auuuucuuuu uuaaugguca uugugaguga uugcucuuuc uuuuacuuag 4031

uuucacauuc uuccuuuguu cuaaaucuua gacugacauc uagcuuugac aaucauagua 4091

uguuuuauuu ccugaggggg aauaacuuau aaugcuguuu aguuuuguac uauuggugug 4151

uuggugaauu uuuaaacugu gugcuaacug caauaaauua uaugaacuga gaa 4204

<210> 26
<211> 3848
<212> RNA
<213> Mus musculus

<220>
<221> CDS
<222> (150)..(1745)

<400> 26
agugugugcg gaucuguggu ggagaaggua ucucauuccu cuccaacauc aucuccacuu 60

ugcaucuguc ucucuuaguc ugcuuuuuuu ccaccgaugu aacagaccug cagccagcaa 120

gacuccgagg gaaggacuua aucaaguuu aug ugu ccu aaa ggu uau gaa gac 173
Met Cys Pro Lys Gly Tyr Glu Asp
1               5

agu aug gag uuu cca gac cac agc aga cau uug cug cag ugu cug agc 221
Ser Met Glu Phe Pro Asp His Ser Arg His Leu Leu Gln Cys Leu Ser
    10                  15                  20

gag cag agg cac cag ggc uuu cuu ugu gac ugc acu guu cug gug gga 269
Glu Gln Arg His Gln Gly Phe Leu Cys Asp Cys Thr Val Leu Val Gly
25                  30                  35                  40

gau gcc cag uuc cgg gca cac cga gcu guu cug gcu uca ugc agc aug 317
Asp Ala Gln Phe Arg Ala His Arg Ala Val Leu Ala Ser Cys Ser Met
                45                  50                  55

uau uuc cac cuc uuu uac aag gac cag cug gac aaa aga gac auu guu 365
Tyr Phe His Leu Phe Tyr Lys Asp Gln Leu Asp Lys Arg Asp Ile Val
            60                  65                  70

cau cug aac agc gac auu gug aca gcc ccc gcu uuc gcu cuc cug cuu 413
His Leu Asn Ser Asp Ile Val Thr Ala Pro Ala Phe Ala Leu Leu Leu
        75                  80                  85

gaa uuc aug uac gaa ggg aaa cuc cag uuc aaa gac uug ccc auu gag 461
Glu Phe Met Tyr Glu Gly Lys Leu Gln Phe Lys Asp Leu Pro Ile Glu
    90                  95                  100

gac gug cua gca gcu gcc agu uau cuc cac aug uau gac auu guc aaa 509
Asp Val Leu Ala Ala Ala Ser Tyr Leu His Met Tyr Asp Ile Val Lys
105                 110                 115                 120

guc ugc aaa aag aag cug aaa gag aaa gcu acc aca gag gcg gac agc 557
Val Cys Lys Lys Lys Leu Lys Glu Lys Ala Thr Thr Glu Ala Asp Ser
                125                 130                 135

acc aaa aaa gaa gaa gau gcu uca agu ugu ucg gau aaa guc gag agc 605
Thr Lys Lys Glu Glu Asp Ala Ser Ser Cys Ser Asp Lys Val Glu Ser
            140                 145                 150

cuc uca gac ggc agc agc cac aug gca ggu gac cug ccc agu gau gaa 653
Leu Ser Asp Gly Ser Ser His Met Ala Gly Asp Leu Pro Ser Asp Glu
        155                 160                 165

gau gaa ggc gaa gau gac aaa uug aac auu cua ccc agc aaa agg gac 701
Asp Glu Gly Glu Asp Asp Lys Leu Asn Ile Leu Pro Ser Lys Arg Asp
    170                 175                 180

uug gca gcu gag ccu ggg aac aug ugg aug cga uug ccc uca gac uca 749
Leu Ala Ala Glu Pro Gly Asn Met Trp Met Arg Leu Pro Ser Asp Ser
185                 190                 195                 200

gca ggc auc ccc cag gcu ggc gga gag gcc gag cca cac gcc aca gca 797
Ala Gly Ile Pro Gln Ala Gly Gly Glu Ala Glu Pro His Ala Thr Ala
                205                 210                 215

gcu gga aaa aca gua gcc agc ccc ugc agc uca aca gag ucu uug ucc 845
Ala Gly Lys Thr Val Ala Ser Pro Cys Ser Ser Thr Glu Ser Leu Ser
            220                 225                 230

cag agg ucu guc acc ucc gug agg gau ucg gca gau guu gac ugu gug 893
Gln Arg Ser Val Thr Ser Val Arg Asp Ser Ala Asp Val Asp Cys Val
        235                 240                 245

cug gac cug ucu guc aag ucc agc cuu uca gga guu gaa aau cug aac 941
Leu Asp Leu Ser Val Lys Ser Ser Leu Ser Gly Val Glu Asn Leu Asn
    250                 255                 260

agc ucu uau uuc ucu uca cag gac gug cug aga agc aac cug gug cag 989
Ser Ser Tyr Phe Ser Ser Gln Asp Val Leu Arg Ser Asn Leu Val Gln
265                 270                 275                 280

gug aag gug gag aaa gag gcg ucc ugu gau gag agu gau guu ggc acu 1037
Val Lys Val Glu Lys Glu Ala Ser Cys Asp Glu Ser Asp Val Gly Thr

                285                 290                 295

aau gac uau gac aug gaa cau agc acu gug aaa gag agu gug agc acu 1085
Asn Asp Tyr Asp Met Glu His Ser Thr Val Lys Glu Ser Val Ser Thr
            300                 305                 310

aac aac agg gua cag uau gag cca gcc cac cug gcu ccu cug agg gag 1133
Asn Asn Arg Val Gln Tyr Glu Pro Ala His Leu Ala Pro Leu Arg Glu
        315                 320                 325

gac ucg guc uua agg gaa cug gau cgg gag gac aaa gcc agc gau gau 1181
Asp Ser Val Leu Arg Glu Leu Asp Arg Glu Asp Lys Ala Ser Asp Asp
    330                 335                 340

gag aug aug acc cca gag agc gag cgg guc cag gug gaa ggg ggc aug 1229
Glu Met Met Thr Pro Glu Ser Glu Arg Val Gln Val Glu Gly Gly Met
345                 350                 355                 360

gag aac agu cug cuu ccc uac guc ucc aau auc cug agc ccc gca ggc 1277
Glu Asn Ser Leu Leu Pro Tyr Val Ser Asn Ile Leu Ser Pro Ala Gly
                365                 370                 375

cag auc uuc aug ugc ccc cug ugc aac aaa guc uuc ccc agc ccc cac 1325
Gln Ile Phe Met Cys Pro Leu Cys Asn Lys Val Phe Pro Ser Pro His
            380                 385                 390

auc cug cag auc cac cug agc acg cac uuc cga gag cag gau ggc auc 1373
Ile Leu Gln Ile His Leu Ser Thr His Phe Arg Glu Gln Asp Gly Ile
        395                 400                 405

cgu agc aag ccu gcc gcc gau guc aau gug ccc acg ugc ucc cug ugu 1421
Arg Ser Lys Pro Ala Ala Asp Val Asn Val Pro Thr Cys Ser Leu Cys
    410                 415                 420

ggg aag acu uuc ucc ugc aug uac acc cuc aag cgc cac gag agg acu 1469
Gly Lys Thr Phe Ser Cys Met Tyr Thr Leu Lys Arg His Glu Arg Thr
425                 430                 435                 440

cac ucg ggg gag aag ccc uac acg ugc acc caa ugc ggc aag agu uuc 1517
His Ser Gly Glu Lys Pro Tyr Thr Cys Thr Gln Cys Gly Lys Ser Phe
                445                 450                 455

cag uac ucg cac aac cug agc cgu cau gcc gug gug cac acc cgc gag 1565
Gln Tyr Ser His Asn Leu Ser Arg His Ala Val Val His Thr Arg Glu
            460                 465                 470

aag ccc cau gcc ugc aag ugg ugc gag cgc agg uuc acg cag ucc gga 1613
Lys Pro His Ala Cys Lys Trp Cys Glu Arg Arg Phe Thr Gln Ser Gly
        475                 480                 485

gac cug uac aga cac auc cgc aag uuc cac ugu gag uug gug aac ucc 1661
Asp Leu Tyr Arg His Ile Arg Lys Phe His Cys Glu Leu Val Asn Ser
    490                 495                 500

uug ucg guc aaa agu gaa gcg cug agc uug ccc acu guc aga gac ugg 1709
Leu Ser Val Lys Ser Glu Ala Leu Ser Leu Pro Thr Val Arg Asp Trp
505                 510                 515                 520

acc uua gaa gau agc ucu caa gaa cuu ugg aaa uaa uuuuauauau 1755
Thr Leu Glu Asp Ser Ser Gln Glu Leu Trp Lys
                525                 530

auauauauau aaauaauaua uauauauaua uauacauaua uauacauaga ucucuauaua 1815

guugugguac ggucuaaaag cagucuuguu uccuggaacu gaaaaguugg gaucuuaacu 1875

uguuuuugca cuuuagaaua auagcaugag aaucucacua auuuagcauu cugauaaaag 1935

aaacuagagc aagucacaag uagagagcug ucuuucuuuu gaggguuagg ccaaguuagc 1995

caauaaaacc uucucaacaa augguucugu cacaaacgcu uucacauggc uuacuuuugu 2055

acacacugca uugucuugag cgcuuucccu gugcuccaca cuguuuuguu uuguuuuguu 2115

uucagaguag aaauccccuc caguuuauua gccucuuuau augucucaaa uugcaugaau 2175

auuuucuggc uguuggaaac cugaaugcuu uuagacccaa auggaaaauu ucugaaaugc 2235

uggauuaucu auuuuuaaac aagcaguuga cuuaaaacuu uguguggcaa cuucugguuu 2295

ucugaugguc cccagugaga gaaaucugac aguacacugg gucacuggga cugucucugu 2355

gaagguuugc uugagaugaa aaaggauauu gcagcuucag caguguugaa cuguguguuu 2415

aaaaugugaa uuacuguucu uguauacugu aauugauuac acgggcuggg ggggguguca 2475

aagaacuuga cagguugugu ugaugcucuu aguugagucu ugaaaaguaa auauuaacgc 2535

uacagaaaug caugaguuuc aauauauuuu uugucuuugu uugcauugua uaacuuuaac 2595

gagugaguuu aaaauuauuu aauuuccuua gaaaaauagc accauuuggg aaaaaaacug 2655

guguuacgaa gaacauaaau gcacugucuu uauuuuuauu uuuauuuuau auaauuuaaa 2715

uugacuuuuc ccacgucuuu aaguuugaaa cuguuaagcu gaauaaaaac uuaagcugca 2775

aauugauaac uucgcuacau uacaaggaac auauaaaugu uuacaaacgg cuuaaagaau 2835

ugcaugugca gugugcauuc auaccacacu uguaauugca cagaacccau gccagcucag 2895

aguuuaggug uacacacgua cccagccgag uguuuuuaga auaacuacug cacaaauuga 2955

caauagguca uaggucauuc ucucucuccc uccuuuuugc uuccccuuuc uuuuucuccc 3015

cuauuuccuu uccuuccccc uacccccuaa cucaugaaaa gauucuaugg acugaaaaag 3075

ccccaggcug aaaggacuga accgccuuga uugacauggg gagggggguu aguagacugu 3135

guaucggcag cagaggcugc agcccgaugu gugguucuga uggccagcgc ggggcagaag 3195

cguuuugcac acuguuuguu uuccugucuu gcacuuacaa auaaggucua ugggaguagc 3255

augggaaaca gugguuguuu uucccuuuuu cuuuuuuaau ugcuuuuguu uaaaauuuga 3315

ucgccuuaac uacuguaaac auagccuauu uuugugcuua agauacugaa uggaaaacuc 3375

cauugugugu ugcuggacug uuuuggaaau auuuggucaa auguauguua auuuggcugu 3435

aauggcauuu aaagcaaaca aacaaacaaa caaacaaagc ugugaaaaug gccuuggagc 3495

auuaucuuua guuacuugaa gaguuucuag uuuuuuuuuu uuuaauacag uuuauguuaa 3555

aauaaucuuu auuaaucuag aggagacaau caaugucugu ggaagaacgg acuuuuuugg 3615

auuuucuuuu uuaaugguca uugugaguga uugcucuuuc uuuuacuuag uuucacauuc 3675

uuccuuuguu cuaaaucuua gacugacauc uagcuuugac aaucauagua uguuuuauuu 3735

ccugaggggg aauaacuuau aaugcuguuu aguuuuguac uauuggugug uuggugaauu 3795

uuuaaacugu gugcuaacug caauaaauua uaugaacuga gaaaaaaaaa aaa 3848

<210> 27
<211> 4954
<212> RNA
<213> Mus musculus

<220>
<221> CDS
<222> (1283)..(2851)

<400> 27
ucagaaggua ucgggguuag gaugaaaguu ucacuguguc ucagcauagu guggaggaga 60

gaaguaguuu cugaauagua gcuucauauc cauccaagau gcacuugcag agguagaugu 120

uggcgagcuc ugccuuuggu cccaggaugg ggaagugggc uccgcugauc uuaagagagu 180

uuuagccacu acagaugaau uuugacugau cuuugccuuc ccuucuuuca cccucucccu 240

ugccaaaaaa caugggaaag augcauaauu ggaagaauag aagguacugg gaaaacaaaa 300

uagaucaggu uauuauugaa acaaaguauu uucuguugga uaccugaggc agagccagaa 360

aaaaaaguuu uauuuauuuu auuuuuucua ggaaggcuuu aaaaaauauu uaacuuacaa 420

aguuuuugca ccucauuuua cagugguaaa aguuugauua auuuuaauau gaaguuccuu 480

uugcccuaua gcagaucaaa guucagauga aaauguucag aguuaacaga cucucugguu 540

gcuaauguug uugagguaga ggaucuacaa agaaacagga cccccccccc auucauuuuu 600

aaaauguuug gauuuguuca uaguaaagau gaucuuuccc aucugucggu acaguucugc 660

uccucugugu guguaguacc ugcagaucuu accgcaccaa caucuguucu uauacacagg 720

gcuuuuguug uuguaacacu cugacuuuaa aaguaaguug uuugcugauu ucugcacuaa 780

uuaugcaauu ucauuuuuuu uccccuucau uucagccaaa uguguguguu cagucugugc 840

uuucuaagua guuggguauc agacguguua caaagaaugg cuggacaugu cuucuugacu 900

cuuccagaau uuguguggga cuuugccuga uauuaugagu ucuuucccuu ugaaguuaaa 960

gcucucuuuu agguuuugua ggaugucauu ugaugaguug uugguuaaau uucuggaaga 1020

ggucugaguu cuuguucuaa gucauaagcu aaaggaagcu uuaaacuuga ccaggaguua 1080

ugaguggggu gagcaaagcc uuggugauug cauuuagguu gaaugugugg cuugggaguc 1140

cuuggggugu gagccugauu uaaaauucag acauguaacu cuccaccacu aaggcggaua 1200

uuuuuuaaag uggcgacuag agucuaaauu ccugacuagg cucuaacgag aaacuccucu 1260

uuccccaggu uaugaagaca gu aug gag uuu cca gac cac agc aga cau uug 1312
Met Glu Phe Pro Asp His Ser Arg His Leu
1               5                   10

cug cag ugu cug agc gag cag agg cac cag ggc uuu cuu ugu gac ugc 1360
Leu Gln Cys Leu Ser Glu Gln Arg His Gln Gly Phe Leu Cys Asp Cys
                15                  20                  25

acu guu cug gug gga gau gcc cag uuc cgg gca cac cga gcu guu cug 1408
Thr Val Leu Val Gly Asp Ala Gln Phe Arg Ala His Arg Ala Val Leu
            30                  35                  40

gcu uca ugc agc aug uau uuc cac cuc uuu uac aag gac cag cug gac 1456
Ala Ser Cys Ser Met Tyr Phe His Leu Phe Tyr Lys Asp Gln Leu Asp
        45                  50                  55

aaa aga gac auu guu cau cug aac agc gac auu gug aca gcc ccc gcu 1504
Lys Arg Asp Ile Val His Leu Asn Ser Asp Ile Val Thr Ala Pro Ala
    60                  65                  70

uuc gcu cuc cug cuu gaa uuc aug uac gaa ggg aaa cuc cag uuc aaa 1552
Phe Ala Leu Leu Leu Glu Phe Met Tyr Glu Gly Lys Leu Gln Phe Lys
75                  80                  85                  90

gac uug ccc auu gag gac gug cua gca gcu gcc agu uau cuc cac aug 1600
Asp Leu Pro Ile Glu Asp Val Leu Ala Ala Ala Ser Tyr Leu His Met
                95                  100                 105

uau gac auu guc aaa guc ugc aaa aag aag cug aaa gag aaa gcu acc 1648
Tyr Asp Ile Val Lys Val Cys Lys Lys Lys Leu Lys Glu Lys Ala Thr
            110                 115                 120

aca gag gcg gac agc acc aaa aaa gaa gaa gau gcu uca agu ugu ucg 1696
Thr Glu Ala Asp Ser Thr Lys Lys Glu Glu Asp Ala Ser Ser Cys Ser
        125                 130                 135

gau aaa guc gag agc cuc uca gac ggc agc agc cac aug gca ggu gac 1744
Asp Lys Val Glu Ser Leu Ser Asp Gly Ser Ser His Met Ala Gly Asp
    140                 145                 150

cug ccc agu gau gaa gau gaa ggc gaa gau gac aaa uug aac auu cua 1792
Leu Pro Ser Asp Glu Asp Glu Gly Glu Asp Asp Lys Leu Asn Ile Leu
155                 160                 165                 170

ccc agc aaa agg gac uug gca gcu gag ccu ggg aac aug ugg aug cga 1840
Pro Ser Lys Arg Asp Leu Ala Ala Glu Pro Gly Asn Met Trp Met Arg
                175                 180                 185

uug ccc uca gac uca gca ggc auc ccc cag gcu ggc gga gag gcc gag 1888
Leu Pro Ser Asp Ser Ala Gly Ile Pro Gln Ala Gly Gly Glu Ala Glu
            190                 195                 200

cca cac gcc aca gca gcu gga aaa aca gua gcc agc ccc ugc agc uca 1936
Pro His Ala Thr Ala Ala Gly Lys Thr Val Ala Ser Pro Cys Ser Ser
        205                 210                 215

aca gag ucu uug ucc cag agg ucu guc acc ucc gug agg gau ucg gca 1984
Thr Glu Ser Leu Ser Gln Arg Ser Val Thr Ser Val Arg Asp Ser Ala
    220                 225                 230

gau guu gac ugu gug cug gac cug ucu guc aag ucc agc cuu uca gga 2032
Asp Val Asp Cys Val Leu Asp Leu Ser Val Lys Ser Ser Leu Ser Gly
235                 240                 245                 250

guu gaa aau cug aac agc ucu uau uuc ucu uca cag gac gug cug aga 2080
Val Glu Asn Leu Asn Ser Ser Tyr Phe Ser Ser Gln Asp Val Leu Arg

255                 260                 265

agc aac cug gug cag gug aag gug gag aaa gag gcg ucc ugu gau gag      2128
Ser Asn Leu Val Gln Val Lys Val Glu Lys Glu Ala Ser Cys Asp Glu
            270                 275                 280

agu gau guu ggc acu aau gac uau gac aug gaa cau agc acu gug aaa      2176
Ser Asp Val Gly Thr Asn Asp Tyr Asp Met Glu His Ser Thr Val Lys
        285                 290                 295

gag agu gug agc acu aac aac agg gua cag uau gag cca gcc cac cug      2224
Glu Ser Val Ser Thr Asn Asn Arg Val Gln Tyr Glu Pro Ala His Leu
    300                 305                 310

gcu ccu cug agg gag gac ucg guc uua agg gaa cug gau cgg gag gac      2272
Ala Pro Leu Arg Glu Asp Ser Val Leu Arg Glu Leu Asp Arg Glu Asp
315                 320                 325                 330

aaa gcc agc gau gau gag aug aug acc cca gag agc gag cgg guc cag      2320
Lys Ala Ser Asp Asp Glu Met Met Thr Pro Glu Ser Glu Arg Val Gln
                335                 340                 345

gug gaa ggg ggc aug gag aac agu cug cuu ccc uac guc ucc aau auc      2368
Val Glu Gly Gly Met Glu Asn Ser Leu Leu Pro Tyr Val Ser Asn Ile
            350                 355                 360

cug agc ccc gca ggc cag auc uuc aug ugc ccc cug ugc aac aaa guc      2416
Leu Ser Pro Ala Gly Gln Ile Phe Met Cys Pro Leu Cys Asn Lys Val
        365                 370                 375

uuc ccc agc ccc cac auc cug cag auc cac cug agc acg cac uuc cga      2464
Phe Pro Ser Pro His Ile Leu Gln Ile His Leu Ser Thr His Phe Arg
    380                 385                 390

gag cag gau ggc auc cgu agc aag ccu gcc gcc gau guc aau gug ccc      2512
Glu Gln Asp Gly Ile Arg Ser Lys Pro Ala Ala Asp Val Asn Val Pro
395                 400                 405                 410

acg ugc ucc cug ugu ggg aag acu uuc ucc ugc aug uac acc cuc aag      2560
Thr Cys Ser Leu Cys Gly Lys Thr Phe Ser Cys Met Tyr Thr Leu Lys
                415                 420                 425

cgc cac gag agg acu cac ucg ggg gag aag ccc uac acg ugc acc caa      2608
Arg His Glu Arg Thr His Ser Gly Glu Lys Pro Tyr Thr Cys Thr Gln
            430                 435                 440

ugc ggc aag agu uuc cag uac ucg cac aac cug agc cgu cau gcc gug      2656
Cys Gly Lys Ser Phe Gln Tyr Ser His Asn Leu Ser Arg His Ala Val
        445                 450                 455

gug cac acc cgc gag aag ccc cau gcc ugc aag ugg ugc gag cgc agg      2704
Val His Thr Arg Glu Lys Pro His Ala Cys Lys Trp Cys Glu Arg Arg
    460                 465                 470

uuc acg cag ucc gga gac cug uac aga cac auc cgc aag uuc cac ugu      2752
Phe Thr Gln Ser Gly Asp Leu Tyr Arg His Ile Arg Lys Phe His Cys
475                 480                 485                 490

gag uug gug aac ucc uug ucg guc aaa agu gaa gcg cug agc uug ccc      2800
Glu Leu Val Asn Ser Leu Ser Val Lys Ser Glu Ala Leu Ser Leu Pro
                495                 500                 505

acu guc aga gac ugg acc uua gaa gau agc ucu caa gaa cuu ugg aaa      2848

Thr Val Arg Asp Trp Thr Leu Glu Asp Ser Ser Gln Glu Leu Trp Lys
            510                 515                 520

uaa uuuuauauau auauauauau aaauaauaua uauauauaua uauacauaua      2901

uauacauaga ucucuauaua guugugguac ggucuaaaag cagucuuguu uccuggaacu      2961

gaaaaguugg gaucuuaacu uguuuuugca cuuuagaaua auagcaugag aaucucacua      3021

auuuagcauu cugauaaaag aaacuagagc aagucacaag uagagagcug ucuuucuuuu      3081

gaggguuagg ccaaguuagc caauaaaacc uucucaacaa augguucugu cacaaacgcu      3141

uucacauggc uuacuuuugu acacacugca uugucuugag cgcuuucccu gugcuccaca      3201

cuguuuuguu uuguuuuguu uucagaguag aaauccccuc caguuuauua gccucuuuau      3261

augucucaaa uugcaugaau auuuucuggc uguuggaaac cugaaugcuu uuagacccaa      3321

auggaaaauu ucugaaaugc uggauuaucu auuuuuaaac aagcaguuga cuuaaaacuu      3381

uguguggcaa cuucugguuu ucugaugguc cccagugaga gaaaucugac aguacacugg      3441

gucacuggga cugucucugu gaagguuugc uugagaugaa aaaggauauu gcagcuucag      3501

caguguugaa cuguguguuu aaaaugugaa uuacuguucu uguauacugu aauugauuac      3561

acgggcuggg ggggguguca aagaacuuga cagguugugu ugaugcucuu aguugagucu      3621

ugaaaaguaa auauuaacgc uacagaaaug caugaguuuc aauauauuuu uugucuuugu      3681

uugcauugua uaacuuuaac gagugaguuu aaaauuauuu aauuuccuua gaaaaauagc      3741

accauuuggg aaaaaaacug guguuacgaa gaacauaaau gcacugucuu uauuuuuauu      3801

uuuauuuuau auaauuuaaa uugacuuuuc ccacgucuuu aaguuugaaa cuguuaagcu      3861

gaauaaaaac uuaagcugca aauugauaac uucgcuacau uacaaggaac auauaaaugu      3921

uuacaaacgg cuuaaagaau ugcaugugca gugugcauuc auaccacacu uguaauugca      3981

cagaacccau gccagcucag aguuuaggug uacacacgua cccagccgag uguuuuuaga      4041

auaacuacug cacaaauuga caauagguca uaggucauuc ucucucuccc uccuuuuugc      4101

uuccccuuuc uuuuucuccc cuauuuccuu uccuuccccc uacccccuaa cucaugaaaa      4161

gauucuaugg acugaaaaag ccccaggcug aaaggacuga accgccuuga uugacauggg      4221

gagggggguu aguagacugu guaucggcag cagaggcugc agcccgaugu gugguucuga      4281

uggccagcgc ggggcagaag cguuuugcac acuguuuguu uuccugucuu gcacuuacaa      4341

auaaggucua ugggaguagc augggaaaca gugguuguuu uucccuuuuu cuuuuuuaau      4401

ugcuuuuguu uaaaauuuga ucgccuuaac uacuguaaac auagccuauu uuugugcuua      4461

agauacugaa uggaaaacuc cauugugugu ugcuggacug uuuuggaaau auuuggucaa      4521

auguauguua auuuggcugu aauggcauuu aaagcaaaca aacaaacaaa caaacaaagc      4581

ugugaaaaug gccuuggagc auuaucuuua guuacuugaa gaguuucuag uuuuuuuuuu      4641

uuuaauacag uuuauguuaa aauaaucuuu auuaaucuag aggagacaau caaugucugu 4701

ggaagaacgg acuuuuuugg auuuucuuuu uuaaugguca uugugaguga uugcucuuuc 4761

uuuuacuuag uuucacauuc uuccuuuguu cuaaaucuua gacugacauc uagcuuugac 4821

aaucauagua uguuuuauuu ccugaggggg aauaacuuau aaugcuguuu aguuuuguac 4881

uauuggugug uuggugaauu uuuaaacugu gugcuaacug caauaaauua uaugaacuga 4941

gaaaaaaaaa aaa 4954

<210> 28
<211> 1596
<212> DNA
<213> Mus musculus

<220>
<221> CDS
<222> (1)..(1596)

<400> 28
atg tgt cct aaa ggt tat gaa gac agt atg gag ttt cca gac cac agc 48
Met Cys Pro Lys Gly Tyr Glu Asp Ser Met Glu Phe Pro Asp His Ser
1               5                   10                  15

aga cat ttg ctg cag tgt ctg agc gag cag agg cac cag ggc ttt ctt 96
Arg His Leu Leu Gln Cys Leu Ser Glu Gln Arg His Gln Gly Phe Leu
            20                  25                  30

tgt gac tgc act gtt ctg gtg gga gat gcc cag ttc cgg gca cac cga 144
Cys Asp Cys Thr Val Leu Val Gly Asp Ala Gln Phe Arg Ala His Arg
        35                  40                  45

gct gtt ctg gct tca tgc agc atg tat ttc cac ctc ttt tac aag gac 192
Ala Val Leu Ala Ser Cys Ser Met Tyr Phe His Leu Phe Tyr Lys Asp
    50                  55                  60

cag ctg gac aaa aga gac att gtt cat ctg aac agc gac att gtg aca 240
Gln Leu Asp Lys Arg Asp Ile Val His Leu Asn Ser Asp Ile Val Thr
65                  70                  75                  80

gcc ccc gct ttc gct ctc ctg ctt gaa ttc atg tac gaa ggg aaa ctc 288
Ala Pro Ala Phe Ala Leu Leu Leu Glu Phe Met Tyr Glu Gly Lys Leu
                85                  90                  95

cag ttc aaa gac ttg ccc att gag gac gtg cta gca gct gcc agt tat 336
Gln Phe Lys Asp Leu Pro Ile Glu Asp Val Leu Ala Ala Ala Ser Tyr
            100                 105                 110

ctc cac atg tat gac att gtc aaa gtc tgc aaa aag aag ctg aaa gag 384
Leu His Met Tyr Asp Ile Val Lys Val Cys Lys Lys Lys Leu Lys Glu
        115                 120                 125

aaa gct acc aca gag gcg gac agc acc aaa aaa gaa gaa gat gct tca 432
Lys Ala Thr Thr Glu Ala Asp Ser Thr Lys Lys Glu Glu Asp Ala Ser
    130                 135                 140

agt tgt tcg gat aaa gtc gag agc ctc tca gac ggc agc agc cac atg 480
Ser Cys Ser Asp Lys Val Glu Ser Leu Ser Asp Gly Ser Ser His Met
145                 150                 155                 160

gca ggt gac ctg ccc agt gat gaa gat gaa ggc gaa gat gac aaa ttg 528
Ala Gly Asp Leu Pro Ser Asp Glu Asp Glu Gly Glu Asp Asp Lys Leu
                165                 170                 175

aac att cta ccc agc aaa agg gac ttg gca gct gag cct ggg aac atg 576
Asn Ile Leu Pro Ser Lys Arg Asp Leu Ala Ala Glu Pro Gly Asn Met
            180                 185                 190

tgg atg cga ttg ccc tca gac tca gca ggc atc ccc cag gct ggc gga 624
Trp Met Arg Leu Pro Ser Asp Ser Ala Gly Ile Pro Gln Ala Gly Gly
        195                 200                 205

gag gcc gag cca cac gcc aca gca gct gga aaa aca gta gcc agc ccc 672
Glu Ala Glu Pro His Ala Thr Ala Ala Gly Lys Thr Val Ala Ser Pro
    210                 215                 220

tgc agc tca aca gag tct ttg tcc cag agg tct gtc acc tcc gtg agg 720
Cys Ser Ser Thr Glu Ser Leu Ser Gln Arg Ser Val Thr Ser Val Arg
225                 230                 235                 240

gat tcg gca gat gtt gac tgt gtg ctg gac ctg tct gtc aag tcc agc 768
Asp Ser Ala Asp Val Asp Cys Val Leu Asp Leu Ser Val Lys Ser Ser
                245                 250                 255

ctt tca gga gtt gaa aat ctg aac agc tct tat ttc tct tca cag gac 816
Leu Ser Gly Val Glu Asn Leu Asn Ser Ser Tyr Phe Ser Ser Gln Asp
            260                 265                 270

gtg ctg aga agc aac ctg gtg cag gtg aag gtg gag aaa gag gcg tcc 864
Val Leu Arg Ser Asn Leu Val Gln Val Lys Val Glu Lys Glu Ala Ser
        275                 280                 285

tgt gat gag agt gat gtt ggc act aat gac tat gac atg gaa cat agc 912
Cys Asp Glu Ser Asp Val Gly Thr Asn Asp Tyr Asp Met Glu His Ser
    290                 295                 300

act gtg aaa gag agt gtg agc act aac aac agg gta cag tat gag cca 960
Thr Val Lys Glu Ser Val Ser Thr Asn Asn Arg Val Gln Tyr Glu Pro
305                 310                 315                 320

gcc cac ctg gct cct ctg agg gag gac tcg gtc tta agg gaa ctg gat 1008
Ala His Leu Ala Pro Leu Arg Glu Asp Ser Val Leu Arg Glu Leu Asp
                325                 330                 335

cgg gag gac aaa gcc agc gat gat gag atg atg acc cca gag agc gag 1056
Arg Glu Asp Lys Ala Ser Asp Asp Glu Met Met Thr Pro Glu Ser Glu
            340                 345                 350

cgg gtc cag gtg gaa ggg ggc atg gag aac agt ctg ctt ccc tac gtc 1104
Arg Val Gln Val Glu Gly Gly Met Glu Asn Ser Leu Leu Pro Tyr Val
        355                 360                 365

tcc aat atc ctg agc ccc gca ggc cag atc ttc atg tgc ccc ctg tgc 1152
Ser Asn Ile Leu Ser Pro Ala Gly Gln Ile Phe Met Cys Pro Leu Cys
    370                 375                 380

aac aaa gtc ttc ccc agc ccc cac atc ctg cag atc cac ctg agc acg 1200
Asn Lys Val Phe Pro Ser Pro His Ile Leu Gln Ile His Leu Ser Thr
385                 390                 395                 400

cac ttc cga gag cag gat ggc atc cgt agc aag cct gcc gcc gat gtc 1248
His Phe Arg Glu Gln Asp Gly Ile Arg Ser Lys Pro Ala Ala Asp Val

405                 410                 415

aat gtg ccc acg tgc tcc ctg tgt ggg aag act ttc tcc tgc atg tac        1296
Asn Val Pro Thr Cys Ser Leu Cys Gly Lys Thr Phe Ser Cys Met Tyr
            420                 425                 430

acc ctc aag cgc cac gag agg act cac tcg ggg gag aag ccc tac acg        1344
Thr Leu Lys Arg His Glu Arg Thr His Ser Gly Glu Lys Pro Tyr Thr
        435                 440                 445

tgc acc caa tgc ggc aag agt ttc cag tac tcg cac aac ctg agc cgt        1392
Cys Thr Gln Cys Gly Lys Ser Phe Gln Tyr Ser His Asn Leu Ser Arg
    450                 455                 460

cat gcc gtg gtg cac acc cgc gag aag ccc cat gcc tgc aag tgg tgc        1440
His Ala Val Val His Thr Arg Glu Lys Pro His Ala Cys Lys Trp Cys
465                 470                 475                 480

gag cgc agg ttc acg cag tcc gga gac ctg tac aga cac atc cgc aag        1488
Glu Arg Arg Phe Thr Gln Ser Gly Asp Leu Tyr Arg His Ile Arg Lys
                485                 490                 495

ttc cac tgt gag ttg gtg aac tcc ttg tcg gtc aaa agt gaa gcg ctg        1536
Phe His Cys Glu Leu Val Asn Ser Leu Ser Val Lys Ser Glu Ala Leu
            500                 505                 510

agc ttg ccc act gtc aga gac tgg acc tta gaa gat agc tct caa gaa        1584
Ser Leu Pro Thr Val Arg Asp Trp Thr Leu Glu Asp Ser Ser Gln Glu
        515                 520                 525

ctt tgg aaa taa                                                        1596
Leu Trp Lys
    530


<210>  29
<211>  531
<212>  PRT
<213>  Mus musculus

<400>  29

Met Cys Pro Lys Gly Tyr Glu Asp Ser Met Glu Phe Pro Asp His Ser
1               5                   10                  15


Arg His Leu Leu Gln Cys Leu Ser Glu Gln Arg His Gln Gly Phe Leu
            20                  25                  30


Cys Asp Cys Thr Val Leu Val Gly Asp Ala Gln Phe Arg Ala His Arg
        35                  40                  45


Ala Val Leu Ala Ser Cys Ser Met Tyr Phe His Leu Phe Tyr Lys Asp
    50                  55                  60


Gln Leu Asp Lys Arg Asp Ile Val His Leu Asn Ser Asp Ile Val Thr
65                  70                  75                  80


Ala Pro Ala Phe Ala Leu Leu Leu Glu Phe Met Tyr Glu Gly Lys Leu

85                  90                  95

Gln Phe Lys Asp Leu Pro Ile Glu Asp Val Leu Ala Ala Ala Ser Tyr
            100                 105                 110

Leu His Met Tyr Asp Ile Val Lys Val Cys Lys Lys Lys Leu Lys Glu
        115                 120                 125

Lys Ala Thr Thr Glu Ala Asp Ser Thr Lys Lys Glu Glu Asp Ala Ser
    130                 135                 140

Ser Cys Ser Asp Lys Val Glu Ser Leu Ser Asp Gly Ser Ser His Met
145                 150                 155                 160

Ala Gly Asp Leu Pro Ser Asp Glu Asp Glu Gly Glu Asp Asp Lys Leu
                165                 170                 175

Asn Ile Leu Pro Ser Lys Arg Asp Leu Ala Ala Glu Pro Gly Asn Met
            180                 185                 190

Trp Met Arg Leu Pro Ser Asp Ser Ala Gly Ile Pro Gln Ala Gly Gly
        195                 200                 205

Glu Ala Glu Pro His Ala Thr Ala Ala Gly Lys Thr Val Ala Ser Pro
    210                 215                 220

Cys Ser Ser Thr Glu Ser Leu Ser Gln Arg Ser Val Thr Ser Val Arg
225                 230                 235                 240

Asp Ser Ala Asp Val Asp Cys Val Leu Asp Leu Ser Val Lys Ser Ser
                245                 250                 255

Leu Ser Gly Val Glu Asn Leu Asn Ser Ser Tyr Phe Ser Ser Gln Asp
            260                 265                 270

Val Leu Arg Ser Asn Leu Val Gln Val Lys Val Glu Lys Glu Ala Ser
        275                 280                 285

Cys Asp Glu Ser Asp Val Gly Thr Asn Asp Tyr Asp Met Glu His Ser
    290                 295                 300

Thr Val Lys Glu Ser Val Ser Thr Asn Asn Arg Val Gln Tyr Glu Pro
305                 310                 315                 320

Ala His Leu Ala Pro Leu Arg Glu Asp Ser Val Leu Arg Glu Leu Asp
                325                 330                 335

Arg Glu Asp Lys Ala Ser Asp Asp Glu Met Met Thr Pro Glu Ser Glu
            340                 345                 350

Arg Val Gln Val Glu Gly Gly Met Glu Asn Ser Leu Leu Pro Tyr Val
        355                 360                 365

Ser Asn Ile Leu Ser Pro Ala Gly Gln Ile Phe Met Cys Pro Leu Cys
    370                 375                 380

Asn Lys Val Phe Pro Ser Pro His Ile Leu Gln Ile His Leu Ser Thr
385                 390                 395                 400

His Phe Arg Glu Gln Asp Gly Ile Arg Ser Lys Pro Ala Ala Asp Val
                405                 410                 415

Asn Val Pro Thr Cys Ser Leu Cys Gly Lys Thr Phe Ser Cys Met Tyr
            420                 425                 430

Thr Leu Lys Arg His Glu Arg Thr His Ser Gly Glu Lys Pro Tyr Thr
        435                 440                 445

Cys Thr Gln Cys Gly Lys Ser Phe Gln Tyr Ser His Asn Leu Ser Arg
    450                 455                 460

His Ala Val Val His Thr Arg Glu Lys Pro His Ala Cys Lys Trp Cys
465                 470                 475                 480

Glu Arg Arg Phe Thr Gln Ser Gly Asp Leu Tyr Arg His Ile Arg Lys
                485                 490                 495

Phe His Cys Glu Leu Val Asn Ser Leu Ser Val Lys Ser Glu Ala Leu
            500                 505                 510

Ser Leu Pro Thr Val Arg Asp Trp Thr Leu Glu Asp Ser Ser Gln Glu
        515                 520                 525

Leu Trp Lys
    530

<210>  30
<211>  1569
<212>  DNA
<213>  Mus musculus

<220>
<221>  CDS
<222>  (1)..(1569)

<400>  30

atg gag ttt cca gac cac agc aga cat ttg ctg cag tgt ctg agc gag 48
Met Glu Phe Pro Asp His Ser Arg His Leu Leu Gln Cys Leu Ser Glu
1               5                   10                  15

cag agg cac cag ggc ttt ctt tgt gac tgc act gtt ctg gtg gga gat 96
Gln Arg His Gln Gly Phe Leu Cys Asp Cys Thr Val Leu Val Gly Asp
            20                  25                  30

gcc cag ttc cgg gca cac cga gct gtt ctg gct tca tgc agc atg tat 144
Ala Gln Phe Arg Ala His Arg Ala Val Leu Ala Ser Cys Ser Met Tyr
        35                  40                  45

ttc cac ctc ttt tac aag gac cag ctg gac aaa aga gac att gtt cat 192
Phe His Leu Phe Tyr Lys Asp Gln Leu Asp Lys Arg Asp Ile Val His
    50                  55                  60

ctg aac agc gac att gtg aca gcc ccc gct ttc gct ctc ctg ctt gaa 240
Leu Asn Ser Asp Ile Val Thr Ala Pro Ala Phe Ala Leu Leu Leu Glu
65                  70                  75                  80

ttc atg tac gaa ggg aaa ctc cag ttc aaa gac ttg ccc att gag gac 288
Phe Met Tyr Glu Gly Lys Leu Gln Phe Lys Asp Leu Pro Ile Glu Asp
                85                  90                  95

gtg cta gca gct gcc agt tat ctc cac atg tat gac att gtc aaa gtc 336
Val Leu Ala Ala Ala Ser Tyr Leu His Met Tyr Asp Ile Val Lys Val
            100                 105                 110

tgc aaa aag aag ctg aaa gag aaa gct acc aca gag gcg gac agc acc 384
Cys Lys Lys Lys Leu Lys Glu Lys Ala Thr Thr Glu Ala Asp Ser Thr
        115                 120                 125

aaa aaa gaa gaa gat gct tca agt tgt tcg gat aaa gtc gag agc ctc 432
Lys Lys Glu Glu Asp Ala Ser Ser Cys Ser Asp Lys Val Glu Ser Leu
    130                 135                 140

tca gac ggc agc agc cac atg gca ggt gac ctg ccc agt gat gaa gat 480
Ser Asp Gly Ser Ser His Met Ala Gly Asp Leu Pro Ser Asp Glu Asp
145                 150                 155                 160

gaa ggc gaa gat gac aaa ttg aac att cta ccc agc aaa agg gac ttg 528
Glu Gly Glu Asp Asp Lys Leu Asn Ile Leu Pro Ser Lys Arg Asp Leu
                165                 170                 175

gca gct gag cct ggg aac atg tgg atg cga ttg ccc tca gac tca gca 576
Ala Ala Glu Pro Gly Asn Met Trp Met Arg Leu Pro Ser Asp Ser Ala
            180                 185                 190

ggc atc ccc cag gct ggc gga gag gcc gag cca cac gcc aca gca gct 624
Gly Ile Pro Gln Ala Gly Gly Glu Ala Glu Pro His Ala Thr Ala Ala
        195                 200                 205

gga aaa aca gta gcc agc ccc tgc agc tca aca gag tct ttg tcc cag 672
Gly Lys Thr Val Ala Ser Pro Cys Ser Ser Thr Glu Ser Leu Ser Gln
    210                 215                 220

agg tct gtc acc tcc gtg agg gat tcg gca gat gtt gac tgt gtg ctg 720
Arg Ser Val Thr Ser Val Arg Asp Ser Ala Asp Val Asp Cys Val Leu
225                 230                 235                 240

gac ctg tct gtc aag tcc agc ctt tca gga gtt gaa aat ctg aac agc 768
Asp Leu Ser Val Lys Ser Ser Leu Ser Gly Val Glu Asn Leu Asn Ser
                245                 250                 255

tct tat ttc tct tca cag gac gtg ctg aga agc aac ctg gtg cag gtg 816
Ser Tyr Phe Ser Ser Gln Asp Val Leu Arg Ser Asn Leu Val Gln Val
            260                 265                 270

aag gtg gag aaa gag gcg tcc tgt gat gag agt gat gtt ggc act aat 864
Lys Val Glu Lys Glu Ala Ser Cys Asp Glu Ser Asp Val Gly Thr Asn
        275                 280                 285

gac tat gac atg gaa cat agc act gtg aaa gag agt gtg agc act aac 912
Asp Tyr Asp Met Glu His Ser Thr Val Lys Glu Ser Val Ser Thr Asn
    290                 295                 300

aac agg gta cag tat gag cca gcc cac ctg gct cct ctg agg gag gac 960
Asn Arg Val Gln Tyr Glu Pro Ala His Leu Ala Pro Leu Arg Glu Asp
305                 310                 315                 320

tcg gtc tta agg gaa ctg gat cgg gag gac aaa gcc agc gat gat gag 1008
Ser Val Leu Arg Glu Leu Asp Arg Glu Asp Lys Ala Ser Asp Asp Glu
                325                 330                 335

atg atg acc cca gag agc gag cgg gtc cag gtg gaa ggg ggc atg gag 1056
Met Met Thr Pro Glu Ser Glu Arg Val Gln Val Glu Gly Gly Met Glu
            340                 345                 350

aac agt ctg ctt ccc tac gtc tcc aat atc ctg agc ccc gca ggc cag 1104
Asn Ser Leu Leu Pro Tyr Val Ser Asn Ile Leu Ser Pro Ala Gly Gln
        355                 360                 365

atc ttc atg tgc ccc ctg tgc aac aaa gtc ttc ccc agc ccc cac atc 1152
Ile Phe Met Cys Pro Leu Cys Asn Lys Val Phe Pro Ser Pro His Ile
    370                 375                 380

ctg cag atc cac ctg agc acg cac ttc cga gag cag gat ggc atc cgt 1200
Leu Gln Ile His Leu Ser Thr His Phe Arg Glu Gln Asp Gly Ile Arg
385                 390                 395                 400

agc aag cct gcc gcc gat gtc aat gtg ccc acg tgc tcc ctg tgt ggg 1248
Ser Lys Pro Ala Ala Asp Val Asn Val Pro Thr Cys Ser Leu Cys Gly
                405                 410                 415

aag act ttc tcc tgc atg tac acc ctc aag cgc cac gag agg act cac 1296
Lys Thr Phe Ser Cys Met Tyr Thr Leu Lys Arg His Glu Arg Thr His
            420                 425                 430

tcg ggg gag aag ccc tac acg tgc acc caa tgc ggc aag agt ttc cag 1344
Ser Gly Glu Lys Pro Tyr Thr Cys Thr Gln Cys Gly Lys Ser Phe Gln
        435                 440                 445

tac tcg cac aac ctg agc cgt cat gcc gtg gtg cac acc cgc gag aag 1392
Tyr Ser His Asn Leu Ser Arg His Ala Val Val His Thr Arg Glu Lys
    450                 455                 460

ccc cat gcc tgc aag tgg tgc gag cgc agg ttc acg cag tcc gga gac 1440
Pro His Ala Cys Lys Trp Cys Glu Arg Arg Phe Thr Gln Ser Gly Asp
465                 470                 475                 480

ctg tac aga cac atc cgc aag ttc cac tgt gag ttg gtg aac tcc ttg 1488
Leu Tyr Arg His Ile Arg Lys Phe His Cys Glu Leu Val Asn Ser Leu
                485                 490                 495

tcg gtc aaa agt gaa gcg ctg agc ttg ccc act gtc aga gac tgg acc 1536
Ser Val Lys Ser Glu Ala Leu Ser Leu Pro Thr Val Arg Asp Trp Thr

500                 505                 510

tta gaa gat agc tct caa gaa ctt tgg aaa taa                         1569
Leu Glu Asp Ser Ser Gln Glu Leu Trp Lys
        515                 520

<210>  31
<211>  522
<212>  PRT
<213>  Mus musculus

<400>  31

Met Glu Phe Pro Asp His Ser Arg His Leu Leu Gln Cys Leu Ser Glu
1               5                   10                  15

Gln Arg His Gln Gly Phe Leu Cys Asp Cys Thr Val Leu Val Gly Asp
            20                  25                  30

Ala Gln Phe Arg Ala His Arg Ala Val Leu Ala Ser Cys Ser Met Tyr
        35                  40                  45

Phe His Leu Phe Tyr Lys Asp Gln Leu Asp Lys Arg Asp Ile Val His
    50                  55                  60

Leu Asn Ser Asp Ile Val Thr Ala Pro Ala Phe Ala Leu Leu Leu Glu
65                  70                  75                  80

Phe Met Tyr Glu Gly Lys Leu Gln Phe Lys Asp Leu Pro Ile Glu Asp
                85                  90                  95

Val Leu Ala Ala Ala Ser Tyr Leu His Met Tyr Asp Ile Val Lys Val
            100                 105                 110

Cys Lys Lys Lys Leu Lys Glu Lys Ala Thr Thr Glu Ala Asp Ser Thr
        115                 120                 125

Lys Lys Glu Glu Asp Ala Ser Ser Cys Ser Asp Lys Val Glu Ser Leu
    130                 135                 140

Ser Asp Gly Ser Ser His Met Ala Gly Asp Leu Pro Ser Asp Glu Asp
145                 150                 155                 160

Glu Gly Glu Asp Asp Lys Leu Asn Ile Leu Pro Ser Lys Arg Asp Leu
                165                 170                 175

Ala Ala Glu Pro Gly Asn Met Trp Met Arg Leu Pro Ser Asp Ser Ala
            180                 185                 190

Gly Ile Pro Gln Ala Gly Gly Glu Ala Glu Pro His Ala Thr Ala Ala

195                 200                 205

Gly Lys Thr Val Ala Ser Pro Cys Ser Ser Thr Glu Ser Leu Ser Gln
    210                 215                 220

Arg Ser Val Thr Ser Val Arg Asp Ser Ala Asp Val Asp Cys Val Leu
225                 230                 235                 240

Asp Leu Ser Val Lys Ser Ser Leu Ser Gly Val Glu Asn Leu Asn Ser
                245                 250                 255

Ser Tyr Phe Ser Ser Gln Asp Val Leu Arg Ser Asn Leu Val Gln Val
            260                 265                 270

Lys Val Glu Lys Glu Ala Ser Cys Asp Glu Ser Asp Val Gly Thr Asn
        275                 280                 285

Asp Tyr Asp Met Glu His Ser Thr Val Lys Glu Ser Val Ser Thr Asn
    290                 295                 300

Asn Arg Val Gln Tyr Glu Pro Ala His Leu Ala Pro Leu Arg Glu Asp
305                 310                 315                 320

Ser Val Leu Arg Glu Leu Asp Arg Glu Asp Lys Ala Ser Asp Asp Glu
                325                 330                 335

Met Met Thr Pro Glu Ser Glu Arg Val Gln Val Glu Gly Gly Met Glu
            340                 345                 350

Asn Ser Leu Leu Pro Tyr Val Ser Asn Ile Leu Ser Pro Ala Gly Gln
        355                 360                 365

Ile Phe Met Cys Pro Leu Cys Asn Lys Val Phe Pro Ser Pro His Ile
    370                 375                 380

Leu Gln Ile His Leu Ser Thr His Phe Arg Glu Gln Asp Gly Ile Arg
385                 390                 395                 400

Ser Lys Pro Ala Ala Asp Val Asn Val Pro Thr Cys Ser Leu Cys Gly
                405                 410                 415

Lys Thr Phe Ser Cys Met Tyr Thr Leu Lys Arg His Glu Arg Thr His
            420                 425                 430

Ser Gly Glu Lys Pro Tyr Thr Cys Thr Gln Cys Gly Lys Ser Phe Gln
        435                 440                 445

Tyr Ser His Asn Leu Ser Arg His Ala Val Val His Thr Arg Glu Lys
    450                 455                 460

Pro His Ala Cys Lys Trp Cys Glu Arg Arg Phe Thr Gln Ser Gly Asp
465                 470                 475                 480

Leu Tyr Arg His Ile Arg Lys Phe His Cys Glu Leu Val Asn Ser Leu
                485                 490                 495

Ser Val Lys Ser Glu Ala Leu Ser Leu Pro Thr Val Arg Asp Trp Thr
            500                 505                 510

Leu Glu Asp Ser Ser Gln Glu Leu Trp Lys
        515                 520

<210>  32
<211>  3428
<212>  DNA
<213>  Artificial

<220>
<223>  Recombinant DNA

<400>  32
ataacttcgt atagcataca ttatacgaag ttatctgaag ttcctatact ttctagagaa      60

taggaacttc tcgagggccc ctgcaggtca attctaccgg gtaggggagg cgcttttccc     120

aaggcagtct ggagcatgcg ctttagcagc cccgctgggc acttggcgct acacaagtgg     180

cctctggcct cgcacacatt ccacatccac cggtaggcgc caaccggctc cgttctttgg     240

tggccccttc gcgccacctt ctactcctcc cctagtcagg aagttccccc ccgccccgca     300

gctcgcgtcg tgcaggacgt gacaaatgga agtagcacgt ctcactagtc tcgtgcagat     360

ggacagcacc gctgagcaat ggaagcgggt aggcctttgg ggcagcggcc aatagcagct     420

ttgctccttc gctttctggg ctcagaggct gggaaggggt gggtccgggg gcgggctcag     480

gggcgggctc aggggcgggg cgggcgcccg aaggtcctcc ggaggcccgg cattctgcac     540

gcttcaaaag cgcacgtctg ccgcgctgtt ctcctcttcc tcatctccgg gcctttcgac     600

ctgcagcctg ttgacaatta atcatcggca tagtatatcg gcatagtata atacgacaag     660

gtgaggaact aaaccatggg atcggccatt gaacaagatg gattgcacgc aggttctccg     720

gccgcttggg tggagaggct attcggctat gactgggcac aacagacaat cggctgctct     780

gatgccgccg tgttccggct gtcagcgcag gggcgcccgg ttctttttgt caagaccgac     840

ctgtccggtg ccctgaatga actgcaggac gaggcagcgc ggctatcgtg gctggccacg     900

acgggcgttc cttgcgcagc tgtgctcgac gttgtcactg aagcgggaag ggactggctg     960

ctattgggcg aagtgccggg gcaggatctc ctgtcatctc accttgctcc tgccgagaaa    1020

gtatccatca tggctgatgc aatgcggcgg ctgcatacgc ttgatccggc tacctgccca 1080

ttcgaccacc aagcgaaaca tcgcatcgag cgagcacgta ctcggatgga agccggtctt 1140

gtcgatcagg atgatctgga cgaagagcat caggggctcg cgccagccga actgttcgcc 1200

aggctcaagg cgcgcatgcc cgacggcgag gatctcgtcg tgacccatgg cgatgcctgc 1260

ttgccgaata tcatggtgga aaatggccgc ttttctggat tcatcgactg tggccggctg 1320

ggtgtggcgg accgctatca ggacatagcg ttggctaccc gtgatattgc tgaagagctt 1380

ggcggcgaat gggctgaccg cttcctcgtg ctttacggta tcgccgctcc cgattcgcag 1440

cgcatcgcct tctatcgcct tcttgacgag ttcttctgag gggatcggca ataaaaagac 1500

agaataaaac gcacgggtgt tgggtcgttt gttcggatct ggacgtaaac tcctcttcag 1560

acctgaagtt cctatacttt ctagagaata ggaacttcgg aatttgatcc tcgagatcct 1620

cggggacacc aaatatggcg atctcggcct tttcgtttct tggagctggg acatgtttgc 1680

catcgatcca tctaccacca gaacggccgt tagatctgct gccaccgttg tttccaccga 1740

agaaaccacc gttgccgtaa ccaccacgac ggttgttgct aaagaagctg ccaccgccac 1800

ggccaccgtt gtagccgccg ttgttgttat tgtagttgct actgttattt ctggcacttc 1860

ttggttttcc tcttaagtga ggaggaacat aaccattctc gttgttgtcg ttgatgctta 1920

aattttgcac ttgttcgctc agttcagcca taatatgaaa tgcttttctt gttgttctta 1980

cggaatacca cttgccacct atcaccacaa ctaacttttt cccgttcctc catctctttt 2040

atattttttt tctcgaggga tctttgtgaa ggaaccttac ttctgtggtg tgacataatt 2100

ggacaaacta cctacagaga tttaaagctc taaggtaaat ataaaatttt taagtgtata 2160

atgtgttaaa ctactgattc taattgtttg tgtattttag attccaacct atggaactga 2220

tgaatgggag cagtggtgga atgcctttaa tgaggaaaac ctgttttgct cagaagaaat 2280

gccatctagt gatgatgagg ctactgctga ctctcaacat tctactcctc caaaaaagaa 2340

gagaaaggta gaagacccca aggactttcc ttcagaattg ctaagttttt tgagtcatgc 2400

tgtgtttagt aatagaactc ttgcttgctt tgctatttac accacaaagg aaaaagctgc 2460

actgctatac aagaaaatta tggaaaaata ttctgtaacc tttataagta ggcataacag 2520

ttataatcat aacatactgt tttttcttac tccacacagg catagagtgt ctgctattaa 2580

taactatgct caaaaattgt gtacctttag ctttttaatt tgtaaagggg ttaataagga 2640

atatttgatg tatagtgcct tgactagaga tcataatcag ccataccaca tttgtagagg 2700

ttttacttgc tttaaaaaac ctcccacacc tccccctgaa cctgaaacat aaaatgaatg 2760

caattgttgt tgttaacttg tttattgcag cttataatgg ttacaaataa agcaatagca 2820

tcacaaattt cacaaataaa gcattttttt cactgcattc tagttgtggt ttgtccaaac 2880

tcatcaatgt atcttatcat gtctggatct gacatggtaa gtaagcttga agttcctata 2940

ctttctagag aataggaact tctttaccac tccctatcag tgatagagaa aagtgaaagt 3000

cgagtttacc actccctatc agtgatagag aaaagtgaaa gtcgagttta ccactcccta 3060

tcagtgatag agaaaagtga aagtcgagtt taccactccc tatcagtgat agagaaaagt 3120

gaaagtcgag tttaccactc cctatcagtg atagagaaaa gtgaaagtcg agtttaccac 3180

tccctatcag tgatagagaa aagtgaaagt cgagtttacc actccctatc agtgatagag 3240

aaaagtgaaa gtcgagctcg gtacccgggt cgagtaggcg tgtacggtgg gaggcctata 3300

taagcagagc tcgtttagtg aaccgtcaga tcgcctggag acgccatcca cgctgttttg 3360

acctccatag aagacaccgg gaccgatcca gcctataact tcgtatagca tacattatac 3420

gaagttat 3428

<210> 33
<211> 3489
<212> DNA
<213> Artificial

<220>
<223> Recombinant DNA

<400> 33
gtcgacaagc ttcccagcgg ccgcgtggat ccacctaata acttcgtata gcatacatta 60

tacgaagtta tctgaagttc ctatactttc tagagaatag gaacttctcg agggcccctg 120

caggtcaatt ctaccgggta ggggaggcgc ttttcccaag gcagtctgga gcatgcgctt 180

tagcagcccc gctgggcact tggcgctaca caagtggcct ctggcctcgc acacattcca 240

catccaccgg taggcgccaa ccggctccgt tctttggtgg ccccttcgcg ccaccttcta 300

ctcctcccct agtcaggaag ttcccccccg ccccgcagct cgcgtcgtgc aggacgtgac 360

aaatggaagt agcacgtctc actagtctcg tgcagatgga cagcaccgct gagcaatgga 420

agcgggtagg cctttggggc agcggccaat agcagctttg ctccttcgct ttctgggctc 480

agaggctggg aaggggtggg tccgggggcg ggctcagggg cgggctcagg ggcggggcgg 540

gcgcccgaag gtcctccgga ggcccggcat tctgcacgct tcaaaagcgc acgtctgccg 600

cgctgttctc ctcttcctca tctccgggcc tttcgacctg cagcctgttg acaattaatc 660

atcggcatag tatatcggca tagtataata cgacaaggtg aggaactaaa ccatgggatc 720

ggccattgaa caagatggat tgcacgcagg ttctccggcc gcttgggtgg agaggctatt 780

cggctatgac tgggcacaac agacaatcgg ctgctctgat gccgccgtgt tccggctgtc 840

agcgcagggg cgcccggttc tttttgtcaa gaccgacctg tccggtgccc tgaatgaact 900

gcaggacgag gcagcgcggc tatcgtggct ggccacgacg ggcgttcctt gcgcagctgt 960

gctcgacgtt gtcactgaag cgggaaggga ctggctgcta ttgggcgaag tgccggggca 1020

ggatctcctg tcatctcacc ttgctcctgc cgagaaagta tccatcatgg ctgatgcaat 1080

gcggcggctg catacgcttg atccggctac ctgcccattc gaccaccaag cgaaacatcg 1140

catcgagcga gcacgtactc ggatggaagc cggtcttgtc gatcaggatg atctggacga 1200

agagcatcag gggctcgcgc cagccgaact gttcgccagg ctcaaggcgc gcatgcccga 1260

cggcgaggat ctcgtcgtga cccatggcga tgcctgcttg ccgaatatca tggtggaaaa 1320

tggccgcttt tctggattca tcgactgtgg ccggctgggt gtggcggacc gctatcagga 1380

catagcgttg gctacccgtg atattgctga agagcttggc ggcgaatggg ctgaccgctt 1440

cctcgtgctt tacggtatcg ccgctcccga ttcgcagcgc atcgccttct atcgccttct 1500

tgacgagttc ttctgagggg atcggcaata aaaagacaga ataaaacgca cgggtgttgg 1560

gtcgtttgtt cggatctgga cgtaaactcc tcttcagacc tgaagttcct atactttcta 1620

gagaatagga acttcggaat ttgatcctcg agatcctcgg ggacaccaaa tatggcgatc 1680

tcggcctttt cgtttcttgg agctgggaca tgtttgccat cgatccatct accaccagaa 1740

cggccgttag atctgctgcc accgttgttt ccaccgaaga aaccaccgtt gccgtaacca 1800

ccacgacggt tgttgctaaa gaagctgcca ccgccacggc caccgttgta gccgccgttg 1860

ttgttattgt agttgctact gttatttctg gcacttcttg gttttcctct taagtgagga 1920

ggaacataac cattctcgtt gttgtcgttg atgcttaaat tttgcacttg ttcgctcagt 1980

tcagccataa tatgaaatgc ttttcttgtt gttcttacgg aataccactt gccacctatc 2040

accacaacta actttttccc gttcctccat ctcttttata ttttttttct cgagggatct 2100

ttgtgaagga accttacttc tgtggtgtga cataattgga caaactacct acagagattt 2160

aaagctctaa ggtaaatata aaatttttaa gtgtataatg tgttaaacta ctgattctaa 2220

ttgtttgtgt attttagatt ccaacctatg gaactgatga atgggagcag tggtggaatg 2280

cctttaatga ggaaaacctg ttttgctcag aagaaatgcc atctagtgat gatgaggcta 2340

ctgctgactc tcaacattct actcctccaa aaaagaagag aaaggtagaa gaccccaagg 2400

actttccttc agaattgcta agttttttga gtcatgctgt gtttagtaat agaactcttg 2460

cttgctttgc tatttacacc acaaaggaaa aagctgcact gctatacaag aaaattatgg 2520

aaaaatattc tgtaaccttt ataagtaggc ataacagtta taatcataac atactgtttt 2580

ttcttactcc acacaggcat agagtgtctg ctattaataa ctatgctcaa aaattgtgta 2640

cctttagctt tttaatttgt aaaggggtta ataaggaata tttgatgtat agtgccttga 2700

ctagagatca taatcagcca taccacattt gtagaggttt tacttgcttt aaaaaacctc 2760

ccacacctcc ccctgaacct gaaacataaa atgaatgcaa ttgttgttgt taacttgttt 2820

attgcagctt ataatggtta caaataaagc aatagcatca caaatttcac aaataaagca 2880

tttttttcac tgcattctag ttgtggtttg tccaaactca tcaatgtatc ttatcatgtc 2940

tggatctgac atggtaagta agcttgaagt tcctatactt tctagagaat aggaacttct 3000

ttaccactcc ctatcagtga tagagaaaag tgaaagtcga gtttaccact ccctatcagt 3060

gatagagaaa agtgaaagtc gagtttacca ctccctatca gtgatagaga aaagtgaaag 3120

tcgagtttac cactccctat cagtgataga gaaaagtgaa agtcgagttt accactccct 3180

atcagtgata gagaaaagtg aaagtcgagt ttaccactcc ctatcagtga tagagaaaag 3240

tgaaagtcga gtttaccact ccctatcagt gatagagaaa agtgaaagtc gagctcggta 3300

cccgggtcga gtaggcgtgt acggtgggag gcctatataa gcagagctcg tttagtgaac 3360

cgtcagatcg cctggagacg ccatccacgc tgttttgacc tccatagaag acaccgggac 3420

cgatccagcc tataacttcg tatagcatac attatacgaa gttattgata aatacgtgct 3480

agtgccacc 3489

<210> 34
<211> 3467
<212> DNA
<213> Artificial

<220>
<223> Recombinant DNA

<400> 34
gcggccgcgt ggatccacct aataacttcg tatagcatac attatacgaa gttatctgaa 60

gttcctatac tttctagaga ataggaactt ctcgagggcc cctgcaggtc aattctaccg 120

ggtaggggag gcgcttttcc caaggcagtc tggagcatgc gctttagcag ccccgctggg 180

cacttggcgc tacacaagtg gcctctggcc tcgcacacat tccacatcca ccggtaggcg 240

ccaaccggct ccgttctttg gtggcccctt cgcgccacct tctactcctc ccctagtcag 300

gaagttcccc cccgccccgc agctcgcgtc gtgcaggacg tgacaaatgg aagtagcacg 360

tctcactagt ctcgtgcaga tggacagcac cgctgagcaa tggaagcggg taggcctttg 420

gggcagcggc caatagcagc tttgctcctt cgctttctgg gctcagaggc tgggaagggg 480

tgggtccggg ggcgggctca ggggcgggct caggggcggg gcgggcgccc gaaggtcctc 540

cggaggcccg gcattctgca cgcttcaaaa gcgcacgtct gccgcgctgt tctcctcttc 600

ctcatctccg ggcctttcga cctgcagcct gttgacaatt aatcatcggc atagtatatc 660

ggcatagtat aatacgacaa ggtgaggaac taaaccatgg gatcggccat tgaacaagat 720

ggattgcacg caggttctcc ggccgcttgg gtggagaggc tattcggcta tgactgggca 780

caacagacaa tcggctgctc tgatgccgcc gtgttccggc tgtcagcgca ggggcgcccg 840

gttctttttg tcaagaccga cctgtccggt gccctgaatg aactgcagga cgaggcagcg 900

cggctatcgt ggctggccac gacgggcgtt ccttgcgcag ctgtgctcga cgttgtcact 960

gaagcgggaa gggactggct gctattgggc gaagtgccgg ggcaggatct cctgtcatct 1020

caccttgctc ctgccgagaa agtatccatc atggctgatg caatgcggcg gctgcatacg 1080

cttgatccgg ctacctgccc attcgaccac caagcgaaac atcgcatcga gcgagcacgt 1140

actcggatgg aagccggtct tgtcgatcag gatgatctgg acgaagagca tcaggggctc 1200

gcgccagccg aactgttcgc caggctcaag gcgcgcatgc ccgacggcga ggatctcgtc 1260

gtgacccatg gcgatgcctg cttgccgaat atcatggtgg aaaatggccg cttttctgga 1320

ttcatcgact gtggccggct gggtgtggcg gaccgctatc aggacatagc gttggctacc 1380

cgtgatattg ctgaagagct tggcggcgaa tgggctgacc gcttcctcgt gctttacggt 1440

atcgccgctc ccgattcgca gcgcatcgcc ttctatcgcc ttcttgacga gttcttctga 1500

ggggatcggc aataaaaaga cagaataaaa cgcacgggtg ttgggtcgtt tgttcggatc 1560

tggacgtaaa ctcctcttca gacctgaagt tcctatactt tctagagaat aggaacttcg 1620

gaatttgatc ctcgagatcc tcggggacac caaatatggc gatctcggcc ttttcgtttc 1680

ttggagctgg gacatgtttg ccatcgatcc atctaccacc agaacggccg ttagatctgc 1740

tgccaccgtt gtttccaccg aagaaaccac cgttgccgta accaccacga cggttgttgc 1800

taaagaagct gccaccgcca cggccaccgt tgtagccgcc gttgttgtta ttgtagttgc 1860

tactgttatt tctggcactt cttggttttc ctcttaagtg aggaggaaca taaccattct 1920

cgttgttgtc gttgatgctt aaattttgca cttgttcgct cagttcagcc ataatatgaa 1980

atgcttttct tgttgttctt acggaatacc acttgccacc tatcaccaca actaactttt 2040

tcccgttcct ccatctcttt tatatttttt ttctcgaggg atctttgtga aggaacctta 2100

cttctgtggt gtgacataat tggacaaact acctacagag atttaaagct ctaaggtaaa 2160

tataaaattt ttaagtgtat aatgtgttaa actactgatt ctaattgttt gtgtatttta 2220

gattccaacc tatggaactg atgaatggga gcagtggtgg aatgccttta atgaggaaaa 2280

cctgttttgc tcagaagaaa tgccatctag tgatgatgag gctactgctg actctcaaca 2340

ttctactcct ccaaaaaaga agagaaaggt agaagacccc aaggactttc cttcagaatt 2400

gctaagtttt ttgagtcatg ctgtgtttag taatagaact cttgcttgct ttgctattta 2460

caccacaaag gaaaaagctg cactgctata caagaaaatt atggaaaaat attctgtaac 2520

ctttataagt aggcataaca gttataatca taacatactg ttttttctta ctccacacag 2580

gcatagagtg tctgctatta ataactatgc tcaaaaattg tgtaccttta gctttttaat 2640

ttgtaaaggg gttaataagg aatatttgat gtatagtgcc ttgactagag atcataatca 2700

gccataccac atttgtagag gttttacttg ctttaaaaaa cctcccacac ctccccctga 2760

acctgaaaca taaaatgaat gcaattgttg ttgttaactt gtttattgca gcttataatg 2820

gttacaaata aagcaatagc atcacaaatt tcacaaataa agcatttttt tcactgcatt 2880

ctagttgtgg tttgtccaaa ctcatcaatg tatcttatca tgtctggatc tgacatggta 2940

agtaagcttg aagttcctat actttctaga gaataggaac ttctttacca ctccctatca 3000

gtgatagaga aaagtgaaag tcgagtttac cactccctat cagtgataga gaaaagtgaa 3060

agtcgagttt accactccct atcagtgata gagaaaagtg aaagtcgagt ttaccactcc 3120

ctatcagtga tagagaaaag tgaaagtcga gtttaccact ccctatcagt gatagagaaa 3180

agtgaaagtc gagtttacca ctccctatca gtgatagaga aaagtgaaag tcgagtttac 3240

cactccctat cagtgataga gaaaagtgaa agtcgagctc ggtacccggg tcgagtaggc 3300

gtgtacggtg ggaggcctat ataagcagag ctcgtttagt gaaccgtcag atcgcctgga 3360

gacgccatcc acgctgtttt gacctccata gaagacaccg ggaccgatcc agcctataac 3420

ttcgtatagc atacattata cgaagttatt gataaatacg tgctagt 3467


<210> 35
<211> 118
<212> DNA
<213> Mus musculus

<400> 35
aacctccgag ggtccgcggt agcggtggac gggtcgatcc gtggagtgga tgtcttcggg 60

ccattgcggt gcttgggact cattaaactg tcactcaccc ccaccaccac cccactgg 118


<210> 36
<211> 84
<212> DNA
<213> Mus musculus

<400> 36
tctgcttttt ttccaccgat gtaacagacc tgcagccagc aagactccga gggaaggact 60

taatcaagtt tatgtgtcct aaag 84

## Description

### Technical Field

The present invention relates to a transgenic non-human animal capable of controlling (increasing or decreasing) the expression of the transcription factor RP58, etc.

### Background Art

From the nascent stage, the transcription factor RP58 (alias: ZNF238, Zfp238, or ZBTB18) is expressed in excitatory neurons, and is important for the proliferation or transition of neurons and projection formation (Non Patent Literature 1: Okado et al., Dev Biol, 2009). RP58 is a responsible gene for 1q44 deletion syndrome that exhibits symptoms such as human mental retardation, agenesis (hypoplasia) of corpus callosum, and epilepsy. It has been reported that a large number of heterozygous mutations of RP58 are found in patients with developmental delays, including intellectual disability (Non Patent Literature 2: Perlman et al., Am J Med Genet A. 2013; Non Patent Literature 3: Westphal et al., Gene, 2017; Non Patent Literature 4: Ehmke et al., Am J Med Genet A. 2017; Non Patent Literature 5: de Munnik et al., Eur J Hum Genet. 2014; Non Patent Literature 6: Rauch et al., Lancet. 2012; Non Patent Literature 7: Srivastava et al., Ann Neurol. 2014; Non Patent Literature 8: Cohen et al., Clin Genet. 2017; Non Patent Literature 9: Deciphering Developmental Disorders Study. Nature. 2017; and Non Patent Literature 10: van der Schoot et al., Mol Genet Genomic Med. 2018). In addition, it has been also reported that the expression level of RP58 is decreased in human aged frontal lobe (Non Patent Literature 11: Lu et al., Nature, 2004). Moreover, it has been suggested that a decrease in the expression level of RP58 promotes acceleration in the malignancy of brain tumor (Non Patent Literature 12: Carro et al., Nature, 2010). These findings suggest that sufficient expression f RP58 is essential for the maintenance of brain functions.

In studies using RP58 animal models, it has been proved using the fetal brain of RP58 knockout mice that a deficiency of RP58 specifically expressed in glutamatergic neurons causes hypoplasia of the cerebral cortex and the hippocampus (Non Patent Literature 1: Okado et al., Dev Biol, 2009). When the molecular mechanism was analyzed by *in utero* electroporation using a viral vector, it was found that suppression of the transcription of Idl-4 by RP58 is essential f or cell cycle exit occurring upon differentiation of neutrons from progenitor cells, and that RP58 binds to the transcriptional regulatory region of Id1-4 and directly suppresses the transcription thereof (Non Patent Literature 13: Hirai et al., EMBO J, 2012). Moreover, it has been reported that, by using conditional RP58 knockout mice, it was found that suppression of the transcription of Ngn2 and Rnd2 by RP58 is required for the radial migration of neurons after differentiation of the neurons from progenitor cells, and that RP58 directly binds to their promoter and suppresses the transcription (Non Patent Literature 14: Ohtaka-Maruyama et al., Cell Rep, 2013; and Non Patent Literature 15: Heng et al., Cereb Cortex. 2015). Furthermore, microcephaly, agenesis of corpus callosum, and cerebellar hypoplasia were found after the birth of conditional knockout *RP58fl*/*fl:Nestin-Cre* mice (Non Patent Literature 16: Xiang C et al., Cell Death Differ. 2012). Further, in conditional knockout *RP58jl*/*fl:Atoh1-CreER* mouse models, severe cerebellar hypoplasia was found, and it has been reported that RP58 is required for both glutamatergic and GABAergic neurons (Non Patent Literature 17: Baubet et al., Development, 2012). In these analyses using RP58-deficient or RP58-conditionally-deficient mouse models, the mice from the fetal stage before the weaning stage (i.e., the nascent stage) were used as subjects, and thus, the mice after the weaning stage, namely, the mice in the adolescence, maturity and old age stages (i.e., during and after the developmental stage) have not been subjected to the analyses.

### Citation List

Non Patent Literature 1: Okado H, Ohtaka-Maruyama C, Sugitani Y, Fukuda Y, Ishida R, Hirai S, Miwa A, Takahashi A, Aoki K, Mochida K, Suzuki O, Honda T, Nakajima K, Ogawa M, Terashima T, Matsuda J, Kawano H, Kasai M. The transcriptional repressor RP58 is crucial for cell-division patterning and neuronal survival in the developing cortex. Dev Biol. 2009 Jul 15; 331(2): 140-51. doi: 10.1016/j.ydbio.2009.04.030.
Non Patent Literature 2: Perlman SJ, Kulkarni S, Manwaring L, Shinawi M. Haploinsufficiency of ZNF238 is associated with corpus callosum abnormalities in 1q44 deletions. Am J Med Genet A. 2013 Apr; 161A(4): 711-6. doi: 10.1002/ajmg.a.35779
Non Patent Literature 3: Westphal DS, Andres S, Beitzel KI, Makowski C, Meitinger T, Hoefele J. Identification of a de novo microdeletion 1q44 in a patient with hypogenesis of the corpus callosum, seizures and microcephaly - A case report. Gene. 2017 Jun 15;616: 41-44. doi: 10.1016/j.gene.2017.03.025.
Non Patent Literature 4: Ehmke N, Karge S, Buchmann J, Korinth D, Horn D, Reis O, Häßler F. A de novo nonsense mutation in ZBTB18 plus a de novo 15q13.3 microdeletion in a 6-year-old female. Am J Med Genet A. 2017 May; 173(5): 1251-1256. doi: 10.1002/ajmg.a.38145.
Non Patent Literature 5: De Munnik SA, García-Miñaúr S, Hoischen A, van Bon BW, Boycott KM, Schoots J, Hoefsloot LH, Knoers NV, Bongers EM, Brunner HG. A de novo non-sense mutation in ZBTB18 in a patient with features of the 1q43q44 microdeletion syndrome. Eur J Hum Genet. 2014 Jun; 22(6): 844-6. doi: 10.1038/ejhg.2013.249.
Non Patent Literature 6: Rauch A, Wieczorek D, Graf E, Wieland T, Endele S, Schwarzmayr T, Albrecht B, Bartholdi D, Beygo J, Di Donato N, Dufke A, Cremer K, Hempel M, Horn D, Hoyer J, Joset P, Röpke A, Moog U, Riess A, Thiel CT, Tzschach A, Wiesener A, Wohlleber E, Zweier C, Ekici AB, Zink AM, Rump A, Meisinger C, Grallert H, Sticht H, Schenck A, Engels H, Rappold G, Schröck E, Wieacker P, Riess O, Meitinger T, Reis A, Strom TM. Range of genetic mutations associated with severe non-syndromic sporadic intellectual disability: an exome sequencing study. Lancet. 2012 Nov 10; 380(9854): 1674-82. doi: 10.1016/S0140-6736(12)61480-9.
Non Patent Literature 7: Srivastava S, Cohen JS, Vernon H, Barañano K, McClellan R, Jamal L, Naidu S, Fatemi A. Clinical whole exome sequencing in child neurology practice. Ann Neurol. 2014 Oct; 76(4): 473-83. doi: 10.1002/ana.24251.
Non Patent Literature 8: Cohen JS, Srivastava S, Farwell Hagman KD, Shinde DN, Huether R, Darcy D, Wallerstein R, Houge G, Berland S, Monaghan KG, Poretti A, Wilson AL, Chung WK, Fatemi A. Further evidence that de novo missense and truncating variants in ZBTB18 cause intellectual disability with variable features. Clin Genet. 2017 May; 91(5): 697-707. doi: 10.1111/cge.12861.
Non Patent Literature 9: McRae JF et al., Deciphering Developmental Disorders Study. Prevalence and architecture of de novo mutations in developmental disorders. Nature. 2017 Feb 23; 542(7642): 433-438. doi: 10.1038/nature21062. Epub 2017 Jan 25.
Non Patent Literature 10: Van der Schoot V, de Munnik S, Venselaar H, Elting M, Mancini GMS, Ravenswaaij-Arts CMA, Anderlid BM, Brunner HG, Stevens SJC. Toward clinical and molecular understanding of pathogenic variants in the ZBTB18 gene. Mol Genet Genomic Med. 2018 May; 6(3): 393-400. doi: 10.1002/mgg3.387. 24.
Non Patent Literature 11: Lu T, Pan Y, Kao SY, Li C, Kohane I, Chan J, Yankner BA. Gene regulation and DNA damage in the ageing human brain. Nature. 2004 Jun 24; 429(6994): 883-91.
Non Patent Literature 12: Carro MS, Lim WK, Alvarez MJ, Bollo RJ, Zhao X, Snyder EY, Sulman EP, Anne SL, Doetsch F, Colman H, Lasorella A, Aldape K, Califano A, Iavarone A. The transcriptional network for mesenchymal transformation of brain tumours. Nature. 2010 Jan 21; 463(7279): 318-25. doi: 10.1038/nature08712.
Non Patent Literature 13: Hirai S, Miwa A, Ohtaka-Maruyama C, Kasai M, Okabe S, Hata Y, Okado H. RP58 controls neuron and astrocyte differentiation by downregulating the expression of Id1-4 genes in the developing cortex. EMBO J. 2012 Mar 7; 31(5): 1190-202. doi: 10.1038/emboj.2011.486.
Non Patent Literature 14: Ohtaka-Maruyama C, Hirai S, Miwa A, Heng JI, Shitara H, Ishii R, Taya C, Kawano H, Kasai M, Nakajima K, Okado H. (2013) RP58 regulates the multipolar-bipolar transition of newborn neurons in the developing cerebral cortex. Cell Rep. 3: 458-71.
Non Patent Literature 15: Heng JI, Qu Z, Ohtaka-Maruyama C, Okado H, Kasai M, Castro D, Guillemot F, Tan SS. The zinc finger transcription factor RP58 negatively regulates Rnd2 for the control of neuronal migration during cerebral cortical development. Cereb Cortex. 2015 Mar; 25(3): 806-16. doi: 10.1093/cercor/bht277.
Non Patent Literature 16: Xiang C, Baubet V, Pal S, Holderbaum L, Tatard V, Jiang P, Davuluri RV, Dahmane N. RP58/ZNF238 directly modulates proneurogenic gene levels and is required for neuronal differentiation and brain expansion. Cell Death Differ. 2012 Apr; 19(4): 692-702. doi: 10.1038/cdd.2011.144.
Non Patent Literature 17: Baubet V, Xiang C, Molczan A, Roccograndi L, Melamed S, Dahmane N. Rp58 is essential for the growth and patterning of the cerebellum and for glutamatergic and GABAergic neuron development. Development. 2012 Jun; 139(11): 1903-9. doi: 10.1242/dev.075606.
Non Patent Literature 18: Vogel-Ciernia A, Wood MA. Examining object location and object recognition memory in mice. Curr Protoc Neurosci. 2014 Oct 8; 69: 8.31.1-17. doi: 10.1002/0471142301.ns0831s69.
Non Patent Literature 19: Denninger, J.K., Smith, B.M., Kirby, E.D. Novel Object Recognition and Object Location Behavioral Testing in Mice on a Budget. J. Vis. Exp. 2018 (141), e58593, doi:10.3791/58593.
Non Patent Literature 20: Tal Yardeni, Michael Eckhaus, H. Douglas Morris, Marjan Huizing, and Shelley Hoogstraten-Miller. Retro-orbital injections in mice. Lab Anim (NY). 2011 May; 40(5): 155-160. doi:10.1038/laban0511-155.
Non Patent Literature 21: Juan C. Brenes JC, Michael Padilla, M, Jaime Fornaguera, J, A detailed analysis of open-field habituation and behavioral and neurochemical antidepressant-like effects in postweaning enriched rats. Behavioural Brain Research. 2009, 197, 125-137 doi:10.1016/j.bbr.2008.08.014
Non Patent Literature 22: Rojas-Carvajal M, Jaime Fornaguera J, Andrea Mora-Gallegos A, Juan C. Brenes JC Testing experience and environmental enrichment potentiated open-field habituation and grooming behaviour in rats Animal Behavior, 2018, 137, 1-11. doi: org/10.1016/j.anbehav.2018.01.018
Non Patent Literature 23: Saito T, Saido TC. Neuroinflammation in mouse models of Alzheimer's disease.Clin Exp Neuroimmunol. 2018 Nov; 9(4): 211-218. doi: 10.1111/cen3.12475.
Non Patent Literature 24: Matsuzaki Y, Konno A, Mochizuki R, Shinohara Y, Nitta K, Okada Y, Hirai H. Intravenous administration of the adeno-associated virus-PHP.B capsid fails to upregulate transduction efficiency in the marmoset brain. Neurosci Lett. 2018 Feb 5; 665: 182-188. doi: 10.1016/j.neulet.2017.11.049.
Non Patent Literature 25: Castillo E, Leon J, Mazzei G, et al. Comparative profiling of cortical gene expression in Alzheimer's disease patients and mouse models demonstrates a link between amyloi- dosis and neuroinflammation. Sci Rep. 2017; 7: 17762. doi: 10.1038/s41598-017-17999-3

### Summary of Invention

Under such circumstances, the present inventor focused on the usefulness of discovering the novel functions of the transcription factor RP58 in cells of the central nervous system such as brain nervous system cells, in particular, the functions of RP58 not only in the nascent stage, but also during and after the developmental stage.

The present invention has been made, while taking into consideration the aforementioned circumstances. The present invention provides, for example, a transgenic non-human animal, a method of screening for a therapeutic or preventive agent against brain dysfunction, etc., and a pharmaceutical composition for use in the treatment or prevention of brain dysfunction, and the like, as described below.

(1) A transgenic non-human animal capable of increasing or decreasing the expression of a transcription factor RP58 in cells of the central nervous system of the non-human animal in the nascent stage and/or during and after the developmental stage.
(2) The transgenic non-human animal according to the above (1), which is capable of increasing or decreasing the expression of the RP58 in cells of the central nervous system of the non-human animal during and after the developmental stage.
(3) The transgenic non-human animal according to the above (1) or (2), which is capable of increasing or decreasing the expression of the RP58, in a desired period in the nascent stage and/or during and after the developmental stage of the non-human animal.
(4) The transgenic non-human animal according to any one of the above (1) to (3), which is capable of increasing or decreasing the expression of the RP58 according to a Tet system.
(5) The transgenic non-human animal according to the above (4), into which a promoter and a tetracycline-controlled transcriptional activator (tTA) gene linked to the promoter so that the tTA gene can function by the promoter, and a Tet operator (tetO) are introduced, and which is capable of increasing the expression of the RP58 by non-administration of doxycycline.
(6) The transgenic non-human animal according to the above (4), into which a promoter and a tetracycline-controlled transcriptional silencer (tTS) gene linked to the promoter so that the tTS gene can function by the promoter, and a Tet operator (tetO) are introduced, and which is capable of decreasing the expression of the RP58 by non-administration of doxycycline.
(7) The transgenic non-human animal according to the above (5) or (6), wherein the promoter is a Camk2a promoter or an Actin promoter.
(8) The transgenic non-human animal according to any one of the above (5) to (7), wherein the expression of the RP58 is maintained at a normal level by administration of doxycycline.
(9) The transgenic non-human animal according to any one of the above (1) to (8), wherein the cells of the central nervous system are brain nervous system cells.
(10) The transgenic non-human animal according to any one of the above (1) to (9), wherein the non-human animal is a rodent.
(11) The transgenic non-human animal according to the above (10), wherein the rodent is a mouse.
(12) An animal model of brain dysfunction or a disease related thereto, in which the expression of the transcription factor RP58 is decreased in cells of the central nervous system of the transgenic non-human animal according to any one of the above (1) to (4) and (6) to (11).
(13) The animal model according to the above (12), wherein the expression of the RP58 is decreased in cells of the central nervous system of the transgenic non-human animal in during and after the developmental stage.
(14) The animal model according to the above (12) or (13), wherein the brain dysfunction is at least one selected from the group consisting of brain tumor, cognitive dysfunction, developmental disability, intellectual disability, memory impairment, and age-related brain disorder.
(15) A method of screening for a therapeutic or preventive agent against brain dysfunction or a disease related thereto, which comprises a step of decreasing the expression of the transcription factor RP58 in cells of the central nervous system of the transgenic non-human animal according to any one of the above (1) to (4) and (6) to (11), a step of administering a candidate substance to the non-human animal in which the expression of the RP58 is decreased, and a step of evaluating the pathological condition of the brain dysfunction or a disease related thereto in the non-human animal after administration of the candidate substance.
(16) The screening method according to the above (15), wherein the step of decreasing the expression of the RP58 is a step of decreasing the expression of the RP58 in cells of the central nervous system of the transgenic non-human animal in during and after the developmental stage.
(17) The screening method according to the above (15) or (16), wherein the brain dysfunction is at least one selected from the group consisting of brain tumor, cognitive dysfunction, developmental disability, intellectual disability, memory impairment, and age-related brain disorder.
(18) A pharmaceutical composition for use in the treatment or prevention of brain dysfunction or a disease related thereto, wherein the pharmaceutical composition comprises a transcription factor RP58 protein or a nucleic acid encoding the transcription factor RP58.
(19) A pharmaceutical composition for use in the treatment or prevention of brain dysfunction or a disease related thereto, wherein the pharmaceutical composition comprises a therapeutic or preventive agent obtained by the screening method according to any one of the above (15) to (17).
(20) The pharmaceutical composition according to the above (18) or (19), wherein the brain dysfunction is at least one selected from the group consisting of brain tumor, cognitive dysfunction, developmental disability, intellectual disability, memory impairment, and age-related brain disorder.
(21) A method for treating or preventing brain dysfunction or a disease related thereto, which comprises administering the pharmaceutical composition according to any one of the above (18) to (20) to a patient who has or is likely to have brain dysfunction or a disease related thereto.
(22) The therapeutic or preventive method according to the above (21), wherein the brain dysfunction is at least one selected from the group consisting of brain tumor, cognitive dysfunction, developmental disability, intellectual disability, memory impairment, and age-related brain disorder.
(23) An animal model of behavioral disorder or a disease related thereto, in which the expression of the transcription factor RP58 is increased in cells of the central nervous system of the transgenic non-human animal according to any one of the above (1) to (5) and (7) to (11).
(24) The animal model according to the above (23), wherein the behavioral disorder or a disease related thereto is at least one selected from the group consisting of borderline personality disorder, schizophrenia, depression, bipolar disorder, eating disorder, dissociative disorder, post-traumatic stress syndrome, addiction, autism, and self-injury.
(25) A method of screening for a therapeutic or preventive agent against behavioral disorder or a disease related thereto, which comprises a step of increasing the expression of the transcription factor RP58 in cells of the central nervous system of the transgenic non-human animal according to any one of the above (1) to (5) and (7) to (11), a step of administering a candidate substance to the non-human animal in which the expression of the RP58 is increased, and a step of evaluating the pathological condition of the behavioral disorder or a disease related thereto in the non-human animal after administration of the candidate substance.
(26) The screening method according to the above (25), wherein the behavioral disorder or a disease related thereto is at least one selected from the group consisting of borderline personality disorder, schizophrenia, depression, bipolar disorder, eating disorder, dissociative disorder, post-traumatic stress syndrome, addiction, autism, and self-injury.
(27) The screening method according to the above (25) or (26), wherein the candidate substance is a nucleic acid capable of suppressing the function of a gene encoding RP58.
(28) The screening method according to the above (27), wherein the nucleic acid is antisense DNA, siRNA, shRNA, or microRNA.
(29) A pharmaceutical composition for use in the treatment or prevention of behavioral disorder or a disease related thereto, wherein the pharmaceutical composition comprises a therapeutic or preventive agent obtained by the screening method according to any one of the above (25) to (28).
(30) The pharmaceutical composition according to the above (29), wherein the behavioral disorder or a disease related thereto is at least one selected from the group consisting of borderline personality disorder, schizophrenia, depression, bipolar disorder, eating disorder, dissociative disorder, post-traumatic stress syndrome, autism, addiction, and self-injury.
(31) A method for treating or preventing behavioral disorder or a disease related thereto, which comprises administering the pharmaceutical composition according to the above (29) or (30) to a patient who has or is likely to have behavioral disorder or a disease related thereto.
(32) The therapeutic or preventive method according to the above (31), wherein the behavioral disorder or a disease related thereto is at least one selected from the group consisting of borderline personality disorder, schizophrenia, depression, bipolar disorder, eating disorder, dissociative disorder, post-traumatic stress syndrome, addiction, autism, and self-injury.
(33) A method of screening for a substance that improves or reduces cognitive function, which comprises administering a candidate substance to a wild-type non-human animal, or allowing a candidate substance to come into contact with wild-type animal cells, and then screening the substance that improves or reduces cognitive function, using, as an indicator, the expression level of the transcription factor RP58 in the brain of the non-human animal after the administration or in the animal cells after the contact.
(34) An agent for improving or reducing cognitive function, comprising a substance obtained by the screening method according to the above (33).
(35) A method for improving or reducing cognitive function, comprising administering the agent for improving or reducing cognitive function according to the above (34) to a subj ect.
(36) A method of screening for an environment that improves or reduces cognitive function, which comprises breeding a wild-type non-human animal under a candidate environment, or culturing wild-type animal cells under a candidate environment, and then screening the environment that improves or reduces cognitive function, using, as an indicator, the expression level of the transcription factor RP58 in the brain of the non-human animal after the breeding or in the animal cells after the culture.
(37) A method for improving or reducing cognitive function, comprising providing the environment screened by the method according to the above (36) to a subject.

### Advantageous Effects of Invention

According to the present invention, there can be provided: a transgenic non-human animal capable of increasing or decreasing the expression of a transcription factor RP58 in cells of the central nervous system of a non-human animal in the nascent stage and/or during and after the developmental stage ; an animal model of brain dysfunction or the like, in which the aforementioned non-human animal is used; and further, a method of screening for a therapeutic or preventive agent against brain dysfunction or the like; a pharmaceutical composition for use in the treatment or prevention of the brain dysfunction or the like; etc.

### Brief Description of Drawings

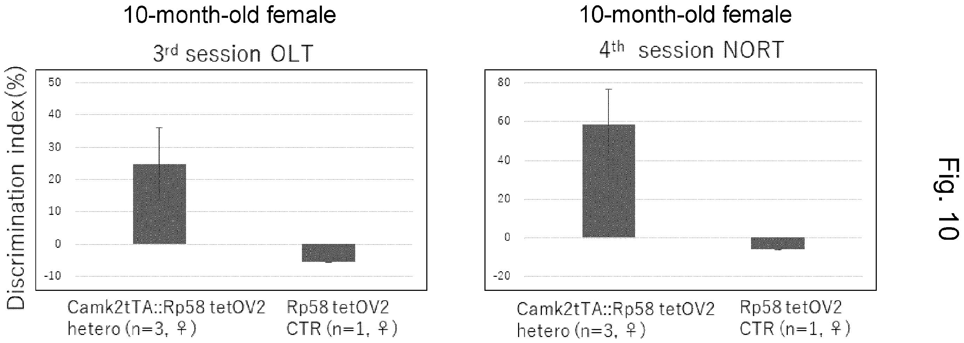

[Figure 1] Figure 1 is a view showing the protocols of an object location recognition test.
[Figure 2] Figure 2 is a view showing the protocols of an object location recognition + novel object recognition test.
[Figure 3] Figure 3 is a schematic view showing an administration method to the orbital sinus of a mouse. The adeno-associated virus vector produced in the Examples of the present application was administered together with Evans blue, using a normal saline as a solvent.
[Figure 4] Figure 4 is a view showing the results of an object location recognition test performed on wild-type male B6 mice with an age of 4 months old (4 mice), 10 months old (3 mice), and 19 months old (4 mice) (±SE, ^{∗∗} P < 0.05, ^{∗∗∗} P < 0.005, TTEST).
[Figure 5] Figure 5 is a view showing the results obtained by staining the hippocampal and dentate gyrus of ActintTS::Rp58 tetO homo or hetero mice (5-month-old), in which the expression of RP58 was decreased after P21, with RP58, NeuN, and DAPI. As control samples, Rp58 tetO homo mice were used.
[Figure 6] Figure 6 is a view showing the results of an object location recognition test performed on RP58 expression decrease models (4- to 5-month-old) after P21 (CTR tetO (n = 6, male), ActintTS::RP58tetO Hetero (n = 6, male), and ActintTS::RP58tetO Homo (n = 8, male), ±SE, ^{∗∗} P < 0.05, ^{∗∗∗} P < 0.005, TTEST).
[Figure 7] Figure 7 is a view showing the results of an object location recognition test performed on RP58 expression decrease models (4-month-old) after P60 (CTR tetO (male, n = 2; female, n = 2), ActintTS::RP58tetO Hetero (male, n = 2), and ActintTS::RP58tetO Homo (male, n = 2; female, n = 1)).
[Figure 8] Figure 8 is a view showing the results obtained by staining Camk2tTA:: Rp58 tetO hetero mice (2-month-old), in which the expression of RP58 in mature excitatory neurons RP58 was increased from the early nascent stage, with RP58. As control samples, Rp58 tetO hetero mice were used.
[Figure 9] Figure 9 is a view showing the results of an object location recognition test performed on RP58 expression increase models (4-month-old and 15-month-old) from the early nascent stage.
   4-Month-old:
      Camk2tTA::RP58tetO Hetero (male, n = 3), CTR tetO (male, n = 3)
      Camk2tTA::RP58tetO Hetero (female, n = 7), CTR tetO (female, n = 4)
   15-Month-old:
      Camk2tTA::RP58tetO Hetero (male, n = 4), CTR tetO (male, n = 5)
      Camk2tTA::RP58tetO Hetero (female, n = 3), CTR tetO (female, n = 5)
      ±S.E, ^{∗∗} P < 0.05, ^{∗∗∗} P < 0.005, TTEST
[Figure 10] Figure 10 is a view showing the results of an object location recognition test (OLT, 3rd session) and a novel object recognition test (NORT, 4th session) performed on RP58 expression increase models (10-month-old) from the early nascent stage (Camk2tTA::RP58tetO Hetero (female, n = 3), and CTR tetO (female, n = 1)).

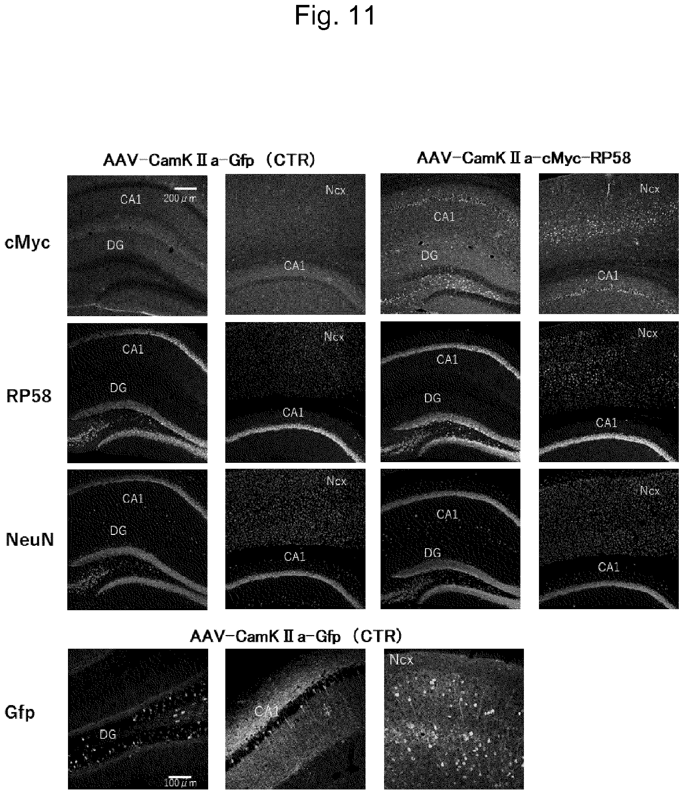

[Figure 11] Figure 11 is a view showing that RP58 can be overexpressed in the mature neurons of hippocampus and cerebral cortex by intravenous gene transfer with AAV-CaMKIIa-cMyc-RP58. The mature neurons were stained with cMyc, RP58, and NeuN, and as a result, the levels of the former two (cMyc and RP58) were increased. As control samples, mice administered with AAV-CaMKIIa-Gfp were used, and the results obtained by staining with a GFP antibody were shown. GFP can be expressed at a high level in the mature neurons.

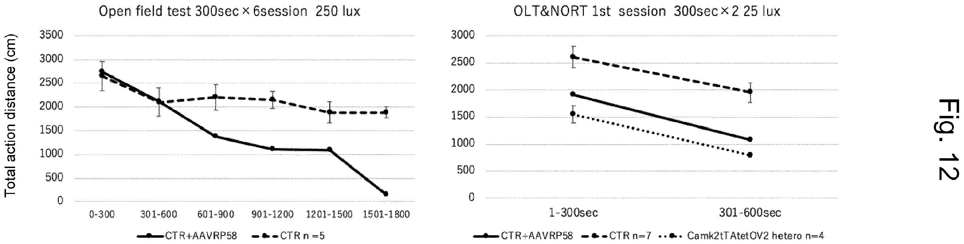

[Figure 12] Figure 12 is a view showing the results of an open field test (left view) and the results of 1st session (habituation) of an object location recognition + novel object recognition test (right view), which were performed on mice (CTR + AAV-RP58) prepared by the forced expression of RP58 in 3-month-old normal controls by using adeno-associated virus (AAV-CaMKIIa-cMyc-RP58).
   Left view: The horizontal axis indicates a total of 30 minutes from the initial 5 minutes (0-300 sec) to the last 5 minutes (1501-1800 sec), which are indicated with the unit "second." Habituation is accelerated in mice in which RP58 is forcibly expressed by AAV.
   Right view: The OLT & NORT 1^{st} session of the initial 5 minutes (0-300 sec) and the subsequent 5 minutes (301-600 sec). It correspond to the same open field in the second session 1 week later. Habituation is accelerated in mice in which RP58 is forcibly expressed by AAV (orange), as with genetically modified mice in which the expression of RP58 is increased (green).

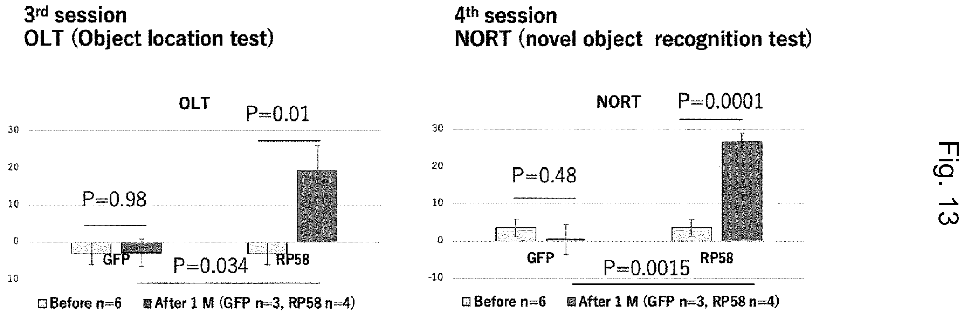

[Figure 13] Figure 13 shows the results obtained by administering a GFP-expressed or RP58-expressed adeno-associated virus to 3-month-old AD mouse models, and then performing an object location recognition (OLT) and a novel object recognition test (NORT) on the mouse models with 4 months old. Cognitive function, which had been almost lost in the 3-month-old mouse models, was returned to the normal level by overexpression of RP58.

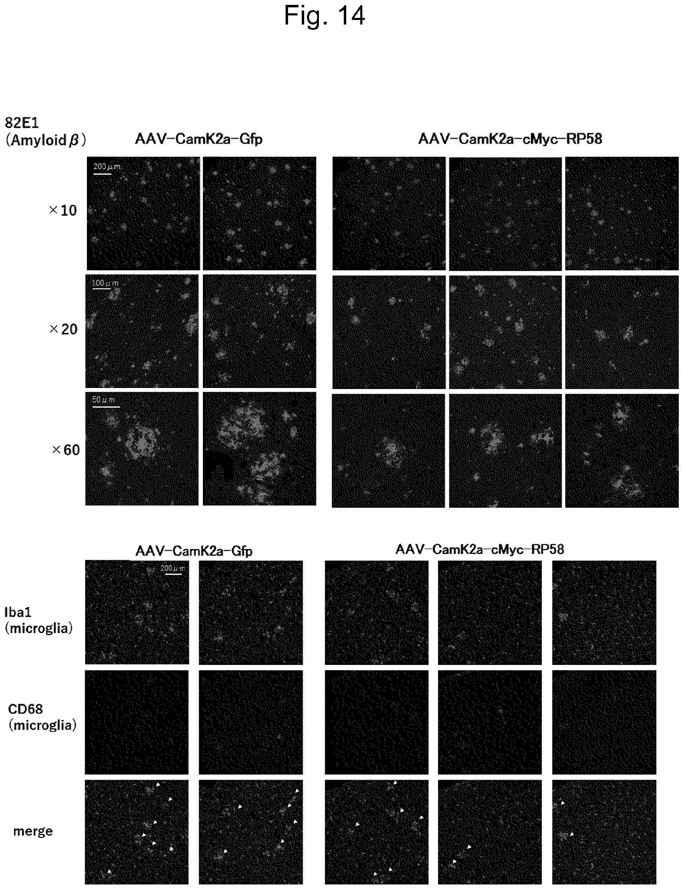

[Figure 14] Figure 14 is a view showing the results obtained by administering an RP58-expressed adeno-associated virus (AAV-CaMKIIa-cMyc-RP58) to 3-month-old Alzheimer's disease mouse models, and then staining the prefrontal cortex of the mouse models one month after the administration (i.e., 4-month-old mice) with the amyloid β marker 82E1 and with the microglia markers Iba1 and CD68. As control samples, mice administered with AAV-CaMKIIa-Gfp were used.

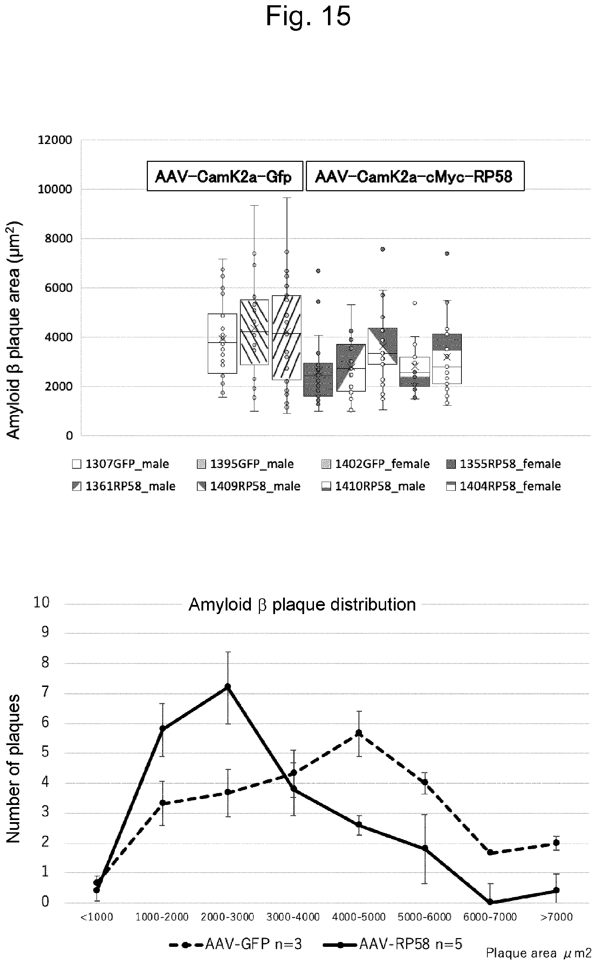

[Figure 15] Figure 15 is a view showing the area of amyloid plaque (upper view) and size distribution (lower view) of 3-month-old Alzheimer's disease mouse models 1 month after administration of RP58-expressed adeno-associated virus (AAV-CaMKIIa-cMyc-RP58) (i.e., 4-month-old mice) (Figure 14). As control samples, mice administered with AAV-CaMKIIa-Gfp were used. In the box plot of the upper view, the upper end, center, and lower end of the box all indicate a quartile, and the x plotted in the box indicates a mean value.

### Description of Embodiments

Hereinafter, the present invention will be described in detail. The scope of the present invention is not constrained by these explanations, and the present invention may be modified, as appropriate, and may be carried out within the range that does not impair the spirit of the present invention, even with regard to those other than the following examples. It is to be noted that all publications cited in the present description, such as, for example, prior art documents, and patent documents such as published unexamined patent applications and patent applications, are incorporated herein by reference in their entireties.

### 1. Summary of the Invention

The transcription factor RP58 is mainly expressed in the excitatory neurons of the cerebral cortex. To date, it has been elucidated that the transcription factor RP58 plays a role for generation and migration of neurons in the nascent stage. In order to newly clarify the function of RP58 for cognitive function during and after the developmental stage, the present inventor has produced mice capable of artificially and freely deceasing the expression of RP58, and mice capable of freely increasing (accelerating) the expression of RP58 in the mature excitatory neurons thereof. Regarding the cognitive function tests, an "object location recognition test" was carried out with reference to the method of Vogel-Ciernia and Wood et al. (the above-mentioned Non Patent Literature 18: Vogel-Ciernia and Wood, Curr Protoc Neurosci, 2014), and continuous evaluation of "object location recognition + novel object recognition test s" was carried out with reference to the method of Denninger et al. (the above-mentioned Non Patent Literature 19: Denninge et al., J. Vis. Exp. 2018). As a result, when the expression of RP58 was decreased in mature mice in an amount-dependent manner, and was further approached to age-related cognitive function. On the other hand, when the expression of RP58 was increased (accelerated) in mature excitatory neurons, cognitive function was rather accelerated, and a reduction in cognitive function due to aging was suppressed. From the aforementioned results, it was demonstrated that the transcription factor RP58 controls cognitive function and a change in cognitive function due to aging in an amount-dependent manner.

Moreover, when RP58 was overexpressed in wild-type mice using an adeno-associated virus vector, an acceleration in cognitive function was observed. Furthermore, when RP58 was overexpressed in knock-in mice with a human mutant amyloid precursor protein (APP) (Alzheimer's disease (AD) mouse models) using an adeno-associated virus vector, reduced cognitive function could be recovered.

By the way, the aforementioned transcription factor RP58 has high species preservation, and at a protein level, a variant of 531 amino acids (531 aa) (human: SEQ ID NO: 17, mouse: SEQ ID NO: 29) and a variant of 522 amino acids (522 aa) (human: SEQ ID NO: 19, mouse: SEQ ID NO: 31) are present between humans and mice. Between humans and mice, 99% of amino acids are identified to one another. Besides, the 531 aa variant protein has a structure in which 9 amino acids are added to the N-terminal side of the 522 aa variant protein (i.e., the 5'-terminal side in the corresponding cDNA sequence).

In the case of RP58, Exons 1 to 4 (SEQ ID NOs: 2 to 5, successively from Exon 1) are present in human genomic DNA (here is exemplified the nucleotide sequence (SEQ ID NO: 1) from the 244048939th nucleotide to the 244057476th nucleotide of DNA registered under GenBank Accession Number: NC_000001.11; hereafter, all registration numbers are GenBank Accession Numbers). Also, in mouse genomic DNA (here is exemplified the nucleotide sequence (SEQ ID NO: 6) from the 177442356th nucleotide to the 177450764th nucleotide of DNA registered under NC_000067.6), Exons 1 to 4 (SEQ ID NOs: 7 to 10, successively from Exon 1) are present, and further, Exon 1b (SEQ ID NO: 35) and Exon 2b (SEQ ID NO: 36) are also present. Regarding human RP58, mainly, three mRNA splicing variants (NM_205768.2 (SEQ ID NO: 13), NM_006352.4 (SEQ ID NO: 15), and NM_001278196.1 (SEQ ID NO: 11)) and the like have been reported. Other than these variants, however, several variants have been reported (XM_017000060.1 (SEQ ID NO: 12) and XM_005273006.2 (SEQ ID NO: 14)). On the other hand, regarding mouse RP58, three mRNA splicing variants (NM_001012330.1 (SEQ ID NO: 26), NM 013915.3 (SEQ ID NO: 27), and NM_001355099.1 (SEQ ID NO: 20)), which correspond to the above-described three human variants, have been reported, and other than these variants, several variants have been reported (XM_006496889.3 (SEQ ID NO: 24), XM 006496890.3 (SEQ ID NO: 25), NM_001355100.1 (SEQ ID NO: 22), NM_001355101.1 (SEQ ID NO: 21), and XM_006496896.3 (SEQ ID NO: 23)). Moreover, it is considered that a plurality of unknown or unreported mRNA splicing variants will be present in both humans and mice. In the present invention, with regard to the transcription factor RP58 used in the following various types of explanations, the sequence information of all of the above-described variants, including unknown or unreported variants, can be referred and/or adopted.

### 2. Transgenic non-human animal

As described above, the transgenic non-human animal of the present invention is a transgenic non-human animal, in which the expression of the transcription factor RP58 can be increased or decreased in cells of the central nervous system of the non-human animal in the nascent stage and/or the during and after developmental stage. Among others, the present transgenic non-human animal is preferably a transgenic non-human animal, in which the expression of RP58 can be increased or decreased in cells of the central nervous system of the non-human animal during and after the developmental stage. Moreover, the present transgenic non-human animal is preferably a transgenic non-human animal, in which the expression of RP58 can be increased or decreased in a desired period of time in the above-described nascent stage and/or during and after the above-described developmental stage, namely, an increase or decrease in the expression of RP58 can be artificially and freely adjusted and/or controlled. Herein, in the present invention, the nascent stage means a period of time from the fetal stage to before the weaning stage (less than the weaning stage), whereas during and after the developmental stage means a period of time after the weaning stage, namely, a period of time including the adolescence, maturity and old age stages.

As mentioned above, conventionally, studies regarding the analysis of the function of the transcription factor RP58 have intended to target only the nascent stage. Thus, the analysis of during and after the developmental stage has not been carried out, and the necessity thereof has not been considered. Hence, the transgenic non-human animal capable of artificially and freely adjusting and/or controlling (i.e., capable of increasing or decreasing) the expression of RP58 in a desired period of time not only in the nascent stage but also during and after the developmental stage of the non-human animal is highly useful as a transgenic non-human animal capable of evaluating the function of RP58, which has not previously been known.

Herein, the method and/or means for artificially and freely adjusting and/or controlling (i.e., increasing or decreasing) the expression of RP58 in cells of the central nervous system in a desired period of time are not limited, and for example, various types of known gene recombination and/or gene transfer methods, such as methods using a Tet system, a Cre/loxP system, and an adeno-associated virus vector (AAV vector), can be adopted. Among others, the Tet system is preferable in that it can control the expression level of RP58 reversibly and gradually.

The Tet system is generally a system capable of reversibly regulating the expression of a gene of interest by administration of doxycycline (Dox) as an antibiotic tetracycline derivative. This Tet system utilizes a Tet repressor (TetR) and a Tet operator sequence (tetO sequence) functioning in the *E. coli* tetracycline resistance operon. The Tet system utilizes the properties of the TetR and the tetO sequence, by which TetR binds to the tetO sequence in the absence of Dox (-Dox), but in the presence of Dox (+Dox), Dox binds to the TetR so that the TetR cannot binds to the tetO sequence. In the present invention, in order to freely adjust and/or control (i.e., increase or decrease) the expression of RP58 in a desired period of time, a tetracycline-controlled transcriptional activator (tTA) or a tetracycline-controlled transcriptional silencer (tTS) is preferably used, instead of the above-described Tet repressor (TetR). The tTA is a fusion protein of the TetR and a herpesvirus-derived VP16 transcriptional activity domain (VP16AD). In the absence of Dox (-Dox), the TetR portion binds to the tetO sequence, so as to activate the expression of a gene located downstream thereof. However, in the presence of Dox (+Dox), the TetR portion binds to Dox and the TetR portion cannot bind to the tetO sequence, so that it does not activate the expression of a gene located downstream thereof (i.e., the normal level of expression is maintained). Meanwhile, the tTS is a fusion protein of the TetR and a KRAB silencing domain (SDKid-1). In the absence of Dox (-Dox), the TetR portion binds to the tetO sequence, so that the gene expression is suppressed. However, in the presence of Dox (+Dox), the TetR portion cannot bind to the tetO sequence, and thus, the gene expression cannot be suppressed (i.e., the normal level of expression is maintained). Herein, the promoter operably fused to the tTA gene or the tTS gene is not limited. For example, a Camk2a promoter, an Actin promoter, or the like is preferable. More preferably, the Camk2a promoter is preferably used as a promoter operably fused to the tTA gene, whereas the Actin promoter is preferably used as a promoter operably fused to the tTS gene. Under the control of the Camk2a promoter, the gene can be expressed in mature excitatory neurons. On the other hand, under the control of the Actin promoter, the gene can be expressed in brain neurons as a whole. When the Tet system is adopted and the state in the presence of Dox (+Dox) (in a state in which Dox is administered to the non-human animal) is converted to the state in the absence of Dox (-Dox) (in a state in which Dox is not administered to the non-human animal) in a desired period of time, the expression of RP58 can be increased from the normal level in the case of adopting a transcriptional activator (tTA). On the other hand, in the case of adopting a transcriptional silencer (tTS), the expression of RP58 can be decreased from the normal level.

Moreover, with regard to the method using a Cre/loxP system or the method using an adeno-associated virus vector (AAV vector), these methods can be appropriately utilized to adjust and/or control the expression of RP58, while taking into consideration known procedures and technical contents.

In the present invention, "cells of the central nervous system" are not particularly limited, and examples of the cells of the central nervous system may include all nerve cells and glial cells (e.g., astrocytes, oligodendrocytes, and microglia) present in brain nervous system cells (for example, individual nerves of cerebrum, diencephalon, midbrain and cerebellum, excitatory neurons (presynaptic cells)), and central nervous systems such as medulla oblongata and spinal nerve.

Furthermore, examples of the non-human animal that can be used in the present invention may include mammals other than humans, such as mice, rats, guinea pigs, rabbits, pigs, weasels, dogs, cats, monkeys, sheep, bovines, and horses. Among others, rodents (Rodentia) such as mice, rats and guinea pigs are preferable, and mice are more preferable.

As mentioned above (or as mentioned in the Examples later), the transgenic non-human animal of the present invention shows an aspect in which when the expression of RP58 is decreased (for example, during and after the developmental stage), the cognitive function is reduced dependently on the expression level thereof, and is further approached to age-related cognitive function. Thus, the transgenic non-human animal of the present invention can be used as an animal model of brain dysfunction or a disease related thereto (or a disorder related thereto).

Herein, the brain dysfunction is not limited, and for example, the brain dysfunction is at least one selected from the group consisting of brain tumor, cognitive dysfunction, intellectual disability, developmental disability, memory impairment, and age-related brain disorder (including age-related memory impairment). Herein, the brain tumor is a refractory disease, the frequency of onset of which is increased with aging, such as glioma or glioblastoma. The cognitive dysfunction is a disorder by which cognitive function is reduced. The intellectual disability is a state in which intellectual ability is reduced to an intelligence quotient of less than 70. The developmental disability is a disorder that becomes apparent in the developmental stage, and it includes autism spectrum disorder, attention deficit hyperactivity disorder, and learning disability (the developmental disability may also include intellectual disability in some cases). The memory impairment is a state in which a disorder exists in memory, and it includes dementia, traumatic memory impairment, and dissociative impairment. The age-related brain disorder is a brain disorder caused by aging, and it includes physiological and pathological cases.

Moreover, the disease (or the disorder) related to the above-described brain dysfunction is not limited, and examples of the disease (or the disorder) related to the brain dysfunction may include 1q44 deletion syndrome, Alzheimer's dementia, non-Alzheimer's dementia, cerebrovascular dementia, cognitive impairment due to extrinsic causes, and mixed dementia thereof. Among others, examples of the related disease exhibiting cognitive dysfunction may include diseases classified into neuropsychiatric diseases, neurodegenerative diseases, mitochondrial diseases, refractory epilepsy, endocrine metabolic disorders, infectious diseases, cerebrovascular disorders, neuroimmune diseases, etc. More specific examples of the neuropsychiatric diseases may include schizophrenia, depression, and bipolar disorder. More specific examples of the neurodegenerative diseases may include Parkinson's disease, spinocerebellar degeneration, and progressive supranuclear palsy. More specific examples of the mitochondrial diseases may include mitochondrial encephalomyopathy, lactic acid acidosis, stroke-like attack syndrome (MELAS), chronic progressive external ocular muscle palsy syndrome (CPEO), myoclonus epilepsy associated with ragged-red fibers (MERRF), Lee encephalopathy, and Kearns-Sayre syndrome (KSS). More specific examples of the refractory epilepsy may include West syndrome, Lennox-Gasteau syndrome, and temporal lobe epilepsy. More specific examples of the endocrine metabolic disorders may include diabetes, hypoglycemia, Wernicke-Korsakov syndrome, Pellagra, vitamin B12 deficiency, hypothyroidism, hypothyroidism, distant symptoms of cancer, Cushing syndrome, urinary toxicosis, viscous edema, parathyroid disease, Wilson's disease, and further, amino acid metabolism abnormality, ammonia metabolism abnormality, glucose metabolism abnormality, organic acid metabolism abnormality, fatty acid metabolism abnormality, lipid metabolism abnormality, metal metabolism abnormality, and nucleic acid metabolism abnormality. More specific examples of the infectious diseases may include bacterial meningitis, fungal meningitis, encephalitis, AIDS encephalopathy, and Kreuzfeld-Jakob disease. More specific examples of the cerebrovascular disorders may include arteriosclerosis, cerebral arteriosclerosis, chronic cerebral circulatory insufficiency, cerebral bleeding, cerebral infarction, cerebral embolism, submucosal bleeding, chronic subdural bleeding, pseudosphere paralysis, aortic arch syndrome, Binswanger's disease, and arteriovenous malformation-obstructive thrombotic arteritis. More specific examples of the neuroimmune diseases may include multiple sclerosis and Bechet syndrome. Other examples of the related disease may include normal pressure hydrocephalus, camp syndrome, systemic erythematosus, fever attack, and cardiac arrest.

Furthermore, when the expression of RP58 is increased in the transgenic non-human animal of the present invention (for example, in the nascent stage or during and after the developmental stage), cognitive function is abnormally accelerated dependently on the expression level thereof, and the transgenic non-human animal shows behavioral disorder such as self-injury. As such, the transgenic non-human animal of the present invention can be used as an animal model of behavioral disorder or a disease related thereto (or a disorder related thereto).

The behavioral disorder in the present invention means abnormal behavior provoked by an abnormality of the central nervous system, and it includes self-injury, hypersensitivity, interpersonal relationship disorder, aggression abnormality, motivation abnormality, ego dysfunction, and stress tolerance disorder. Specific examples of the behavioral disorder or a disease related thereto are not limited. The behavioral disorder or a disease related thereto may be, for example, at least one selected from the group consisting of borderline personality disorder, schizophrenia, depression, bipolar disorder, eating disorder, dissociative disorder, post-traumatic stress syndrome, addiction, autism, and self-injury. Herein, the borderline personality disorder is a disease often found in young women, which is characterized by instable intrapersonal relationship, and this disorder often causes self-injury such as wrist cutting. The schizophrenia is a chronic refractory psychiatric disorder that is mainly developed in persons with an age of 15 to 35 years old, and exhibits cognitive dysfunction, and positive and negative symptoms. The depression is a disease having a periodic depressive condition, and it is a mood disorder that causes delusion of sin, delusion of poverty, and delusion of mind, when the disease becomes severe. The bipolar disorder is a mood disorder that periodically exhibits a manic state and a depressive state, and this disease is developed at a young age and may show grandiose delusions when it is in a manic state. The eating disorder is a refractory psychiatric disorder that is characterized by overeating and vomiting and is often developed in young women, and in particular, adolescent thinness involves significant weight loss. The dissociative disorder is an ego disorder that causes memory impairment and personality change, probably due to great stress. The post-traumatic stress syndrome is a chronic stress disorder in which after a lot of extraordinary stress such as war, disaster, and assault, aftereffects such as depression, sleep disorder, and flashback continue for more than one month. The addiction is a disease in which a patient repeats drug use, gambling, and the like, and feels intolerable without them. The autism is a developmental disability that exhibits commitment, interpersonal relationship disorders, oversensitivity, and the like. The self-injury is an action that is not suicide but intentionally hurts oneself, and is assumed to have functions such as elimination of emptiness, living (to not commit suicide), and seeking help from others. Diseases caused by such self-injury account for 1.3% of the world's disability-adjusted life years (DALY) and is ranked 20th (WHO, 2003). In other words, at present, self-injury itself is understood as a disease.

### 3. Screening method

As mentioned above, since the transgenic non-human animal of the present invention can be used as an animal model of brain dysfunction or a disease related thereto, the present transgenic non-human animal is useful for the development of a therapeutic agent against the aforementioned dysfunction or a disease related thereto. Hence, the present invention can provide: a method of screening for a therapeutic or preventive agent against brain dysfunction or a disease related thereto, using the above-described animal model; and a therapeutic or preventive agent against brain dysfunction or a disease related thereto, which is obtained by the aforementioned method. Herein, as specific examples of the brain dysfunction or a disease related thereto, the aforementioned explanations can be applied.

In the present invention, the method of screening for a therapeutic or preventive agent against brain dysfunction or a disease related thereto comprises the following steps (a), (b), and (c):
(a) a step of decreasing the expression of the transcription factor RP58 in cells of the central nervous system of the transgenic non-human animal of the present invention (animal model-producing step);
(b) a step of administering a candidate substance to the above-described non-human animal (animal model) in which the expression of RP58 has been decreased (administration step); and
(c) a step of evaluating the pathological condition of brain dysfunction or a disease related thereto in the above-described non-human animal (animal model) after administration of the candidate substance.

The phrase "to evaluate the pathological condition of brain dysfunction or a disease related thereto" means that the phenotype of the pathological condition regarding the brain dysfunction or a disease related thereto in the transgenic non-human animal after administration of the candidate substance is analyzed. This phase means either to conduct a comparative investigation on the pathological condition of the transgenic non-human animal before and after administration of the candidate substance, or to conduct a comparative investigation on the pathological condition between a test animal administered with the candidate substance and a control animal that has not been administered with the candidate substance.

The method of comparing the phenotype of a test animal with the phenotype of a control animal will be described below. The method is not limited, but examples of the method may include an "object location recognition test" and an "object location recognition + novel object recognition test."

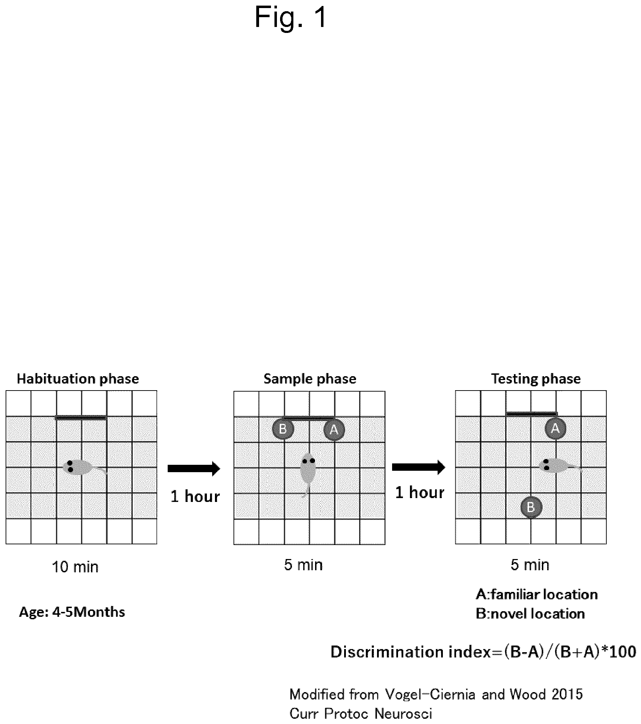

The protocols of the object location recognition test are as shown in Figure 1. This test is an evaluation method that is based on a principle in which a mouse acts to more often come into contact with an object moved to a new location, if the cognitive function of the mouse is normal. Specifically, when two identical objects are placed in a box, a mouse comes into contact with these objects at almost the same level. The mouse is returned to a home cage, and 1 hour later, one object is moved to another location, and the mouse is then placed in the box again. When the mouse has normal cognitive function, the mouse more often comes into contact with the object moved to another location. When the level of the contact is high, the mouse is evaluated to "have accelerated in terms of cognitive function," and when the level of the contact is low, the mouse is evaluated to "have reduced in terms of cognitive function." As the mouse is aged, the mouse rather comes into contact with an object that is not moved. At this time, the mouse is evaluated to have age-related cognitive function.

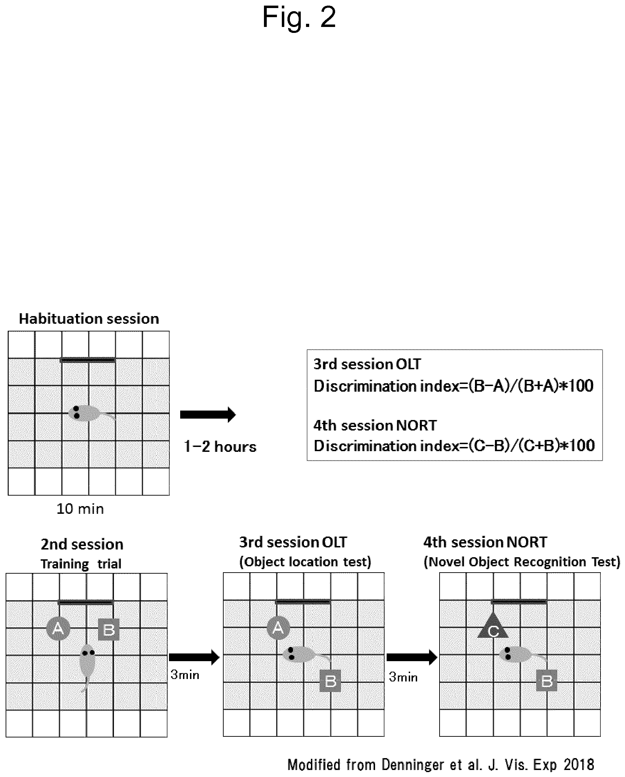

The protocols of the object location recognition + novel object recognition test are as shown in Figure 2. This test is an evaluation method that is based on a principle in which a mouse acts to more often come into contact with an object moved to a new location, if the cognitive function of the mouse is normal, and acts to more often come into contact with a newly placed object. Specifically, when objects A and B are placed in a box, a mouse comes into contact with these objects at almost the same level. The mouse is returned to a home cage, and 1 hour later, the mouse is placed in the box again in a state in which the object B is moved to another location. When the mouse has normal cognitive function, the mouse more often comes into contact with the object B. When the level of the contact is high, the mouse is evaluated to "have accelerated in terms of cognitive function," and when the level of the contact is low, the mouse is evaluated to "have reduced in terms of cognitive function." In addition, the mouse is returned to a home cage, and 1 hour later, the mouse is placed in the box in which an object C is placed instead of the object A. When the mouse has normal cognitive function, the mouse more often comes into contact with the object C. When the level of the contact is high, the mouse is evaluated to "have accelerated in terms of cognitive function," and when the level of the contact is low, the mouse is evaluated to "have reduced in terms of cognitive function."

The "control animal" is not limited, as long as it is suitably used in a comparison with a test subject. The control animal may be the transgenic non-human animal of the present invention, in which the expression of RP58 is not decreased (i.e., in which RP58 is expressed at a normal level), or may also be a non-transgenic non-human animal born of the same mother, or may further be a wild-type animal that is not a transgenic animal. Otherwise, a transgenic non-human animal that is not administered with a candidate substance can also be used as a control animal. A wild-type non-human animal born of the same mother as the non-human animal of the present invention, or a wild-type non-human animal of the same species as the non-human animal of the present invention, is preferable as a control animal in that a precise comparative experiment can be carried out using such a control animal.

It is to be noted that the screening method of the present invention may comprise some other steps, as necessary.

Hereafter, the above-described individual steps will be described.

### (a) Animal model-producing step

An animal model of brain dysfunction or a disease related thereto, in which the expression of the transcription factor RP58 is decreased in cells of the central nervous system of the transgenic non-human animal of the present invention, is produced. A period of decrease in the expression of RP58 is not limited, and it may be carried out in the nascent stage, or during and after the developmental stage, or in a period of time that is a combination of the above two stages. It is preferably carried out, for example, during and after the developmental stage. The method of decreasing the expression of RP58 is not limited, and for example, the aforementioned Tet system or the like can be adopted. In this case, continuous administration of doxycycline (Dox) to a transgenic non-human animal can be carried out orally or parenterally, and the administration route is not limited. In both cases, a known administration method, known administration conditions and the like can be adopted. Even regarding an applied dose, the dose can be determined, as appropriate, while taking into consideration the type and condition of a test animal, the type of a candidate substance, etc.

### (b) Administration step

The candidate substance to be administered to the above-described animal model used as a test animal is not limited. Examples of the candidate substance may include naturally or artificially synthesized, various types of peptides, proteins (including enzymes and antibodies), nucleic acids (polynucleotides (DNA and RNA), oligonucleotides (siRNA, etc.), peptide nucleic acids (PNA), etc.), and low molecular weight, middle molecular weight or high molecular weight organic compounds.

Administration of the candidate substance can be carried out orally or parenterally, and is not limited. In both cases, a known administration method, known administration conditions and the like can be adopted. Even regarding an applied dose, the dose can be determined, as appropriate, while taking into consideration the type and condition of a test animal, the type of a candidate substance, etc.

### (c) Evaluation step

When a therapeutic or preventive agent against brain dysfunction or a disease related thereto is screened, a test animal that has been administered with a candidate substance is preferably compared and evaluated with a control animal that has not been administered with the candidate substance, in terms of various types of evaluation items (the "object location recognition test" or the "object location recognition + novel object recognition test," etc.; see Figures 1 and 2). When the above-described evaluation items in the test animal can be evaluated to be equivalent to or superior to those in the control animal, the candidate substance can be selected as a therapeutic or preventive agent against brain dysfunction or a disease related thereto.

Moreover, as mentioned above, since the transgenic non-human animal of the present invention can also be used as an animal model of behavioral disorder or a disease related thereto, the present transgenic non-human animal is also useful for the development of a therapeutic agent against the aforementioned behavioral disorder or a disease related thereto. Hence, the present invention can provide: a method of screening for a therapeutic or preventive agent against behavioral disorder or a disease related thereto, using the above-described animal model; and a therapeutic or preventive agent against behavioral disorder or a disease related thereto, which is obtained by the aforementioned method. Herein, as specific examples of the behavioral disorder or a disease related thereto, the aforementioned explanations can be applied.

In the present invention, the method of screening for a therapeutic or preventive agent against behavioral disorder or a disease related thereto comprises the following steps (a), (b), and (c):
(a) a step of increasing (accelerating) the expression of the transcription factor RP58 in cells of the central nervous system of the transgenic non-human animal of the present invention (animal model-producing step);
(b) a step of administering a candidate substance to the above-described non-human animal (animal model) in which the expression of RP58 has been increased (accelerated) (administration step); and
(c) a step of evaluating the pathological condition of behavioral disorder or a disease related thereto in the above-described non-human animal (animal model) after administration of the candidate substance.

The phrase "to evaluate the pathological condition of behavioral disorder or a disease related thereto" means that the phenotype of the pathological condition regarding the behavioral disorder or a disease related thereto in the transgenic non-human animal after administration of the candidate substance is analyzed. This phase means either to conduct a comparative investigation on the pathological condition of the transgenic non-human animal before and after administration of the candidate substance, or to conduct a comparative investigation on the pathological condition between a test animal administered with the candidate substance and a control animal that has not been administered with the candidate substance.

The method of comparing the phenotype of a test animal with the phenotype of a control animal is not particularly limited, and a known evaluation method can be adopted, as appropriate, for each of various types of disorders and/or diseases that are classified into behavioral disorder or a disease related thereto. For instance, in the case of self-injury, a method of evaluating items such as excessive grooming and the resulting alopecia, hair loss and skin damage, tail damage due to biting the tail, severe pulling or biting of the chest skin, and weight loss due to anorexia nervosa.

The "control animal" is not limited, as long as it is suitably used in a comparison with a test subject. The control animal may be the transgenic non-human animal of the present invention, in which the expression of RP58 is not increased (accelerated) (i.e., in which RP58 is expressed at a normal level), or may also be a non-transgenic non-human animal born of the same mother, or may further be a wild-type animal that is not a transgenic animal. Otherwise, a transgenic non-human animal that is not administered with a candidate substance can also be used as a control animal. A wild-type non-human animal born of the same mother as the non-human animal of the present invention, or a wild-type non-human animal of the same species as the non-human animal of the present invention, is preferable as a control animal in that a precise comparative experiment can be carried out using such a control animal.

It is to be noted that the screening method of the present invention may comprise some other steps, as necessary.

Hereafter, the above-described individual steps will be described.

### (a) Animal model-producing step

An animal model of behavioral disorder or a disease related thereto, in which the expression of the transcription factor RP58 is increased (accelerated) in cells of the central nervous system of the transgenic non-human animal of the present invention, is produced. A period of increase (acceleration) in the expression of RP58 is not limited, and it may be carried out, for example, in the nascent stage, or during and after the developmental stage, or in a period of time that is a combination of the above two stages. The method of increasing (accelerating) the expression of RP58 is not limited, and for example, the aforementioned Tet system or the like can be adopted. In this case, continuous administration of doxycycline (Dox) to a transgenic non-human animal can be carried out orally or parenterally, and the administration route is not limited. In both cases, a known administration method, known administration conditions and the like can be adopted. Even regarding an applied dose, the dose can be determined, as appropriate, while taking into consideration the type and condition of a test animal, the type of a candidate substance, etc.

### (b) Administration step

The candidate substance to be administered to the above-described animal model used as a test animal is not limited. Examples of the candidate substance may include naturally or artificially synthesized, various types of peptides, proteins (including enzymes and antibodies), nucleic acids (polynucleotides (DNA (antisense DNA, etc.) and RNA), oligonucleotides (siRNA, shRNA, microRNA, etc.), peptide nucleic acids (PNA), etc.), and low molecular weight, middle molecular weight or high molecular weight organic compounds.

Administration of the candidate substance can be carried out orally or parenterally, and is not limited. In both cases, a known administration method, known administration conditions and the like can be adopted. Even regarding an applied dose, the dose can be determined, as appropriate, while taking into consideration the type and condition of a test animal, the type of a candidate substance, etc.

### (c) Evaluation step

When a therapeutic or preventive agent against behavioral disorder or a disease related thereto is screened, a test animal that has been administered with a candidate substance and a control animal that has not been administered with the candidate substance are preferably compared and evaluated, in terms of the conditions and/or pathological conditions of various types of behavioral disorders or diseases related thereto. When the test animal can be evaluated to be equivalent to or superior to the control animal, the candidate substance can be selected as a therapeutic or preventive agent against behavioral disorder or a disease related thereto.

### 4. Pharmaceutical composition

The present invention can provide a pharmaceutical composition for use in the treatment or prevention of brain dysfunction or a disease related thereto, wherein the pharmaceutical composition comprises a transcription factor RP58 protein. In addition, the present invention can also provide a pharmaceutical composition for use in the treatment or prevention of brain dysfunction or a disease related thereto, and a pharmaceutical composition for use in the treatment or prevention of behavioral disorder or a disease related thereto, wherein each pharmaceutical composition comprises a therapeutic or preventive agent obtained by each of the aforementioned screening methods. As specific examples of the brain dysfunction or a disease related thereto and the behavioral disorder or a disease related thereto, the aforementioned explanations can be applied.

Examples of the RP58 protein that is an active ingredient of the above-described pharmaceutical composition or the substance serving as a therapeutic or preventive agent obtained by each of the aforementioned screening methods are not limited to themselves, and for example, their derivatives can also be used instead of or in combination of them. Examples of the derivatives may include, but are not limited to, those assumed to be derivatives based on common general knowledge to persons skilled in the art, such as those having a chemical structure derived from the original protein, compound or the like.

Examples of the derivatives may include those being subjected to metabolism such as oxidation, reduction, hydrolysis, or conjugation *in vivo,* and those that are generated as active ingredients by being subjected to metabolism such as oxidation, reduction, or hydrolysis *in vivo* (so-called prodrugs).

As an active ingredient of the pharmaceutical composition of the present invention, together with the above-described derivatives or prodrugs, or instead of the above-described derivatives or prodrugs, pharmacologically acceptable salts thereof can also be used. Preferred examples of the pharmacologically acceptable salts may include, but are not limited to, hydrohalogenates (e.g., hydrochlorides, hydrobromides, and hydroiodides), inorganic salts (e.g., sulfates, nitrates, perchlorates, phosphates, carbonates, and bicarbonates), organic carboxylates (e.g., acetates, trifluoroacetates, maleates, tartrates, fumarates, and citrates), organic sulfonates (e.g., methane sulfonate, trifluoromethane sulfonate, ethane sulfonate, benzene sulfonate, toluene sulfonate, and camphor sulfonate), amino acid salts (e.g., aspartate and glutamate), quaternary amine salts, alkali metal salts (e.g., sodium salts and potassium salts), and alkaline earth metal salts (e.g., magnesium salts and calcium salts).

Examples of the active ingredient of the pharmaceutical composition of the present invention may also include all isomers possibly generated due to the chemical structure of a compound (for example, geometric isomers, asymmetric carbon-based optical isomers, rotational isomers, stereoisomers, and tautomers), and mixtures of two or more types of these isomers. The active ingredient may be any of an S-form, an R-form, and an RS-form, and is not limited. Furthermore, the active ingredient of the pharmaceutical composition of the present invention may also include those that are present in the form of a hydrate or a solvate, depending on the type thereof. The solvate is not limited, and an example thereof may be a solvate with ethanol.

The amino acid sequence of the RP58 protein that can be used as an active ingredient described above is not limited, and examples thereof may include the amino acid sequences as set forth in SEQ ID NOs: 17, 19, 29, and 31. Examples of the cDNAs encoding these amino acid sequences may include those consisting of the nucleotide sequences as set forth in SEQ ID NOs: 16, 18, 28, and 30.

In the pharmaceutical composition of the present invention, instead of the RP58 protein, or together with the RP58 protein, a protein consisting of an amino acid sequence having an identity of 80% or more to the amino acid sequence of the RP58 protein and having the activity of a transcription factor (i.e., a mutant protein of the RP58 protein) can also be used. Herein, the activity of a transcription factor can be assayed by a method of evaluating transcriptional function, which is called luciferase assay, or an *in vivo* function evaluation method involving *in utero* electroporation. This mutant protein may be more preferably a protein consisting of an amino acid sequence having an identity of 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more, to the amino acid sequence of the RP58 protein, and having the activity of a transcription factor.

In addition, the mutant protein may also be, for example, a protein having an amino acid sequence comprising a deletion, substitution or addition of one or several amino acids with respect to the amino acid sequence of the RP58 protein, and having the activity of a transcription factor. Herein, the "amino acid sequence comprising a deletion, substitution or addition of one or several amino acids" is not particularly limited. For example, it is an amino acid sequence comprising a deletion, substitution or addition of 1 to 15, 1 to 14, 1 to 13, 1 to 12, 1 to 11, 1 to 10, 1 to 9, 1 to 8, 1 to 7, 1 to 6 (1 to several), 1 to 5, 1 to 4, 1 to 3, 1 or 2, or 1 amino acid, but is not limited thereto. In general, the number of amino acids deleted, substituted or added is preferably as small as possible. Introduction of the mutation such as a deletion, substitution or addition can be carried out using a mutation introduction kit that utilizes site-directed mutagenesis, such as, for example, GeneTailor^{™} Site-Directed Mutagenesis System (Invitrogen) or TaKaRa Site-Directed Mutagenesis System (Prime STAR(registered trademark) Mutagenesis Basal kit, Mutan(registered trademark)-Super Express Km, etc. (manufactured by Takara Bio Inc.). Moreover, whether or not the concerned peptide is a peptide, into which the above-described mutation such as a deletion, substitution or addition has been introduced, can be confirmed by various types of amino acid sequence determination methods, structural analysis methods using X-ray and NMR, and the like.

The content percentage of the above-described active ingredient, such as an RP58 protein, in the pharmaceutical composition of the present invention is not limited, and it can be determined, as appropriate. The content percentage of the active ingredient can be set within the range of, for example, 0.01% to 99% by weight, 0.01% to 30% by weight, 0.05% to 20% by weight, or 0.1% to 10% by weight, with respect to the total weight of the pharmaceutical composition. By setting the content percentage of the active ingredient within the above-described range, the pharmaceutical composition of the present invention can exhibit therapeutic or preventive effects against brain dysfunction or a disease related thereto, or therapeutic or preventive effects against behavioral disorder or a disease related thereto.

The pharmaceutical composition of the present invention can be administered to a human or a non-human mammal (e.g., a mouse, a rat, a guinea pig, a weasel, a rabbit, sheep, a pig, a bovine, a cat, a dog, a monkey, etc.) used as a test subject, via various administration routes, specifically, via oral administration or parenteral administration (e.g., intravenous injection (IV), intramuscular injection, intraperitoneal injection, subcutaneous injection, rectal administration, and transdermal administration). The present pharmaceutical composition can be formulated into a suitable dosage form using a pharmaceutically acceptable carrier according to a commonly used method, depending on the administration route.

Examples of the dosage form as an oral agent may include tablets, powders, fine granules, granules, coated tablets, capsules, liquids for internal use, suspensions, emulsions, syrups, and troches. Examples of the dosage form as a parenteral agent may include injections (including infusions), inhalants, ointments, nasal drops, and liposomal agents. Besides, when the therapeutic agent and pharmaceutical composition of the present invention are used as various types of the aforementioned oral agents, the therapeutic agent and the pharmaceutical composition can also be used as supplements (corresponding to, for example, functional foods) in some cases.

Examples of the carrier that can be used in formulation of these preparations may include commonly used excipients, binders, disintegrants, lubricants, colorants, and flavoring agents, and also, as necessary, stabilizers, emulsifiers, absorption enhancers, surfactants, pH adjusters, antiseptics, antioxidants, thickeners, wetting agents, surface activators, dispersants, buffers, preservatives, solubilizers, and soothing agents. Known ingredients that can be used as raw materials for pharmaceutical preparations are mixed with the active ingredient, so that a preparation can be formulated according to an ordinary method.

Examples of nontoxic substances that can be used as the aforementioned ingredients may include: animal and vegetable oils, such as soybean oil, beef fat, and synthetic glyceride; hydrocarbons, such as liquid paraffin, squalane, and solid paraffin; ester oils, such as octyldodecyl myristate and isopropyl myristate; higher alcohols, such as cetostearyl alcohol and behenyl alcohol; silicon resins; silicon oils; surfactants, such as polyoxyethylene fatty acid ester, sorbitan fatty acid ester, glycerin fatty acid ester, polyoxyethylene sorbitan fatty acid ester, polyoxyethylene hydrogenated castor oil, and a polyoxyethylene-polyoxypropylene block copolymer; water-soluble polymers, such as hydroxyethyl cellulose, a polyacrylic acid, carboxyvinyl polymer, polyethylene glycol, polyvinylpyrrolidone, and methyl cellulose; lower alcohols, such as ethanol and isopropanol; polyhydric alcohols (polyols), such as glycerin, propylene glycol, dipropylene glycol, sorbitol, and polyethylene glycol; sugars, such as glucose and sucrose; inorganic powders, such as silicic anhydride, aluminum magnesium silicate, and aluminum silicate; inorganic salts, such as sodium chloride and sodium phosphate; and purified water. All of these substances may also be the salts thereof or the hydrates thereof.

Preferred examples of the excipients may include lactose, fructose, corn starch, white sugar, glucose, mannitol, sorbit, crystalline cellulose, and silicon dioxide. Preferred examples of the binders may include polyvinyl alcohol, polyvinyl ether, methyl cellulose, ethyl cellulose, gum Arabic, tragacanth, gelatin, shellac, hydroxypropylmethyl cellulose, hydroxypropyl cellulose, polyvinylpyrrolidone, a polypropylene glycol-polyoxyethylene block polymer, and meglumine. Preferred examples of the disintegrants may include starch, agar, gelatin powder, crystalline cellulose, calcium carbonate, sodium hydrogen carbonate, calcium citrate, dextrin, pectin, and carboxymethyl cellulose calcium. Preferred examples of the lubricants may include magnesium stearate, talc, polyethylene glycol, silica and hydrogenated vegetable oil. Preferred examples of the colorants may include colorants that have been approved to be added to pharmaceutical products. Preferred examples of the flavoring agents may include cocoa powder, Mentha brain, fragrance powder, mint oil, dragon brain, and cinnamon powder. All of these substances may also be the salts thereof or the hydrates thereof.

Furthermore, the pH adjuster is used in a case where the present pharmaceutical composition is formulated into an injection. The pH adjuster is not particularly limited, as long as it is, for example, a medically, pharmacologically (pharmaceutically) or physiologically acceptable pH adjuster. Specific examples of such a pH adjuster may include: alkali metal hydroxides, such as sodium carbonate, potassium carbonate, sodium ethoxide, potassium butoxide, sodium hydroxide, and potassium hydroxide; alkali metal hydrides, such as sodium hydride and potassium hydride; carbonates of alkali metals or alkaline earth metals; and alkali metal alkoxides. The pH adjuster to be mixed may be used alone or in arbitrary combination of two or more types. When the present pharmaceutical composition is formulated into an injection or the like, it can be appropriately adjusted to an appropriate pH value, using the pH adjuster. In the present invention, the pH value is preferably pH 3 to 6, more preferably pH 3 to 5, further preferably pH 3 to 4.5, and particularly preferably pH 3.5 to 4.5.

Besides, when the present pharmaceutical composition is formulated into an injection, the injection may contain cyclodextrins. Such cyclodextrins are not particularly limited, as long as they are medically, pharmacologically (pharmaceutically) or physiologically acceptable. Examples of such cyclodextrins may include unmodified cyclodextrin and modified cyclodextrin. Examples of the unmodified cyclodextrin may include α-cyclodextrin, β-cyclodextrin, and γ-cyclodextrin. Examples of the modified cyclodextrin may include alkylated cyclodextrin (for example, dimethyl-α-cyclodextrin, dimethyl-P-cyclodextrin, dimethyl-γ-cyclodextrin, etc.), hydroxyalkylated cyclodextrin (for example, hydroxypropyl-α-cyclodextrin, hydroxypropyl-P-cyclodextrin, hydroxypropyl-γ-cyclodextrin, etc.), sulfoalkyl ether cyclodextrin (for example, sulfobutyl ether-α-cyclodextrin, sulfobutyl ether-P-cyclodextrin, sulfobutyl ether-γ-cyclodextrin, etc.), and branched cyclodextrin (for example, maltosyl-α-cyclodextrin, maltosyl-β-cyclodextrin, maltosyl-γ-cyclodextrin, etc.).

Cyclodextrins may be used alone or in arbitrary combination of two or more types. The cyclodextrin to be mixed is preferably hydroxypropyl-P-cyclodextrin or sulfobutyl ether-P-cyclodextrin, and is more preferably sulfobutyl ether-P-cyclodextrin.

The content of such cyclodextrins is not particularly limited, and it is determined, as appropriate, depending on the type thereof, the intended use of an injection, usage, etc. The content of cyclodextrins is preferably 5% to 50% by weight, more preferably 10% to 40% by weight, and further preferably 10% to 30% by weight, based on the total amount of the injection.

In general, in consideration of the ratio of the active ingredient mixed into the preparation, the dose of the pharmaceutical composition of the present invention can be appropriately determined in a wide range, while taking into consideration the age and body weight of an administration target (patient), the type of disease and/or progression status, an administration route, the number of doses (/day), an administration period, etc.

The case of using the pharmaceutical composition of the present invention as a parenteral agent or an oral agent will be specifically described below.

In the case of using the present pharmaceutical composition as a parenteral agent, in general, the dosage form thereof is not limited. In the case of various types of injections, they may be provided, for example, in the state of a unit dose ampoule or a high dose container, or in the state of a lyophilized powder that is to be redissolved in a solution at the time of use. The parenteral agent contains the RP58 protein serving as an active ingredient, and depending on various types of forms, may also contain various types of known excipients or additives in a range that does not impair the effects of the above-described active ingredient. For example, in the case of various types of injections, water, glycerol, propylene glycol, aliphatic polyalcohols such as polyethylene glycol, and the like may be used.

The dose (per day) of the parenteral agent is not limited. For example, in the case of various types of injections, in general, the RP58 protein serving as an active ingredient, etc. can be administered to an application target (a subject, a patient, etc.) at a dose of 0.01 to 1000 mg, 0.05 to 500 mg, or 0.1 to 50 mg per kg of body weight. Otherwise, the dose can be set to be 0.5 to 20 mg, or 1 to 10 mg.

In the case of using as an oral agent, in general, the form thereof is not limited, and any of the aforementioned dosage forms may be applied, or it may also be a dried product that is to be redissolved at the time of use. The oral agent contains the RP58 protein serving as an active ingredient, and depending on various types of forms, may also contain various types of known excipients or additives in a range that does not impair the effects of the above-described active ingredient. Examples of the excipients or additives may include binders (syrup, gum Arabic, gelatin, sorbitol, tragacanth, polyvinylpyrrolidone, etc.), fillers (lactose, sugar, corn starch, potato starch, calcium phosphate, sorbitol, glycine, etc.), lubricants (magnesium stearate, talc, polyethylene glycol, silica, etc.), disintegrants (various types of starches, etc.), and wetting agents (sodium lauryl sulfate, etc.).

With regard to the dose (per day) of the oral agent, in general, the RP58 protein serving as an active ingredient, etc. can be administered to an application target (a subject, a patient, etc.) at a dose of 0.05 to 5000 mg, 0.1 to 1000 mg, or 0.1 to 100 mg per kg of body weight. Otherwise, the dose can be set to be 0.5 to 50 mg, or 1 to 10 mg. The ratio of the active ingredient mixed into the oral agent is not limited, and can be appropriately determined, while taking into consideration the number of doses per day, etc.

Further, the present invention can also provide a pharmaceutical composition for use in the treatment or prevention of brain dysfunction or a disease related thereto, wherein the pharmaceutical composition comprises a nucleic acid encoding the transcription factor RP58, what is called, a gene therapy agent (hereinafter referred to as "the gene therapy agent of the present invention").

The nucleic acid used in the gene therapy agent of the present invention is not limited, and it may be any of DNA (chromosomal DNA, plasmid DNA, etc.), RNA (mRNA, etc.), peptide nucleic acid (PNA), and oligonucleotide (siRNA, etc.). It may also be a hybrid of any two or more of these.

Specific examples of the nucleic acid encoding the RP58 protein may preferably include, but are not limited to, the following DNAs.

(a1) DNA comprising the nucleotide sequence as set forth in SEQ ID NO: 1.
(b1) RNA consisting of the nucleotide sequence as set forth in SEQ ID NO: 11, 12, 13, 14, or 15.
(c1) DNA consisting of the nucleotide sequence as set forth in SEQ ID NO: 16 or 18.
(d1) DNA or RNA having an identity of 80% or more to the DNA or RNA consisting of the nucleotide sequence having any sequence number described in the above (a1) to (c1), and encoding a protein having the activity of a transcription factor.
(a2) DNA comprising the nucleotide sequence as set forth in SEQ ID NO: 6.
(b2) RNA consisting of the nucleotide sequence as set forth in SEQ ID NO: 20, 21, 22, 23, 24, 25, 26, or 27.
(c2) DNA consisting of the nucleotide sequence as set forth in SEQ ID NO: 28 or 30.
(d2) DNA or RNA having an identity of 80% or more to the DNA or RNA consisting of the nucleotide sequence having any sequence number described in the above (a2) to (c2), and encoding a protein having the activity of a transcription factor.

The DNA described in each of the above (a1) and (a2) is not limited, and may also be DNA consisting of the nucleotide sequence as set forth in SEQ ID NO: 1 or 6.

The DNA or RNA described in each of the above (d1) and (d2) corresponds to a mutant of the DNA or RNA described in each of the above (a1) to (c1) and (a2) to (c2). Such mutant DNA can be prepared according to the site-directed mutagenesis described, for example, in Molecular cloning 4th Edt. Cold Spring Harbor Laboratory Press (2012), etc. Specifically, such mutant DNA can be prepared according to a known method such as a Kunkel method or a Gapped duplex method, using a mutation introduction kit that utilizes site-directed mutagenesis. Preferred examples of the mutation introduction kit may include QuickChange^{™} Site-Directed Mutagenesis Kit (manufactured by Stratagene), GeneTailor^{™} Site-Directed Mutagenesis System (manufactured by Invitrogen), and PrimeSTAR(registered trademark) Mutagenesis Basal Kit (manufactured by Takara Bio, Inc.). Alternatively, such mutant DNA, etc. can also be prepared by using the DNA, etc. described in each of the above (a1) to (c1) and (a2) to (c2) as a template, and performing PCR under suitable conditions, employing PCR primers designed to introduce a missense mutation to obtain nucleotides showing a desired amino acid codon. The DNA polymerase used in PCR is not limited, and it is preferably highly precise DNA polymerase. Preferred examples of such DNA polymerase may include Pwo DNA polymerase (Roche Diagnostics), Pfu DNA polymerase (Promega), Platinum Pfx DNA polymerase (Invitrogen), and KOD-plus-polymerase (Toyobo Co., Ltd.) The PCR reaction conditions may be appropriately determined depending on the optimal temperature of the DNA polymerase used, the length and type of the DNA to be synthesized, and the like.

The mutant DNA or RNA described in each of the above (d1) and (d2) can be obtained by performing known hybridization methods such as colony hybridization, plaque hybridization and Southern blot, using, as a probe, the DNA or RNA described in each of the above (a1) to (c1) and (a2) to (c2), or DNA or RNA consisting of a nucleotide sequence complementary thereto, or a fragment thereof, and then obtaining it from a cDNA library or a genomic library. As such a library, a library produced by a known method may be utilized, or a commercially available cDNA library or genomic library may also be utilized, and thus, the library is not limited. Regarding detailed procedures of the hybridization method, Molecular cloning 4th Edt. Cold Spring Harbor Laboratory Press (2012), etc. can be referred, as appropriate.

As described above, the mutant DNA or RNA of each of the above-described (d1) and (d2) preferably consists of a nucleotide sequence having an identity of at least 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more, to the nucleotide sequence of the DNA or RNA of each of the above-described (a1) to (c1) and (a2) to (c2).

The above-described mutant DNA or RNA is DNA encoding a protein having the activity of a transcription factor. Herein, the activity of a transcription factor can also be assayed by a method of evaluating transcriptional function, which is called luciferase assay, or an *in vivo* function evaluation method involving *in utero* electroporation.

The gene therapy agent of the present invention may comprise other ingredients other than the above-described DNA or RNA. Examples of such other ingredients may include various types of pharmaceutical ingredients (various types of pharmaceutically acceptable carriers, etc.) that are required depending on the usage (use form) of the gene therapy agent of the present invention. Examples of such pharmaceutical ingredients may include excipients, fillers, thickeners, binders, wetting agents, disintegrants, lubricants, surfactants, dispersants, buffers, preservatives, solubilizers, antiseptics, soothing agents, stabilizers, and tonicity agents, which are commonly used in drug manufacturing.

With regard to administration of the gene therapy agent of the present invention, administration methods include a method of directly administering the gene therapy agent of the present invention by injection and a method of administering a vector into which a gene comprising the above-described DNA has been incorporated. Regarding the above-described vector, various types of known plasmid vectors that can be used as expression vectors, or viral vectors such as an adenovirus vector, an adeno-associated virus vector, a herpesvirus vector, a vaccinia virus vector, a retrovirus vector and a lentivirus vector are used for efficient administration. In addition, commercially available gene transfer kits (for example, product name: AdenoExpress, manufactured by Clontech) can also be used. The present invention also includes a vector (recombinant vector) in which a gene containing a nucleic acid (DNA, etc.) encoding the fusion protein has been incorporated. Furthermore, the present invention also includes a transformant that can be obtained by introducing the aforementioned recombinant vector into a host, so that a gene of interest can be expressed therein. The host is not limited, as long as it can express the gene DNA of the present invention, and examples of the host that can be used herein may include bacteria, yeasts, and animal cells, which are well known in the present technical field.

In general, in consideration of the ratio of the active ingredient mixed into the preparation, the dose of the gene therapy agent of the present invention can be appropriately determined in a wide range, while taking into consideration the age and body weight of an administration target (patient), the type of disease and/or pathological conditions, an administration route, the number of doses, an administration period, etc. For example, when the gene therapy agent of the present invention is administered to an adult patient, the dose of the present gene therapy agent is not limited, but it is preferably approximately 0.1 µg/kg to 1000 mg/kg per body weight of the patient per day, and more preferably approximately 1 µg/kg to 100 mg/kg per body weight of the patient per day.

Besides, the present invention also includes: (i) use of the pharmaceutical composition of the present invention or the gene therapeutic agent of the present invention, and specifically, for example, a method for treating or preventing brain dysfunction or a disease related thereto, or behavioral disorder or a disease related thereto, wherein the method comprises administering an effective amount of the pharmaceutical composition of the present invention or the gene therapeutic agent of the present invention to a test subject (a patient who has or is likely to have brain dysfunction or a disease related thereto or behavioral disorder or a disease related thereto, or such a non-human mammal (e.g., a mouse, a rat, a guinea pig, a rabbit, sheep, a pig, a bovine, a weasel, a cat, a dog, a monkey, etc.); (ii) use of the pharmaceutical composition of the present invention or the gene therapy agent of the present invention for production of a drug for treating or preventing brain dysfunction or a disease related thereto or behavioral disorder or a disease related thereto; and (iii) use of the pharmaceutical composition of the present invention or the gene therapy agent of the present invention for the treatment or prevention of brain dysfunction or a disease related thereto or behavioral disorder or a disease related thereto. In the present invention, specific examples of the treatment of brain dysfunction or a disease related thereto or behavioral disorder or a disease related thereto may include suppression of the progression of the disorder or the disease, the improvement of the prognosis, and/or prevention of the recurrence.

### 5. Kit

Upon the treatment or prevention of brain dysfunction or a disease related thereto or behavioral disorder or a disease related thereto, a kit including the aforementioned pharmaceutical composition of the present invention can be used.

The form of the active ingredient contained in the pharmaceutical composition included in the kit is not limited, and taking into consideration stability (preservation property), the ease of use, and the like, the active ingredient may be prepared in a dissolved state.

The kit can comprise other constitutional elements, as appropriate, as well as the pharmaceutical composition of the present invention.

6. Screening method as another aspect, etc.

The present invention can also provide a screening method as described in (a) or (b) below (i.e., a method of screening for a substance or an environment that improves or reduces cognitive function), in addition to the screening method explained in the above section 3, etc.
(a) A method of screening for a substance that improves or reduces cognitive function, which comprises administering a candidate substance to a wild-type non-human animal, or allowing a candidate substance to come into contact with wild-type animal cells, and then screening the substance that improves or reduces cognitive function, using, as an indicator, the expression level of the transcription factor RP58 in the brain of the non-human animal after the administration or in the animal cells after the contact.
(b) A method of screening for an environment that improves or reduces cognitive function, which comprises breeding a wild-type non-human animal under a candidate environment, or culturing wild-type animal cells under a candidate environment, and then screening the environment that improves or reduces cognitive function, using, as an indicator, the expression level of the transcription factor RP58 in the brain of the non-human animal after the breeding or in the animal cells after the culture.

As described in the Examples of the present application later, when the expression level of the transcription factor RP58 was increased in the neurons of a wild-type non-human animal, the improvement of cognitive function was observed. From these results, a substance or an environmental factor that can increase or decrease the expression level of RP58 may become a substance or an environmental factor that improves or decreases cognitive function. The screening method of the above (a) or (b) was discovered based on these findings, etc.

Examples of the non-human animal that can be used in the methods of the above (a) and (b) may include mammals other than humans, such as a mouse, a rat, a guinea pig, a rabbit, a pig, a weasel, a dog, a cat, a monkey, sheep, a bovine, and a horse. Among these, rodent animals (Rodentia) such as a mouse, a rat and a guinea pig are preferable, and a mouse is more preferable.

Moreover, examples of the animal cells that can be used in the methods of the above (a) and (b) may include cells derived from the above-described non-human animals and human-derived cells. The types of the cells are not limited, and cells of the central nervous system are preferable. Examples of the cells of the central nervous system may include, but are not particularly limited, brain nerve (for example, each nerve of cerebrum, diencephalon, midbrain or cerebellum, and excitatory neurons (presynaptic cells)) system cells, all neurons existing in the central nervous system such as medullary nerve and spinal cord nerve, and glial cells (for example, astrocytes, oligodendrocytes, and microglia).

In the method of the above (a), the above-described candidate substance to be administered or contacted is not limited, and examples of the candidate substance may include naturally or artificially synthesized, various types of peptides, proteins (including enzymes and antibodies), nucleic acids (polynucleotides (DNA (antisense DNA, etc.) and RNA), oligonucleotides (siRNA, shRNA, microRNA, etc.), peptide nucleic acids (PNA), etc.), and low molecular weight, middle molecular weight or high molecular weight organic compounds. Furthermore, various types of drugs or various types of foods, which contain or do not contain the above-exemplified substances, can also be exemplified as candidate substances.

Administration of the candidate substance to a non-human animal can be carried out orally or parenterally, and is not limited. In both cases, a known administration method, known administration conditions and the like can be adopted. Even regarding the applied dose of the candidate substance, the dose can be determined, as appropriate, while taking into consideration the type and condition of a test animal, the type of the candidate substance, etc.

Further, with regard to the contact of the candidate substance with animal cells, a method of allowing the candidate substance to come into contact with the cells or to be absorbed by the cells, such as addition of the candidate substance into a medium or a culture solution containing the cells, or conditions for allowing the candidate substance to come into contact with the cells or to be absorbed by the cells, or the like can be adopted, as appropriate. Also, the amount of the candidate substance used to come into contact with the cells can be determined, as appropriate, taking into consideration the types and conditions of animal cells, the type of the candidate substance, etc.

In the method of the above (b), the above-described candidate environment applied upon the breeding or the culture is not limited, and examples of the candidate environment may include a light environment (the wavelength and/or intensity of an illumination light, etc.), a sound environment (the wavelength and/or intensity of an environmental sound, etc.), a thermal environment (temperature), a gas layer environment (humidity, gas ratio, wind velocity, and air volume), a liquid phase environment (the type and/or concentration of a solute and/or a solvent, gas ratio, flow velocity, flow rate, and viscosity), a solid phase environment (the material, quality, hardness, texture, unevenness, color tone, shape, and size of a cage, a floor, incubation equipment, etc.), a gravity environment (gravity, rotation/stirring, vibration, and acceleration), an electromagnetic field (electric field strength and/or electric flux density, magnetic field strength and/or magnetic flux density, and electromagnetic potential), electromagnetic waves (the wavelength and/or intensity of radio waves, infrared rays, visible rays, ultraviolet rays, X-rays, etc.) and/or radiation (particle radiation such as alpha rays, beta rays, neutron rays, proton rays, heavy ion rays, mesonic rays, etc.), social factors (the number, density, type, gender and age of cells, individuals, fakes, etc. that may cause competition, coexistence, or communication), the content of feed as a nutritional environment (ketone diet, high cholesterol diet, high carbohydrate diet, high fat diet, high vitamin diet, excessive sucrose, etc.), the content of drinking water (addition of vitamin, addition of catechins, anti-inflammatory agent, addition of antibiotic, alcohol, sugar, salt, etc.), administration intervals of feed or drinking water, a stress environment (mother-child separation in various periods from immediately after birth to weaning stage, foster parents, isolated breeding, restraint, overcrowding, mixed breeding with different strains or other species in siblings, new cage movement, insufficient bedding, sleep deprivation, etc.), a rich (excellent) environment (an optimal number of breeding, various toys (turntable, tunnel, etc.), a hiding place, gnawing stick, a sufficient amount of bedding, a nesting material, etc.), the presence or absence of handling and the amount thereof, the presence or absence of administration of drugs (narcotics, stimulants, anti-inflammatory agents, and antibiotics), behavioral experiments, a feeding environment (artificial milk and breast milk), a combination of the above-described candidate environments (complexity of patterns), and environmental factors such as spatiotemporal changes in the candidate environment (frequency and/or the number of repetitions). In addition, applicable conditions for each environmental factor can be appropriately determined, while taking into consideration the types of non-human animals or animal cells, the types of the environmental factors, and the like.

When compared with a non-human animal and non-human animal cells before the use of a candidate substance or application of a candidate environment, or when compared with a non-human animal and non-human animal cells used as controls, to which a candidate substance has not been used or a candidate environment has not been applied, if the expression level of the transcription factor RP58 is significantly increased in the non-human animal and non-human animal cells after the use of the candidate substance or application of the candidate environment, the candidate substance or the candidate environment can be evaluated to be a substance or an environment that improves cognitive function. On the other hand, if the expression level of the transcription factor RP58 is significantly decreased in the non-human animal and non-human animal cells after the use of the candidate substance or application of the candidate environment, the candidate substance or the candidate environment can be evaluated to be a substance or an environment that reduces cognitive function. With regard to the expression level of RP58 in a non-human animal, the expression level of RP58 in brain (in brain cells) may be measured according to various types of conventionally known methods. With regard to the expression level of RP58 in non-human animal cells, the expression level of RP58 in the non-human animal cells may be measured according to various types of conventionally known methods.

The substance or environment improving cognitive function, which can be screened by the methods of the above (a) and (b), can also be used as, for example, those capable of treating or preventing brain dysfunction or a disease related thereto (or a disorder related thereto), as explained in the above section 2. Moreover, the substance or environment reducing cognitive function, which can be screened by the same methods as those mentioned above, can also be used as, for example, those capable of treating or preventing behavioral disorder or a disease related thereto (or a disorder related thereto), as explained in the above section 2.

Accordingly, the present invention can also provide: an agent for improving or reducing cognitive function, comprising the substance obtained by the screening method of the above (a); and further, a method for improving or reducing cognitive function, comprising administering the aforementioned agent for improving or reducing cognitive function to a subject. Besides, for the above-described agent for improving or reducing cognitive function, the explanation of the above section 4. ("Pharmaceutical composition") can be partially applied, as appropriate.

Moreover, the present invention can also provide a method for improving or reducing cognitive function, comprising providing the environment screened by the method of the above (b) to a subject.

The subject is not particularly limited. The subject (or patient) who wants to improve cognitive function or wants to reduce cognitive function can be arbitrarily adopted. In such a case, a patient who has or is likely to have brain dysfunction or a disease related thereto (or a disorder related thereto), or a patient who has or is likely to have behavioral disorder or a disease related thereto (or a disorder related thereto) can also be used as a subj ect.

Hereinafter, the present invention will be more specifically described in the following examples. However, the present invention is not limited to these examples.

### [Example 1]

### [Method]

### < Normal control mice >

Wild-type B6 male mice with an age of 4 months old (4 mice), 10 months old (3 mice), and 19 months old (4 mice) were bred under an ordinary environment, and were then used in behavioral experiments.

### < Production of transgenic mice (RP58 expression-controlled mouse models) >

FAST system (Tanaka et al., Biol Psychiatry, 2010) was used. Specifically, first, two types of mice (B6 mice) were produced depending on a difference in the introduction (insertion) site of a loxP-FRT-Neo-STOP-FRT-TetO-loxP sequence (hereinafter referred to as a "STOP sequence"; SEQ ID NO: 32), in which the loxP-FRT-Neo-STOP-FRT-TetO-loxP sequence was introduced into a site located upstream of the translation initiation site of RP58 according to a genetic recombination technology (VI: immediately before the translation initiation site (ATG) of transcription product variant 1; and V2: approximately 140 bp before the translation initiation site (ATG) of transcription product variant 2). Specifically, Exons 1 to 4 (SEQ ID NOs: 7 to 10, successively from Exon 1) are present in the genomic DNA (SEQ ID NO: 6) of mouse RP58. In the case of the above-described V1 mouse, the STOP sequence (which is actually a sequence comprising nucleotides further added to the 5'- and 3'-termini of the STOP sequence (SEQ ID NO: 33)) is inserted into a site directly above the start codon (ATG) in Exon 2 (SEQ ID NO: 8) (i.e., between the 149th nucleotide (T) and the 150th nucleotide (A) of Exon 2). On the other hand, in the above-described V2 mouse, the STOP sequence (which is actually a sequence comprising nucleotides further added to the 5'- and 3'-termini of the STOP sequence (SEQ ID NO: 34)) is inserted into a site located 132 nucleotides upstream of the start codon (ATG) in Exon 3 (SEQ ID NO: 9) (i.e., between the 1130th nucleotide (G) and the 1131st nucleotide (C) of Exon 3). The thus produced two types of mice (VI and V2) were each mated with Flipases expression mice to produce TetO mice from which the Stop sequence was removed. Furthermore, transsilencer (tTS) expression mice or transactivator (tTA) expression mice were mated with these mice, so as to produce RP58 expression-controlled mouse models (a total of 4 types). As a tTS promoter for RP58 expression decrease models, an Actin promoter was used. As a tTA promoter for expression increase models, a Camk2a promoter was used. In the RP58 expression decrease mouse models, tTS is expressed in almost all cells including brain neurons as a whole. In the RP58 expression increase mouse models, tTA is expressed in mature excitatory neurons. These tTS and tTA bind to tetO in the absence of doxycycline (Dox), so as to cause a decrease or increase in the expression of RP58. In contrast, in the presence of Dox, the tTS and tTA do not bind to tetO, and each exhibit a wild-type phenotype (i.e., a normal expression level of RP58).

As expression decrease models, ActintTS::tetO Rp58 homo and hetero mice were produced, and Dox was then administered thereto until the 21st day after birth (P21; weaning stage), so that the expression level of RP58 was maintained at a normal level. From the 21st day after birth, administration of Dox was suspended, so that tTS was allowed to bind to tetO, and the expression of RP58 was thereby decreased. The same applied to the mice on the 60th day after birth (P60) and thereafter.

As expression increase models, Camk2tTA::tetO Rp58 hetero mice were produced, and then, without administration of doxycycline (Dox), the expression of Rp58 in mature excitatory neurons was increased from the early nascent stage.

### < Quantification of cognitive function by behavioral test >

4-Month-old, 10-month-old, and 19-month old mice were used as normal control mice. 4- to 5-Month old mice were used as expression decrease mouse models. 4-Month-old and 15-month old mice were used as expression increase mouse models. With reference to the method of Vogel-Ciernia and Wood et al., an "object location recognition test (1 hour memory)" was carried out. Each mouse was habituated in an open field box for 10 minutes. One hour later, two objects having an identical shape were placed in parallel, and the mice were each allowed to search freely for 5 minutes. One hour later, one object was moved to a novel position, and the mice were then allowed to search freely for 5 minutes. The time in which the mice contacted with the novel location object (B) and the familiar location object (A) was calculated with Discrimination Index = (B-A)/(B+A)^{∗} 100. A plus value indicates that the contact time with the novel location object is long, whereas a minus value indicates that the contact time with the familiar location object is long. The protocols of this object location recognition test are shown in Figure 1.

Regarding expression increase models, with reference to the method of Denninger et al., a continuous evaluation was carried out also on 10-month old mice according to an "object location recognition + novel object recognition test (3 minutes memory)." Each mouse was habituated in an open field box for 10 minutes. One hour later, objects (A) and (B) each having a different shape were placed in parallel, and the mice were each allowed to search freely for 5 minutes. Three minutes later, the object (B) was moved to a novel location, and the mice were then allowed to search freely for 5 minutes. Further, three minutes later, the familiar object (A) was replaced with a novel object (C), and the mice were then allowed to search freely for 5 minutes. The time in which the mice contacted with the novel location object (B) and the familiar location object (A) was calculated with Discrimination Index = (B-A)/(B+A)^{∗}100. The time in which the mice contacted with the novel object (C) and the novel location object (B) was calculated with Discrimination Index = (C-B)/(C+B)^{∗}100. A plus value indicates that the contact time with the novel location object or the novel object is long, whereas a minus value indicates that the contact time with the familiar location object or the familiar object is long. The protocols of this object location recognition + novel object recognition test are shown in Figure 2.

### < Production of adeno-associated virus and administration method to mice >

(i) Production of plasmid into which gene of interest is inserted
   pAAV-CaMKIIa-eNpHR3.0-EYFP-WPRE (Plasmid #26971, Addgene) was cleaved with the restriction enzymes BamH1/EcoR1, so that eNpHR 3.0-EYFP was removed therefrom, and cMyc-RP58 or GFP was then inserted into the plasmid, so as to produce pAAV-CaMKIIa-cMyc-RP58-WPRE and pAAV-CaMKIIa-GFP-WPRE.
(ii) Production of adeno-associated virus (AAV) vector
   Three types of plasmid vectors, namely, a plasmid into which a gene of interest had been inserted, an AAV helper plasmid, and an adenovirus helper plasmid were transfected into 293 cells, so as to produce adeno-associated virus vectors with the serotype PHB, comprising the gene of interest therein (AAV-CaMKIIa-cMyc-RP58 and AAV-CaMKIIa-GFP).
(iii) AAV administration method

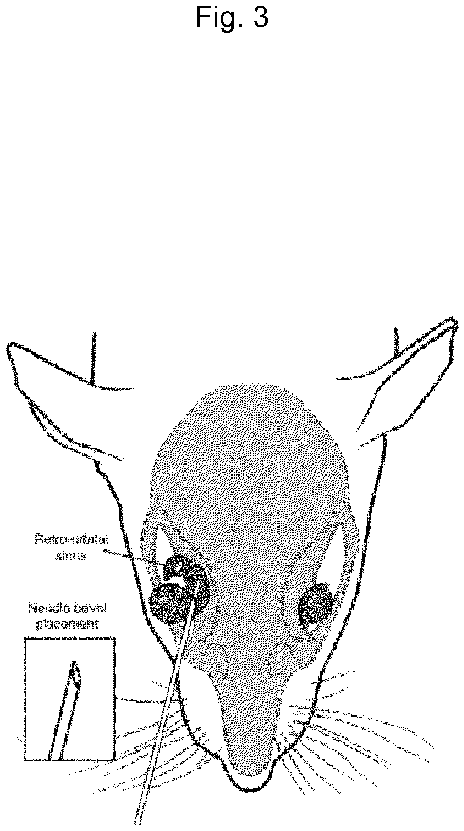

To the orbital sinus of 3-month-old normal control mice and human-type mutation APP homo mice (AD mouse models), a purified adeno-associated virus vector 5 x 10¹² v.g. (vector genomes) with high purity, together with Evans blue (approximately 1%), was administered using a normal saline as a solvent (Figure 3; the above-mentioned Non Patent Literature 20: Tal et al. Lab Anim (NY). 2011).

### < Tissue analysis >

The mice were subjected to perfusion fixation with 4% paraformaldehyde under isoflurane anesthesia. Thereafter, the mice were subj ected to post-fixation overnight, and were then subjected to substitution with 20% sucrose and cryostat, so as to produce a frozen section (with a thickness of 50 µm) of the mouse brain tissues. The section was suspended in a phosphate buffered saline, and was then preserved at 4°C. Fluorescent antibody staining was carried out using RP58, cMyc, NeuN, GFP, the amyloid β marker 82E1, and the microglia markers Iba1 and CD68.

### [Results and Consideration]

### (1) Behavior analysis

### < Normal control mice >

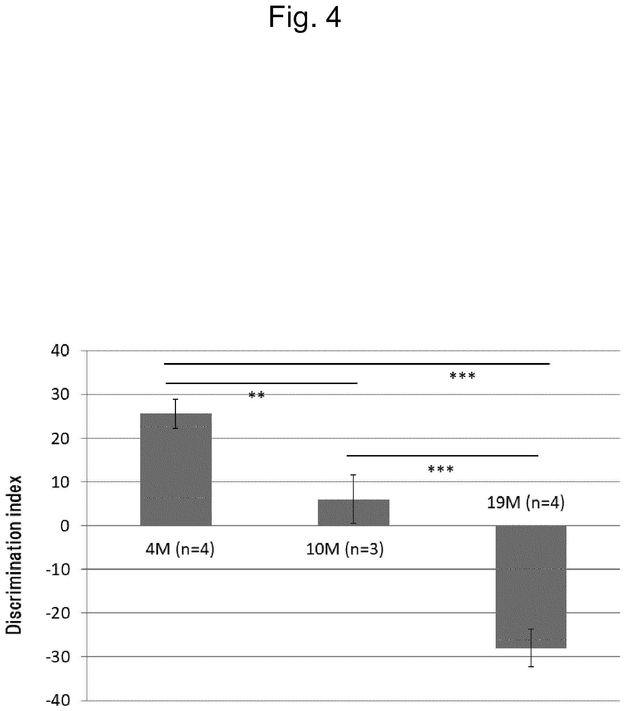

With regard to the value of the discrimination index for evaluation of cognitive function, the value of 4-month-old mice was 25.6 (n = 4), the value of 10-month-old mice was 6.1 (n = 3), and the value of 19-month-old mice was -28.0 (n = 4). The discrimination index was considered to quantitatively show cognitive function, and the discrimination index decreased with aging. It was considered that these results show a reduction in cognitive function due to aging (Figure 4).

### < Expression decrease models >

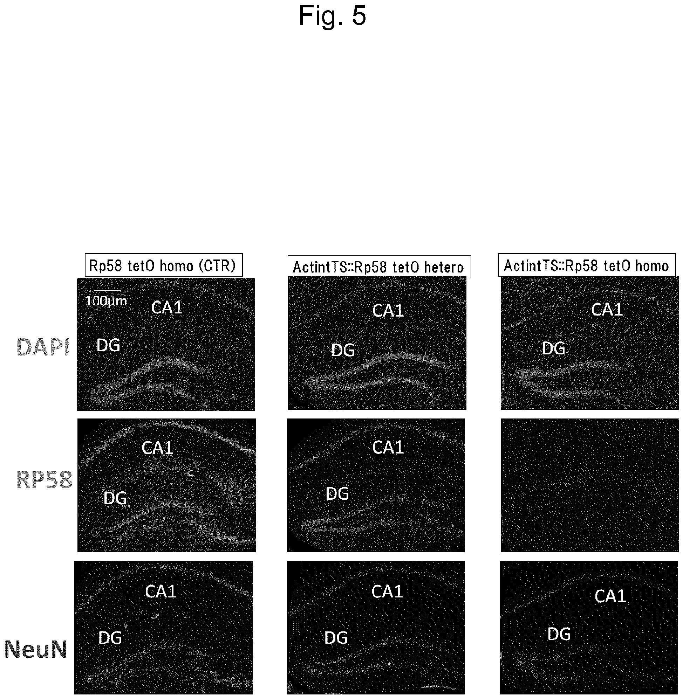

The results obtained by staining ActintTS:: Rp58 tetO homo or hetero mice (5-month-old), in which the expression of RP58 was decreased after P21, with RP58, NeuN, and DAPI are shown (Figure 5). As control samples, Rp58 tetO homo mice were used. The expression of RP58 was successfully, artificially suppressed.

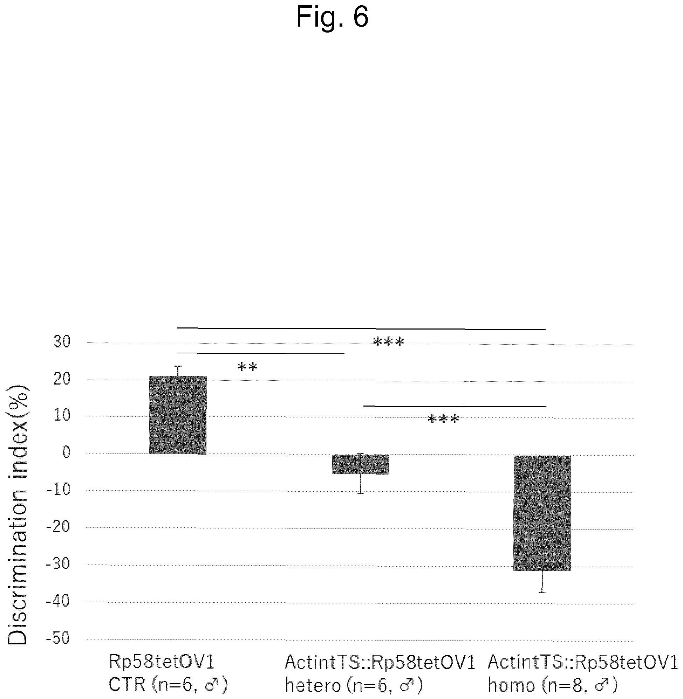

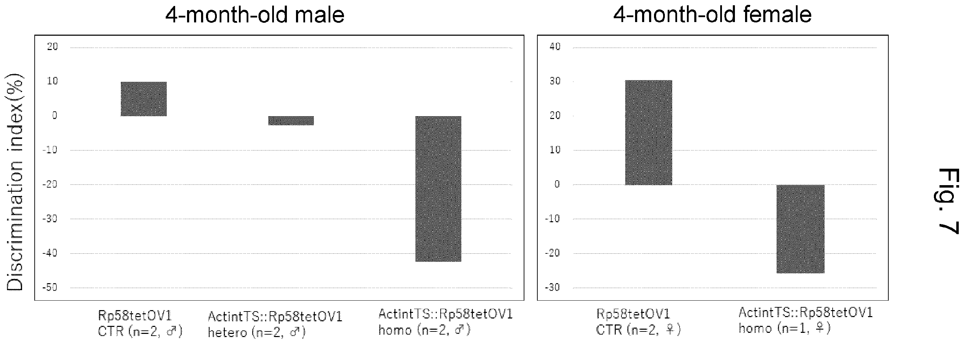

With regard to ActintTS::tetO (VI) Rp58 homo and hetero mice, in which the expression of RP58 had been decreased after P21 and P60, a reduction in cognitive function was found according to an object location recognition test performed on the 4-to 5-month-old mice. A change from the normal level was larger in the homo mice in which a decrease in the expression of RP58 was large, than in the hetero mice, and thus, it was found that cognitive function is controlled dependently on the amount of RP58 (Figures 6 and 7).

Therefore, it was demonstrated that a decrease in the expression level of RP58 provokes a reduction in cognitive function.

### < Expression increase models >

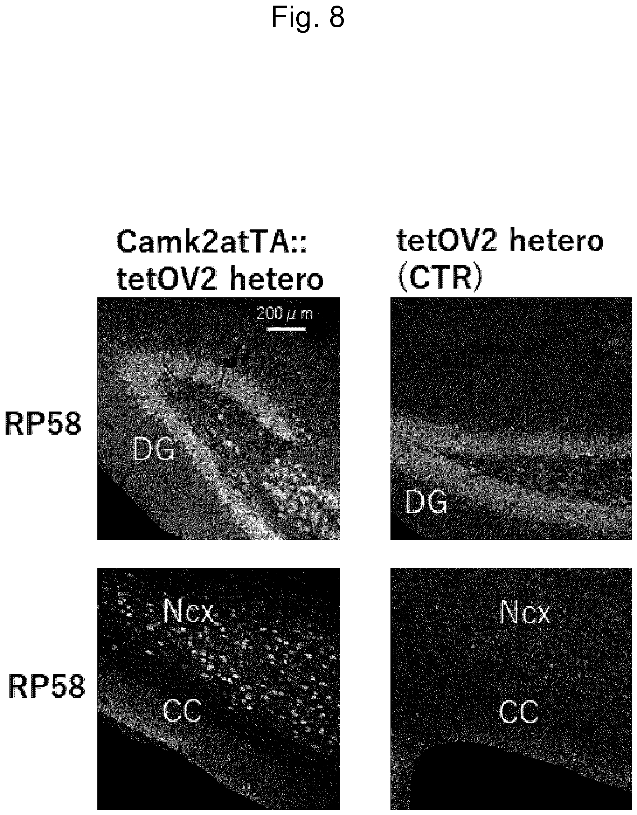

The results obtained by staining with RP58, Camk2tTA:: Rp58 tetO hetero mice (2-month-old), in which the expression of RP58 in mature excitatory neurons was increased from the early nascent stage, are shown (Figure 8). As control samples, Rp58 tetO hetero mice were used. The expression of RP58 was successfully, artificially increased.

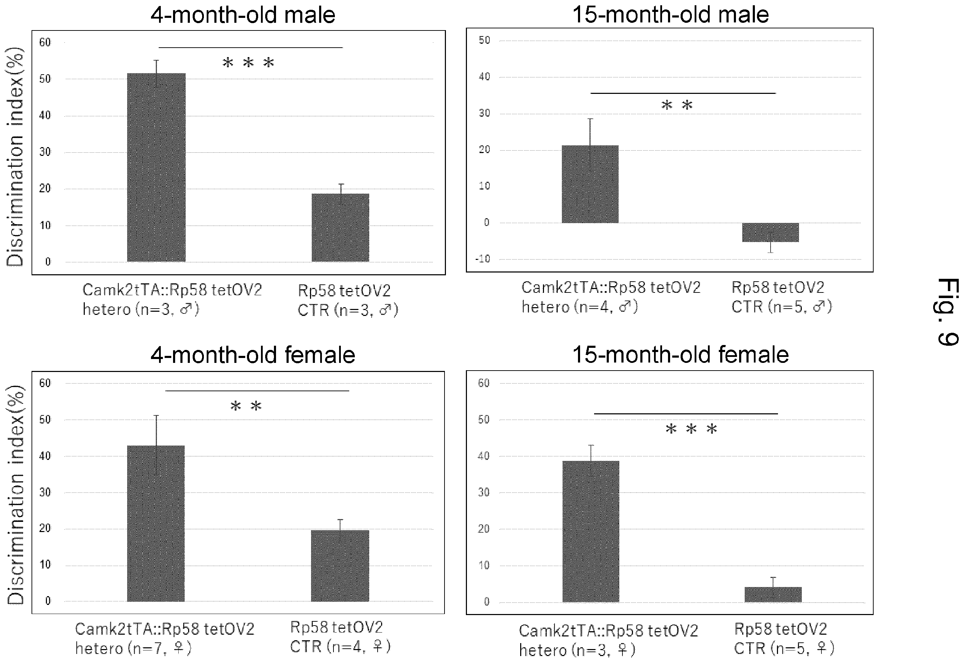

With regard to Camk2tTA::tetO (V2) Rp58 hetero mice, in which the expression of RP58 in mature excitatory neurons had been increased from the early nascent stage, an increase in cognitive function was found on the 4th month according to an object location recognition test (1 hour memory), and further, on the 15th month, a reduction in cognitive function due to aging was suppressed (Figure 9). Furthermore, on the 10th month, continuous evaluation was carried out according to an object location recognition + novel object recognition test (3 minutes memory). As a result, suppression of a reduction in cognitive function due to aging was observed in the Camk2tTA::tetO Rp58 hetero mice in the two tests (Figure 10). Therefore, it was demonstrated that a quantitative increase in RP58 induces acceleration in cognitive function, or that a reduction in cognitive function due to aging can be prevented by such a quantitative increase in RP58.

In addition, female Camk2tTA::tetO (VI) Rp58 hetero mice exhibited an action to bite their tail. This action was considered to be self-injury.

### < Expression increase models using AAV vector >

RP58-expressed adeno-associated virus (AAV-CaMKIIa-cMyc-RP58) was intravenously injected into 3-month-old wild-type mice, so that RP58 was overexpressed therein. As a result, cMyc as a marker was detected, and the stainability of RP58 was enhanced. When GFP-expressed AAV was used as a control, the expression of GFP was observed (Figure 11). Therefore, RP58 could be overexpressed in the neurons of the cerebral cortex and hippocampus.

No change was found in the RP58 overexpression mice according to an object location recognition + novel object recognition test. However, habituation was promoted in an open field test, and one week later, the second contextual searching behavior of the mice was decreased. Thus, acceleration in the habituation was observed (Figure 12). It is assumed that these results mean the improvement of contextual memory, which is considered to be the improvement of cognitive function. Rodents that were bred under an enrichment environment exhibited acceleration in the above-described habituation (the above-mentioned Non Patent Literature 21: Brenes et al., 2009, the above-mentioned Non Patent Literature 22: Rojas-Carvajal et al., 2018).

### < Effects of forced expression of RP58 on Alzheimer's disease mouse models >

It was elucidated that RP58-expressed adeno-associated virus was administered (the above-mentioned Non Patent Literature 24: Matsuzaki et al., Neurosci Lett. 2018) to human-type mutation APP homo mice (AD mouse models; the above-mentioned Non Patent Literature 23: Saito et al., Clin Exp Neuroimmunol. 2018), and as a result, cognitive function was improved (Figure 13). Specifically, before administration of the virus, the cognitive function of the 3-month-old APP homo mice was reduced according to an object location recognition + novel object recognition test. However, one month after administration of the RP58-expressed virus, the cognitive function of the APP homo mice was returned to a normal level. On the other hand, cognitive function remained low one month after administration of a GFP-expressed virus.

The results obtained by staining with RP58, cMyc, NeuN, GFP, the amyloid β marker 82E1, the microglia markers Iba1 and CD68, and the activated-form microglia Iba1+/CD68+ (white arrowhead) in the aforementioned tissue analysis are shown (Figure 14). As control samples, mice administered with AAV-CaMKIIa-Gfp were used. In an RP58 replenishment group, the size of an amyloid plaque tended to be smaller than the size of a control (Figures 14 and 15). These results show that an increase in the amyloid plaques was suppressed by the replenishment of RP68 in 3 months. On the other hand, activation of the microglia tended to be decreased, but a significant change was not observed.

These results demonstrate that the reduced cognitive function of Alzheimer's disease mouse models could be increased by increasing the amount of RP58. In addition, at that time, together with the recovery of the cognitive function, a slight decrease was observed in formation of the amyloid plaques and activation of the microglia. Hence, it was demonstrated that the expression of RP58 in mature excitatory neurons suppresses generation of amyloid plaques and activation of the microglia, and that this can be a factor for the recovery of cognitive function. The level of suppression of the generation of the amyloid plaques and the activation of the microglia due to an increase in RP58 can be demonstrated by increasing the number of cases.

### (2) Conclusion and future prospects

Amouse strain capable of increasing or decreasing the amount of RP58 in a stage-specific manner could be constructed using a genetically modified mouse and a viral vector. As a result, it was demonstrated that the amount of RP58 regulates cognitive function, and that a reduction in cognitive function due to aging is caused by a decrease in the expression of RP58.

In particular, by increasing the expression of RP58 in wild-type mice, cognitive function can be accelerated and a reduction in cognitive function due to aging can be suppressed. Hence, a compound capable of increasing the expression of RP58 is screened in cultured cells, or various environmental factors, foods, drugs, etc. are given to wild-type mice, and those capable of increasing the expression of RP58 in the brain are then searched, so that it also becomes possible to discover a method or a substance that promotes acceleration in cognitive function or suppression of a reduction in cognitive function.

Furthermore, the cognitive function of Alzheimer's disease mouse models was improved by replenishment and/or introduction of an RP58 gene. It has been reported that RP58 is decreased in the brain of Alzheimer's disease patients (the above-mentioned Non Patent Literature 25: Castillo et al., 2017). The results of the present example suggest that a reduction in cognitive function found in the brain of Alzheimer's disease patients be related to the expression level of RP58. Accordingly, elucidation of the type of a gene whose expression is suppressed by RP58, in which the suppression of the gene expression leads to suppression of a reduction in cognitive function and the pathological condition of Alzheimer's disease, could be a clue of clarification of the cognitive function and the molecular mechanism of Alzheimer's disease. Moreover, medically, there is a possibility of performing a gene therapy using RP58 on Alzheimer's disease, and at the same time, the searching of a compound capable of increasing the expression of RP58, the searching of a drug for regulating the genetic cascade of a gene located downstream of RP58, and the like are possibly utilized in the discovery of a drug for Alzheimer's disease, and such searching can contribute to the development of a drastic preventive agent or therapeutic agent for Alzheimer's disease, for which a treatment method has not yet been established.

### Industrial Applicability

The transgenic non-human animal capable of increasing or decreasing the expression of the transcription factor RP58 in cells of the central nervous system of the non-human animal in the nascent stage and/or the during and after developmental stage, and the animal model of brain dysfunction or the like, in which the aforementioned non-human animal is used, according to the present invention are extremely useful, in that the present transgenic non-human animal and the present animal model are able to provide a method of screening for a therapeutic or preventive agent against brain dysfunction or the like, a pharmaceutical composition for use in the treatment or prevention of the brain dysfunction or the like, etc.

### Sequence Listing Free Text

SEQ ID NO: 32: Recombinant DNA
SEQ ID NO: 33: Recombinant DNA
SEQ ID NO: 34: Recombinant DNA

## Claims

1. A transgenic non-human animal capable of increasing or decreasing the expression of a transcription factor RP58 in cells of the central nervous system of the non-human animal in the nascent stage and/or during and after the developmental stage.

2. The transgenic non-human animal according to claim 1, which is capable of increasing or decreasing the expression of the RP58 in cells of the central nervous system of the non-human animal during and after the developmental stage.

3. The transgenic non-human animal according to claim 1 or 2, which is capable of increasing or decreasing the expression of the RP58, in a desired period in the nascent stage and/or the during and after developmental stage of the non-human animal.

4. The transgenic non-human animal according to any one of claims 1 to 3, which is capable of increasing or decreasing the expression of the RP58 according to a Tet system.

5. The transgenic non-human animal according to claim 4, into which a promoter and a tetracycline-controlled transcriptional activator (tTA) gene linked to the promoter so that the tTA gene can function by the promoter, and a Tet operator (tetO) are introduced, and which is capable of increasing the expression of the RP58 by non-administration of doxycycline.

6. The transgenic non-human animal according to claim 4, into which a promoter and a tetracycline-controlled transcriptional silencer (tTS) gene linked to the promoter so that the tTS gene can function by the promoter, and a Tet operator (tetO) are introduced, and which is capable of decreasing the expression of the RP58 by non-administration of doxycycline.

7. The transgenic non-human animal according to claim 5 or 6, wherein the promoter is a Camk2a promoter or an Actin promoter.

8. The transgenic non-human animal according to any one of claims 5 to 7, wherein the expression of the RP58 is maintained at a normal level by administration of doxycycline.

9. The transgenic non-human animal according to any one of claims 1 to 8, wherein the cells of the central nervous system are brain nervous system cells.

10. The transgenic non-human animal according to any one of claims 1 to 9, wherein the non-human animal is a rodent.

11. The transgenic non-human animal according to claim 10, wherein the rodent is a mouse.

12. An animal model of brain dysfunction or a disease related thereto, in which the expression of the transcription factor RP58 is decreased in cells of the central nervous system of the transgenic non-human animal according to any one of claims 1 to 4 and 6 to 11.

13. The animal model according to claim 12, wherein the expression of the RP58 is decreased in cells of the central nervous system of the transgenic non-human animal during and after the developmental stage.

14. The animal model according to claim 12 or 13, wherein the brain dysfunction is at least one selected from the group consisting of brain tumor, cognitive dysfunction, developmental disability, intellectual disability, memory impairment, and age-related brain disorder.

15. A method of screening for a therapeutic or preventive agent against brain dysfunction or a disease related thereto, which comprises a step of decreasing the expression of the transcription factor RP58 in cells of the central nervous system of the transgenic non-human animal according to any one of claims 1 to 4 and 6 to 11, a step of administering a candidate substance to the non-human animal in which the expression of the RP58 is decreased, and a step of evaluating the pathological condition of the brain dysfunction or a disease related thereto in the non-human animal after administration of the candidate substance.

16. The screening method according to claim 15, wherein the step of decreasing the expression of the RP58 is a step of decreasing the expression of the RP58 in cells of the central nervous system of the transgenic non-human animal during and after the developmental stage.

17. The screening method according to claim 15 or 16, wherein the brain dysfunction is at least one selected from the group consisting of brain tumor, cognitive dysfunction, developmental disability, intellectual disability, memory impairment, and age-related brain disorder.

18. A pharmaceutical composition for use in the treatment or prevention of brain dysfunction or a disease related thereto, wherein the pharmaceutical composition comprises a transcription factor RP58 protein or a nucleic acid encoding the transcription factor RP58.

19. A pharmaceutical composition for use in the treatment or prevention of brain dysfunction or a disease related thereto, wherein the pharmaceutical composition comprises a therapeutic or preventive agent obtained by the screening method according to any one of claims 15 to 17.

20. The pharmaceutical composition according to claim 18 or 19, wherein the brain dysfunction is at least one selected from the group consisting of brain tumor, cognitive dysfunction, developmental disability, intellectual disability, memory impairment, and age-related brain disorder.

21. A method for treating or preventing brain dysfunction or a disease related thereto, which comprises administering the pharmaceutical composition according to any one of claims 18 to 20 to a patient who has or is likely to have brain dysfunction or a disease related thereto.

22. The therapeutic or preventive method according to claim 21, wherein the brain dysfunction is at least one selected from the group consisting of brain tumor, cognitive dysfunction, developmental disability, intellectual disability, memory impairment, and age-related brain disorder.

23. An animal model of behavioral disorder or a disease related thereto, in which the expression of the transcription factor RP58 is increased in cells of the central nervous system of the transgenic non-human animal according to any one of claims 1 to 5 and 7 to 11.

24. The animal model according to claim 23, wherein the behavioral disorder or a disease related thereto is at least one selected from the group consisting of borderline personality disorder, schizophrenia, depression, bipolar disorder, eating disorder, dissociative disorder, post-traumatic stress syndrome, addiction, autism, and self-injury.

25. A method of screening for a therapeutic or preventive agent against behavioral disorder or a disease related thereto, which comprises a step of increasing the expression of the transcription factor RP58 in cells of the central nervous system of the transgenic non-human animal according to any one of claims 1 to 5 and 7 to 11, a step of administering a candidate substance to the non-human animal in which the expression of the RP58 is increased, and a step of evaluating the pathological condition of the behavioral disorder or a disease related thereto in the non-human animal after administration of the candidate substance.

26. The screening method according to claim 25, wherein the behavioral disorder or a disease related thereto is at least one selected from the group consisting of borderline personality disorder, schizophrenia, depression, bipolar disorder, eating disorder, dissociative disorder, post-traumatic stress syndrome, addiction, autism, and self-injury.

27. The screening method according to claim 25 or 26, wherein the candidate substance is a nucleic acid capable of suppressing the function of a gene encoding RP58.

28. The screening method according to claim 27, wherein the nucleic acid is antisense DNA, siRNA, shRNA, or microRNA.

29. A pharmaceutical composition for use in the treatment or prevention of behavioral disorder or a disease related thereto, wherein the pharmaceutical composition comprises a therapeutic or preventive agent obtained by the screening method according to any one of claims 25 to 28.

30. The pharmaceutical composition according to claim 29, wherein the behavioral disorder or a disease related thereto is at least one selected from the group consisting of borderline personality disorder, schizophrenia, depression, bipolar disorder, eating disorder, dissociative disorder, post-traumatic stress syndrome, autism, addiction, and self-injury.

31. A method for treating or preventing behavioral disorder or a disease related thereto, which comprises administering the pharmaceutical composition according to claim 29 or 30 to a patient who has or is likely to have behavioral disorder or a disease related thereto.

32. The therapeutic or preventive method according to claim 31, wherein the behavioral disorder or a disease related thereto is at least one selected from the group consisting of borderline personality disorder, schizophrenia, depression, bipolar disorder, eating disorder, dissociative disorder, post-traumatic stress syndrome, addiction, autism, and self-injury.

33. A method of screening for a substance that improves or reduces cognitive function, which comprises administering a candidate substance to a wild-type non-human animal, or allowing a candidate substance to come into contact with wild-type animal cells, and then screening the substance that improves or reduces cognitive function, using, as an indicator, the expression level of the transcription factor RP58 in the brain of the non-human animal after the administration or in the animal cells after the contact.

34. An agent for improving or reducing cognitive function, comprising a substance obtained by the screening method according to claim 33.

35. A method for improving or reducing cognitive function, comprising administering the agent for improving or reducing cognitive function according to claim 34 to a subject.

36. A method of screening for an environment that improves or reduces cognitive function, which comprises breeding a wild-type non-human animal under a candidate environment, or culturing wild-type animal cells under a candidate environment, and then screening the environment that improves or reduces cognitive function, using, as an indicator, the expression level of the transcription factor RP58 in the brain of the non-human animal after the breeding or in the animal cells after the culture.

37. A method for improving or reducing cognitive function, comprising providing the environment screened by the method according to claim 36 to a subject.
